# EUROPEAN PATENT SPECIFICATION

(11) **EP 4 298 225 B1**
(45) Date of publication and mention of the grant of the patent: **11.11.2026**
(21) Application number: 22760413.9
(22) Date of filing: 24.02.2022
(51) Int. Cl.: C12N 15/63, C12N 7/00, A61K 39/39, A61K 35/761

(54) **ONCOLYTIC VIRUS AND METHODS OF USE**
ONKOLYTISCHES VIRUS UND VERFAHREN ZUR VERWENDUNG
VIRUS ONCOLYTIQUE ET PROCÉDÉS D'UTILISATION

(30) Priority: 26.02.2021 US 202163154118 P
(43) Date of publication of application: 03.01.2024
(73) Proprietor: NorthShore University HealthSystem, Evanston, IL 60201 (US)
(72) Inventor: SETH, Prem, Evanston, Illinois 60201 (US)
(74) Representative: Boult Wade Tennant LLP
(86) International application number: PCT/US2022/017716
(87) International publication number: WO 2022/182884

(56) References cited:
- WO-A1-2015/152609
- WO-A1-2020/198293
- WO-A1-2020/198293
- HOWARD L. KAUFMAN ET AL: "Oncolytic viruses: a new class of immunotherapy drugs", NATURE REVIEWS DRUG DISCOVERY, vol. 14, no. 9, 1 September 2015 (2015-09-01), GB, pages 642 - 662, XP055266462, ISSN: 1474-1776, DOI: 10.1038/nrd4663
- KIM SO YOUNG ET AL: "Prime-boost immunization by both DNA vaccine and oncolytic adenovirus expressing GM-CSF and shRNA of TGF-[beta]2 induces anti-tumor immune activation", ONCOTARGET, vol. 8, no. 9, 28 February 2017 (2017-02-28), United States, pages 15858 - 15877, XP055879746, ISSN: 1949-2553, DOI: 10.18632/oncotarget.15008
- ZHANG: "Therapeutic effects of mesenchymal stem cells loaded with oncolytic adenovirus carrying decorin on a breast cancer lung metastatic mouse model", MOLECULAR THERAPY ONCOLYTICS., 27 January 2022 (2022-01-27), XP055965080, DOI: 10.1016/j.omto. 2022.01.00 7
- ANDERS: "Chimeric Granulocyte/Macrophage Colony-stimulating Factor/Transforming Growth Factor- (TGF- ) Receptors Define a Model System for Investigating the Role of Homomeric and Heteromeric Receptors in TGF- Signaling", JOURNAL OF BIOLOGICAL CHEMISTRY, pages 21758 - 21766, XP002432036, DOI: 10.1074/jbc.271.36.21758

## Description

A sequence listing is provided herewith as a text file, "AMUN-N001WO_SEQ_LIST_ST25.txt," created on February 11, 2022 and having a size of 66000 bytes. The contents of the text file are incorporated by reference herein in their entirety.

### BACKGROUND

Recently there has been a tremendous interest in developing oncolytic viruses for cancer therapy and for improving immunotherapies. However, to date only one oncolytic virus, Herpes virus expressing GM-CSF, has been approved for clinical use in treating melanomas (see WO 2015/152609 A1, WO 2020/198293 A1, H.L. Kaufman et al. (NATURE REVIEWS DRUG DISCOVERY, vol. 14, no. 9, pages 642-662,) and K. Young et al. (ONCOTARGET, vol. 8, no. 9, pages 15858-15877)). Therefore, there is a great need for oncolytic viruses that can be used for cancer therapy and for improving immunotherapies.

### SUMMARY

The present disclosure provides an oncolytic virus comprising a nucleic acid encoding a granulocyte-macrophage colony-stimulating factor (GM-CSF) and a soluble form of the TGF-B receptor-II (sTGF-BRII). The present disclosure also provides compositions comprising the oncolytic virus and treatment methods using the oncolytic virus. The treatment methods include local and/or systemic administration of the oncolytic virus for treating cancers, such as solid tumors.

In one aspect, the disclosure provides an oncolytic virus comprising a nucleic acid encoding a granulocyte-macrophage colony-stimulating factor (GM-CSF) and a soluble form of the TGF-B receptor-II (sTGF-BRII). In some embodiments, the oncolytic virus is an adenovirus, herpes virus, lentivirus, vaccinia virus, Reo virus, maraba virus, Newcastle disease virus, sendai virus, pox virus, poliovirus, myxoma virus, or retrovirus. In some embodiments, the oncolytic virus is an adenovirus. Some embodiments provide an oncolytic virus that preferentially replicates in cancer cells. In some embodiments, the expression of genes required for replication of the virus is under the control of a promoter active in the cancer cells, such as a telomerase reverse transcriptase (TERT) promoter. In some embodiments, the expression of sTGF-BRII is under the control of a cytomegalovirus (CMV) promoter. In some embodiments, the expression of GM-CSF is under the control of an adenoviral E1B promoter. In some embodiments, the virus comprises a capsid protein that specifically binds to a cancer cell that overexpresses an adenovirus receptor. In some embodiments, the cancer cell is a breast cancer cell, skin cancer cell, lung cancer cell, renal cancer cell, ovarian cancer cell, prostate cancer cell, pancreatic cancer cell, brain cancer cell, musculoskeletal cancer, astrocytoma cancer cell, cervical cancer cell, testicular cancer cell, hepatic cancer cell, lymphoma cancer cell, colon cancer cell, or bladder cancer cell. In some embodiments, the cancer cell is a breast cancer cell, such as a triple negative breast cancer cell. In some embodiments, the cancer cell is a skin cancer cell, such as a melanocyte. In some embodiments, the nucleic acid encoding the GM-CSF is codon-optimized to increase expression of the GM-CSF in a human subject. In some embodiments, the GM-CSF is a human GM-CSF. In some embodiments, the sTGF-BRII is fused to a heterologous protein. In some embodiments, the heterologous protein comprises a half-life-extending moiety, such as an immunoglobulin Fc region.

Another aspect of the disclosure is drawn to a pharmaceutical composition comprising: a therapeutically effective amount of the oncolytic virus described above and a pharmaceutically acceptable excipient, diluent, or carrier.

Still another aspect of the disclosure is directed to a method of treating cancer in a subject, the method comprising: administering a therapeutically effective amount of the oncolytic virus described above or the composition described above to the subject. In some embodiments of the method, the cancer is breast cancer, skin cancer, lung cancer, bladder cancer, renal cancer, astrocytoma, hepatic cancer, lymphoma, colon cancer, or head/neck cancer. In some embodiments, the administering comprises an intratumoral or intravenous administration. In some embodiments, the cancer is a primary cancer. In some embodiments, the cancer is metastatic cancer. In some embodiments, the method further comprises administering to the subject one or more of an immunotherapy, a chemotherapy, a surgical treatment, a radiation therapy, a hormone therapy, an anti-cancer small molecule, a growth factor inhibitor therapy, CAR-T therapy, or a cytokine therapy. In some embodiments, an immunotherapy is administered to the subject. In some embodiments, a chemotherapy is administered to the subject. In some embodiments, an immune checkpoint inhibitor is administered to the subject. In some embodiments, the immune checkpoint inhibitor comprises an inhibitor of PD-1, PD-L1, CTLA-4, LAG-3, BCH-3, BCH-4, or TIM-3. In some embodiments, the inhibitor is an antibody.

Other features and advantages of the disclosure will be apparent from the following detailed description and figures, and from the claims.

### BRIEF DESCRIPTION OF THE DRAWINGS

Included in the drawings are the following figures:
FIGS. 1A-1B. Schematics of adenoviral vectors based on Ad5 vector.
FIG. 1C. Virus replication assay to measure virus production using the listed adenoviral vectors.
FIGS. 2A-2B. Effect of adenovirus comprising the vectors depicted in FIG. 1A on tumor volume and tumor weight in a mouse model for mammary tumor.
FIGS. 3A-3C. Cytotoxicity of replicating viruses rAd.sT.GM, rAd.GM, rAd.sT, rAd.Null and replication-deficient Ad(E1-) in mouse breast cancer cells 4T1 and in human breast cancer cells (MDA-MB-231 and MCF-7).
FIGS. 4A-4B. Concentration of TGFbRIIFc and hGM-CSF in virus infected cell cuture supernatant.
FIGS. 5A-5B. sTGFbRIIFc expression in 4T1 tumors in mice on day 10 and day 25 post-infection.
FIGS. 5C-5D. GM-CSF expression in 4T1 tumors in mice on day 10 and day 25 post-infection.

### DETAILED DESCRIPTION

The present disclosure provides an oncolytic virus comprising a nucleic acid encoding a granulocyte-macrophage colony-stimulating factor (GM-CSF) and a soluble form of the TGF-B receptor-II (sTGF-BRII). The present disclosure provides compositions comprising the oncolytic virus and treatment methods using the oncolytic virus. The treatment methods include local and/or systemic administration of the oncolytic virus for treating cancers, such as solid tumors.

Before exemplary embodiments of the present invention are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials may now be described.

It must be noted that as used herein and in the appended claims, the singular forms "a", "an", and "the" include plural referents unless the context clearly dictates otherwise. Thus, for example, reference to "an adenovirus virion" includes a plurality of such virions and reference to "the vector" includes reference to one or more vectors, "a mutation" refers to one or more mutations, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. To the extent such publications may set out definitions of a term that conflicts with the explicit or implicit definition of the present disclosure, the definition of the present disclosure controls.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

### DEFINITIONS

A "recombinant adenovirus vector" as used herein refers to an adenovirus vector comprising a polynucleotide sequence not of adenoviral origin *(i.e.,* a polynucleotide heterologous to the adenovirus), typically a sequence of interest for expression in a cell. In general, the heterologous polynucleotide is flanked by at least one, and generally by two inverted terminal repeat sequences (ITRs). The term recombinant adenovirus vector encompasses both adenoviral vector particles and adenoviral vector plasmids.

An "adenovirus" or " adenoviral particle" or " adenovirus vector particle" refers to a viral particle composed of at least one adenovirus capsid protein (typically of all of the capsid proteins of a wild-type adenovirus) and an encapsidated polynucleotide adenovirus vector. If the particle comprises a heterologous polynucleotide (*i.e.,* a polynucleotide other than a wild-type adenovirus genome, such as a transgene to be delivered to a mammalian cell), it is typically referred to as a "recombinant adenovirus vector particle" or simply a "rAd vector". Thus, production of rAd particle necessarily includes production of rAd vector, as such a vector contained within a rAd particle.

An "infectious" virus or viral particle is one that comprises a polynucleotide component which it is capable of delivering into a cell for which the viral species is tropic. The term does not necessarily imply any replication capacity of the virus. As used herein, an "infectious" virus or viral particle is one that can access a target cell, can infect a target cell, and can express a heterologous nucleic acid in a target cell. Thus, "infectivity" refers to the ability of a viral particle to access a target cell, infect a target cell, and express a heterologous nucleic acid in a target cell. Infectivity can refer to *in vitro* infectivity or *in vivo* infectivity. Assays for counting infectious viral particles are described in the art. Viral infectivity can be expressed as the ratio of infectious viral particles to total viral particles. Total viral particles can be expressed as the number of viral genome (vg) copies. The ability of a viral particle to express a heterologous nucleic acid in a cell can be referred to as "transduction." The ability of a viral particle to express a heterologous nucleic acid in a cell can be assayed using a number of techniques, including assessment of a marker gene, such as a green fluorescent protein (GFP) assay (*e.g.,* where the virus comprises a nucleotide sequence encoding GFP), where GFP is produced in a cell infected with the viral particle and is detected and/or measured; or the measurement of a produced protein, for example by an enzyme-linked immunosorbent assay (ELISA). Viral infectivity can be expressed as the ratio of infectious viral particles to total viral particles. Methods of determining the ratio of infectious viral particle to total viral particle are known in the art.

The term "polynucleotide" or "nucleic acid" refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a nucleic acid encompasses both the double-stranded form and each of two complementary single-stranded forms known or predicted to make up the double-stranded form.

A "gene" refers to a polynucleotide containing at least one open reading frame that is capable of encoding a particular protein after being transcribed and translated.

"Recombinant," as applied to a polynucleotide means that the polynucleotide is the product of various combinations of cloning, restriction or ligation steps, and other procedures that result in a construct that is distinct from a polynucleotide found in nature. A recombinant virus is a viral particle comprising a recombinant polynucleotide. The terms respectively include replicates of the original polynucleotide construct and progeny of the original virus construct.

A "control element" or "control sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Control elements known in the art include, for example, transcriptional regulatory sequences such as promoters and enhancers. A promoter is a DNA region capable under certain conditions of binding RNA polymerase and initiating transcription of a coding region usually located downstream (in the 3' direction) from the promoter. Reference to a gene promoter refers to both the promoter for the expression of the gene as found in nature as well as modified versions of the natural promoter, *e.g.,* a shortened promoter, chimeric promoter, a mutated promoter, etc.

"Operatively linked" or "operably linked" refers to a juxtaposition of genetic elements, wherein the elements are in a relationship permitting them to operate in the expected manner. For instance, a promoter is operatively linked to a coding region if the promoter helps initiate transcription of the coding sequence. There may be intervening residues between the promoter and coding region so long as this functional relationship is maintained.

An "expression vector" is a vector comprising a region which encodes a polypeptide of interest, and is used for effecting the expression of the protein in an intended target cell. An expression vector also comprises control elements operatively linked to the coding region to facilitate expression of the protein in the target. The combination of control elements and a gene or genes to which they are operably linked for expression is sometimes referred to as an "expression cassette," a large number of which are known and available in the art or can be readily constructed from components that are available in the art.

"Heterologous" means derived from a genotypically distinct entity from that of the rest of the entity to which it is being compared. For example, a polynucleotide introduced by genetic engineering techniques into a plasmid or vector derived from a different species is a heterologous polynucleotide. A promoter removed from its native coding sequence and operatively linked to a coding sequence with which it is not naturally found linked is a heterologous promoter. Thus, for example, a recombinant adenovirus that includes a heterologous nucleic acid encoding a heterologous gene product is an adenovirus that includes a nucleic acid not normally included in a naturally occurring, wild-type adenovirus, and the encoded heterologous gene product is a gene product not normally encoded by a naturally occurring, wild-type adenovirus.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, particularly in a human, and includes: a) inhibiting the disease, *i.e.,* arresting or slowing its development; and (b) relieving the disease, *i.e.,* causing regression of the disease. One form of treatment includes prevention of occurrence of a disease or delay in onset of the disease and/or reduced severity of the disease after onset of the disease in a subject which may be predisposed to the disease or at risk of acquiring the disease but has not yet been diagnosed as having it.

The terms "individual," "host," "subject," and "patient" are used interchangeably herein, and refer to a mammal, including, but not limited to, human and non-human primates, including simians and humans; mammalian sport animals (*e.g.,* horses, camels, etc.); mammalian farm animals (*e.g.,* sheep, goats, cows); mammalian pets (dogs, cats, etc.); and rodents (*e.g.,* mice, rats, etc.). In some cases, the individual is a human.

The term "pharmaceutically acceptable" refers to a non-toxic material which preferably does not interfere with the action of the active ingredient of the pharmaceutical composition. In particular, the term "pharmaceutically acceptable" means that the subject substance has been approved by a governmental regulatory agency for use in animals, and particularly humans, or in U.S. Pat. Pharmacopoeia, European Pharmacopoeia or other recognized pharmacopoeias for use in animals and in particular humans.

The term "cancer" as used herein refers to a cancer of any kind and origin including tumor-forming cells, blood cancers and/or transformed cells.

The term "cancer cell" includes cancer or tumor-forming cells, transformed cells or a cell that is susceptible to becoming a cancer or tumor-forming cell.

The term "recombinant oncolytic virus" refers to an engineered oncolytic virus, such as an adenovirus, generated in vitro using recombinant DNA technology and/or an oncolytic virus derived from such a recombined oncolytic virus (*e.g.,* progeny virus).

The term "oncolytic" as used herein refers to a tumor-selective replicating virus that induces cell death in the infected cell, and/or tissue. Although normal or non-tumor cells may be infected, tumor cells are infected and lysed preferentially in comparison to the normal or non-tumor cells. For example, an oncolytic virus induces at least 5-fold, at least 6-fold, at least 10-fold, at least 15-fold, or at least 20-fold more cell death in a population of cancer cells compared to normal cells.

The term "normal tissue" as used herein refers to non-cancer tissue and/or tissue derived from a subject that is free of cancer of the particular tissue (*e.g.,* when the tissue is pancreas, "normal tissue" can be derived from a subject that does not have pancreatic cancer). The term "normal cell of the same tissue type" as used herein refers to a cell or cells derived from such normal tissue.

The term "resistant cancer" or "chemotherapeutic resistant cancer" refers to a cancer that has decreased sensitivity to one or more chemotherapeutic drugs, for example by amplifying a gene that allows it to persist in the presence of the drug, for example by increasing expression of pumps that decrease amount of the drug inside a cancer cell, such as, MDR1.

The term "antibody" as used herein is intended to include monoclonal antibodies, polyclonal antibodies, chimeric antibodies, humanized antibodies. The antibody may be from recombinant sources and/or produced in transgenic animals. The term "antibody fragment" as used herein is intended to include Fab, Fab', F(ab')2, scFv, dsFv, ds-scFv, dimers, minibodies, diabodies, and multimers thereof and bi-specific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bi-specific antibody fragments and other fragments can also be synthesized by recombinant techniques. Methods for making antibodies are well known in the art.

The term "nucleic acid" includes DNA and RNA and can be either double-stranded or single-stranded.

The term "sequence identity" as used herein refers to the percentage of sequence identity between two polypeptide sequences or two nucleic acid sequences. To determine the percent identity of two amino acid sequences or of two nucleic acid sequences, the sequences are aligned for optimal comparison purposes (e. g. , gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i. e., % identity=number of identical overlapping positions/total number of positions times). The determination of percent identity between two sequences can also be accomplished using a mathematical algorithm. A preferred, non-limiting example of a mathematical algorithm utilized for the comparison of two sequences is the algorithm of Karlin and Altschul, 1990, Proc Natl Acad Sci USA 87 2264-2268, modified as in Karlin and Altschul, 1993, Proc Natl Acad Sci U S A 90 5873-5877. Such an algorithm is incorporated into the NBLAST and XBLAST programs.

As used herein, "contemporaneous administration" and "administered contemporaneously" mean that two substances are administered to a subject such that they are both biologically active in the subject at the same time. The exact details of the administration will depend on the pharmacokinetics of the two substances in the presence of each other, and can include administering one substance within 24 hours of administration of the other, if the pharmacokinetics are suitable. In particular embodiments, two substances will be administered substantially simultaneously, *i.e.* within minutes of each other, or in a single composition that comprises both substances.

As used herein, the phrase "effective amount" or "therapeutically effective amount" means an amount effective, at dosages and for periods of time necessary to achieve the desired result. For example, in the context or treating a cancer, an effective amount is an amount that for example induces remission, reduces tumor burden, and/or prevents tumor spread or growth compared to the response obtained without administration of the oncolytic virus provided herein. Effective amounts may vary according to factors such as the disease state, age, sex, weight of the subject. The amount of an oncolytic virus herein that will correspond to such an amount will vary depending upon various factors, such as the pharmaceutical formulation, the route of administration, the type of cancer, the severity of the cancer, and the like, but can nevertheless be routinely determined by one skilled in the art.

The term "replication competent" refers to any viral vector that is not deficient in any gene function required for viral replication in specific cells or tissues. The vector must be capable of replicating and being packaged, but might replicate only conditionally in specific cells or tissues. Replication competent adenoviral vectors disclosed herein may be engineered to reduce or eliminate their ability to replicate in normal cells while retaining their ability to replicate efficiently in cancer cells.

### RECOMBINANT ONCOLYTIC VIRUS

The present disclosure provides an oncolytic virus comprising a nucleic acid encoding a granulocyte-macrophage colony-stimulating factor (GM-CSF) and a soluble form of the transforming growth factor-beta (TGF-B) receptor-II (sTGF-BRII). This oncolytic virus may be used for treating cancer, such as solid tumors as well as metastatic cancer, in part by providing GM-CSF and sTGF-BRII in the blood stream of a subject receiving the oncolytic virus. In addition, the oncolytic virus may inhibit tumor metastasis.

The oncolytic virus may be an adenovirus, herpes virus, lentivirus (e.g., an integration deficient lentivirus), vaccinia virus, Reo virus, maraba virus, New Castle disease virus, sendai virus, pox virus, poliovirus, myxoma virus, or retrovirus. In certain aspects, the oncolytic virus may be a replication-competent oncolytic virus.

In certain aspects, the oncolytic virus is a vaccinia virus, such as an orthopox virus, non-limiting examples of which include Western Reserve, Wyeth, and Copenhagen strains, optionally modified to increase cancer selectivity. Such modifications include, but are not limited to non-functional thymidine kinase gene, non-functional vaccinia growth factor gene, and non-functional type 1 interferon-binding gene.

In another aspect, the oncolytic virus is a herpes simplex virus (HSV), such as HSV1, HSV2 or HSV1716 strain. The HSV may include mutations that allow the virus to replicate in actively dividing cells, such as in cancer cells but which prevent significant replication in normal cells. Such mutations include disruption of the genes encoding ICP34.5 *(i.e.,* γ34.5), ICP6 and thymidine kinase.

In certain aspects, the oncolytic virus may be an adenovirus. The adenovirus may be a replication-competent adenovirus that can replicate in a host cell, such as, a mammalian cancer cell. The adenovirus may be of any of the 57 human adenovirus serotypes (HAdV-1 to 57). In one embodiment, the adenovirus is a subgroup B, for example Ad3, Ad5, Ad7, Ad11, Ad14, Ad16, Ad21, Ad34 or Ad51. In some examples, the oncolytic adenovirus is a replication competent Ad5 serotype adenovirus or a hybrid serotype adenovirus comprising an Ad5 component. The adenovirus may be a wild-type strain or may be genetically modified to enhance tumor selectivity, for example by attenuating the ability of the virus to replicate within normal quiescent cells without affecting the ability of the virus to replicate in tumor cells or by increasing replication of the virus in cancer cells as compared to normal cells. Non-limiting examples of replication competent oncolytic adenoviruses that can be used to provide sTGF-BRII and GM-CSF expression in a subject, such as a human, include Delta-24, Delta-24-RGD, ICOVIR-5, ICOVIR-7, ONYX-015, ColoAd1, H101 and AD5/3. Onyx-015 is a hybrid of virus serotype Ad2 and Ad5 with deletions in the E1B-55K and E3B regions to enhance cancer selectivity. H101 is a modified version of Onyx-015. ICOVIR-5 and ICOVIR-7 comprise an Rb-binding site deletion of E1A and a replacement of the E1A promoter by an E2F promoter. ColoAd1 is a chimeric Add11p/Ad3 serotype. AD5/3 is a serotype 5/3 capsid-modified adenovirus (the Ad5 capsid protein knob is replaced with a knob domain from serotype 3). In some cases, the oncolytic adenovirus may be an adenoviral d101/07 mutant that can replicate in a variety of cancer cells regardless of their genetic defects. The d101/07 mutant adenovirus includes a mutant E1A protein in which amino acids from position 4-25 and 111-123 are deleted. The mutant E1A protein cannot bind with p300 or Rb proteins. Thus, d101/07 is ineffective for S-phase progression in normal cells and cannot replicate in normal cells. However, cancer cells are able to progress to S-phase, permitting virus replication in cancer cells. In other cases, the adenovirus may include a wild type E1A protein that binds with p300 or Rb proteins. The expression of the E1A gene may be placed under the control of human TERT promoter to provide for selective replication of the virus in human cancer cells. The recombinant adenovirus may include an active E1A gene and an inactivated E1B 19 gene, an inactivated E1B 55 gene, or an inactivated E1B 19/E1B 55 gene. As used herein the term "inactivation" in the context of a gene means that the transcription and/or translation of the gene is reduced or absent, and thus the function of the protein encoded by the gene is reduced or undetectable. For example, the inactivated E1B 19 gene is a gene that cannot produce an active E1B 19 kDa protein due to mutation (substitution, addition, partial deletion or total deletion) of the gene. When E1B 19 is deleted, cell apoptosis can be increased, and when the E1B 55 gene is deleted, tumor cell specificity can be increased. The term deletion, used in connection with the viral genome sequence in the present disclosure, means complete deletion or partial deletion of a gene. The adenovirus of the present disclosure may include an active E1A gene, an inactivated E1B 19/E1B 55 gene, and an inactivated E3 gene.

The cancer cell that is targeted by the disclosed oncolytic virus may be a breast cancer cell, skin cancer cell, lung cancer cell, renal cancer cell, ovarian cancer cell, prostate cancer cell, pancreatic cancer cell, brain cancer cell, astrocytoma cancer cell, hepatic cancer cell, lymphoma cancer cell, colon cancer cell, or bladder cancer cell. In certain embodiments, the cancer cell may be a human cancer cell. In particular embodiments, the breast cancer cell may be a triple negative breast cancer cell, the skin cancer cell may be a melanocyte.

The nucleic acid(s), encoding the sTGF-BRII and GM-CSF proteins, present in the oncolytic virus may be a DNA or RNA which may be single- or double-stranded depending upon the type of oncolytic virus. In certain aspects, the nucleic acid may be double-stranded DNA. In certain aspects, the nucleic acid(s) encoding the human sTGF-BRII and human GM-CSF may be a codon-optimized sequence(s), where the codons are codons from highly expressed human genes. The nucleic acids may be present in a single vector, such as a viral vector, that includes, at a minimum, a nucleic acid encoding sTGF-BRII, a nucleic acid encoding GM-CSF, and one or more promoters controlling expression of sTGF-BRII and GM-CSF, and 5' and 3' inverted terminal repeats (ITRs) flanking the promoter(s), the sTGF-BRII-encoding polynucleotide, and the GM-CSF-encoding polynucleotide. The vector may also include polynucleotides encoding proteins required for replication of the oncolytic virus. In certain embodiments, the viral vector only provides expression of the two therapeutic proteins, sTGF-BRII and GM-CSF, while in other embodiments, the viral vector can include additional nucleic acids encoding other therapeutic proteins or polynucleotides, such as RNA.

Nucleic acids encoding sTGF-BRII and GM-CSF can be inserted, e.g., at any nonessential location in the oncolytic virus genome so long as the oncolytic virus remains replication-competent. In one embodiment, the oncolytic virus is an adenovirus with a heterologous nucleic acid comprising a sequence encoding sTGF-BRII inserted downstream of a heterologous promoter. In another embodiment, a heterologous nucleic acid comprising a sequence encoding GM-CSF is inserted in the E3 region of a replication-competent adenovirus backbone. The E3 region is nonessential for viral replication; however, the E3 proteins play a role in regulating host immune response. The replication-competent adenovirus can comprise a full or partial E3 deletion. For example, the replication-competent adenovirus can comprise deletions of one, two, three or more open reading frames (ORFs) in the E3 region, with the heterologous nucleic acid encoding GM-CSF inserted in its place. In one embodiment, the gpl9k and 6.7K genes are deleted and the heterologous nucleic acid encoding GM-CSF is inserted into a gpl9k/6.7K-deleted E3 region. In related aspects, the full E3 region is deleted from the replication-competent adenovirus backbone and the heterologous nucleic acid GM-CSF is inserted in place of the full E3 region.

The recombinant adenovirus of the present disclosure includes a promoter functional in animal cells, *e.g.,* mammalian cells, such as, human cells. Suitable promoters include promoters derived from mammalian virus and promoters derived from the genome of mammalian cells, such as CMV (Cytomegalovirus) promoter, a chicken beta actin promoter, human β-actin / CMV hybrid promoter, chicken β-actin / CMV hybrid promoter, CMV-actin-globin (CAG) hybrid promoter, Math Promoter, VGLUT3 promoter, parvalbumin promoter, calretinin promoter, calbindin 28k promoter, prestin promoter, a liver-specific or liver-preferential promoter, *e.g.,* albumin promoter, U6 promoter, H1 promoter, MLV (Murine Leukemia Virus) LTR (Long terminal repeat) promoter, adenovirus early promoter, adenovirus late promoter, vaccinia virus 7.5K promoter, SV40 promoter, HSV *tk* promoter, RSV promoter, EF1 alpha promoter, metallothionine promoter, beta-actin promoter, human IL -2 promoter of gene, promoter of human IFN gene, promoter of human IL-4 gene, promoter of human lymphotoxin gene, promoter of human GM-CSF gene, inducible promoter, cancer cell-specific promoter (*e.g.,* TERT promoter, modified TERT Promoter, PSA promoter, PSMA promoter, CEA promoter, Survivin promoter, E2F promoter, modified E2F promoter, AFP promoter, modified AFP promoter, E2F-AFP hybrid promoter, and E2F-TERT hybrid promoter) and tissue-specific promoters (*e.g.,* albumin promoter), human phosphoglycerate kinase (PGK) promoter, mouse phosphoglycerate kinase (PGK) promoter, etc. In certain aspects, two different promoters for controlling expression of GM-CSF and sTGF-B-RII are used. For example, a CMV promoter may be used to drive sTGF-B-RII expression and a E1B promoter may drive expression of GM-CSF. A polyadenylation sequence may be linked downstream of a nucleic acid encoding GM-CSF and/or sTGF-B-RII. The polyadenylation sequence may be a bovine growth hormone terminator, a polyadenylation sequence derived from SV40, HIV-1 polyA, β-globin polyA, HSV TK polyA, or polyomavirus polyA, etc.

In certain instances, the recombinant vector may be an Ad5 vector or Ad2 vector. Adenovirus produced using Ad5 vector can transduce cell expressing Coxsackie-Adenovirus Receptor (CAR). Ad5 vector may include Ad5 ITRs. The absence or the presence of low levels of the coxsackievirus and adenovirus receptor (CAR) on several tumor types can limit the efficacy of the oncolytic adenovirus. Various peptide motifs may be added to the fiber knob, for instance an RGD motif (RGD sequences mimic the normal ligands of cell surface integrins), Tat motif, polylysine motif, NGR motif, CTT motif, CNGRL motif, CPRECES motif or a strept-tag motif. A motif can be inserted into the H1 loop of the adenovirus fiber protein. Modifying the capsid allows CAR-independent target cell infection. Specific receptors found exclusively or preferentially on the surface of cancer cells may be used as a target for adenoviral binding and infection, such as EGFRvIII.

In certain aspects, one or both of sTGF-BRII and GM-CSF may be a fusion protein where a heterologous protein is fused to the amino acid sequence of sTGF-BRII and/or GM-CSF at either the N-terminus or the C-terminus. For example, sTGF-BRII and/or GM-CSF may be fused to a heterologous protein that increases its serum half-life and/or increases solubility. Such heterologous proteins that increase serum half-life include the Fc region of immunoglobulins, *e.g.,* human IgG1, human serum albumin, etc. Fusion to maltose binding protein may be used to increase solubility of sTGF-BRII and/or GM-CSF. In other examples, only sTGF-BRII may be expressed as a fusion protein, where the heterologous protein comprises a half-life-extending moiety. The half-life-extending moiety may be a Fc region of a human IgG1. As used herein, Fc region refers to the Fc region found in immunoglobulins and variants thereof. Variants can include sequences that have deletions, insertions, or substitutions or other modifications, such as modified glycosylation that retain the half-life-extending effect associated with the naturally occurring Fc region of an immunoglobulin.

The sTGF-BRII protein may be encoded by a nucleic acid comprising a polynucleotide sequence from any source, *e.g.,* a cDNA or a synthetic sequence. The sTGF-BRII protein may include only the extracellular region of the TGF-BRII such that the receptor is secreted from cells expressing the receptor. The sTGF-BRII binds to the ligands for TGF-BRII but do not activate TGF-B signaling, thus decreasing TGF-B signaling. In certain aspects, the subject to whom the oncolytic virus is administered is a mammal and the polynucleotide sequence encoding sTGF-BRII protein is the TGF-BRII cDNA sequence from the mammal. In certain aspects, the subject may be human and the sTGF-BRII may be a human sTGF-BRII. The human sTGF-BRII may have an amino acid sequence that is at least 80%, 85%, 90%, 95%, 96%, 97%, 98%, or 99% or a 100% identical to the amino acid sequence set forth in SEQ ID NO:1. In some instances, the human sTGF-BRII may include amino acid differences from SEQ ID NO:1, such as deletions, insertions, or substitutions. The amino acid differences may be such that they do not significantly affect binding to TGF-BRII ligands, such as, TGF-B1, TGF-B2, and/or TGF-B3. The sequence of SEQ ID NO:1 is as follows:

MGRGLLRGLWPLHIVLWTRIASTIPPHVQKSVNNDMIVTDNNGAVKFPQ
LCKFCDVRFSTCDNQKSCMSNCSITSICEKPQEVCVAVWRKNDENITLETVCHDPKLPY
HDFILEDAASPKCIMKEKKKPGETFFMCSCSSDECNDNIIFS EEYNTSNPD.

The nucleic acid encoding GM-CSF may be from any source and may encode a functionally active variant (*e.g.,* fragment or mutant) of GM-CSF. In certain examples, the GM-CSF is secreted from cells infected with the oncolytic virus. In certain aspects, the subject to whom the oncolytic virus is administered is a mammal and the GM-CSF protein sequence matches (*e.g.* is identical to) the GM-CSF protein sequence from the mammal. In certain aspects, the subject may be a human and the GM-CSF may be a human GM-CSF. The human GM-CSF may have an amino acid sequence that is at least 80%, 85%, 90%, 95%, 96%, 97%, 98%, or 99% or a 100% identical to the amino acid sequence set forth in SEQ ID NO:2. In some instances, the human GM-CSF may include amino acid differences from SEQ ID NO:2, such as deletions, insertions, or substitutions. The amino acid differences may be such that they do not significantly affect GM-CSF activity.

In certain aspects, the Fc region fused to the N-terminus and/or the C-terminus of GM-CSF or sTGF-BRII may include an amino acid sequence at least 80%, 85%, 90%, 95%, 96%, 97%, 98%, or 99% or a 100% identical to the amino acid sequence set forth in SEQ ID NO:3. In some instances, the Fc region may include amino acid differences from SEQ ID NO:3, such as deletions, insertions, or substitutions. The amino acid differences may be such that they do not significantly affect the half-life-extending property of the Fc region. The sequence of SEQ ID NO:3 is as follows:

MDPKSCDKTHTCPPCPAPELLGGPSVFLFPPKPKDTLMISR
TPEVTCVVVDVSHEDPEVKFNWYVDGVEVHNAKTKPREEQYNSTYRVVSV
LTVLHQDWLNGKEYKCKVSTKALPAPIEKTISKAKGQPREPQVYTLPPSR
DELTKNQVSLTCLVKGFYPSDIAVEWESNGQPENNYKATPPVLDSDGSFF
LYSKLTVDKSRWQQGNVFSCSVMHEALHNHYTQKSLSLSPGK.

In certain instances, the oncolytic virus comprises a nucleic acid encoding a human GM-CSF and a sTGF-BRIIFc. sTGF-BRIIFc may have an amino acid sequence at least 80%, 85%, 90%, 95%, 96%, 97%, 98%, or 99% or a 100% identical to the amino acid sequence set forth in SEQ ID NO:6.

### Compositions comprising subject oncolytic virus

Also provided herein are compositions, such as pharmaceutical compositions comprising a therapeutically effective amount of the oncolytic virus disclosed herein and a pharmaceutically acceptable excipient, diluent, or carrier.

The compositions of the invention may comprise an oncolytic virus alone, or in combination with one or more other viruses (*e.g.,* a second oncolytic virus comprising a nucleic acid encoding one or more different therapeutic genes). In some embodiments, a composition comprises 1, 2, 3, 4, 5, 6, 7, 8, 9, 10, or more different oncolytic viruses, each having one or more different therapeutic genes. In certain aspects, the composition may include an additional therapeutic agent for treatment of cancer.

Suitable carriers may be readily selected by one of skill in the art in view of the type of cancer for which the oncolytic virus is administered. For example, one suitable carrier includes saline, which may be formulated with a variety of buffering solutions (*e.g.,* phosphate-buffered saline). Other exemplary carriers include sterile saline, lactose, sucrose, calcium phosphate, gelatin, dextran, agar, pectin, peanut oil, sesame oil, and water. Optionally, the compositions of the present disclosure may contain, in addition to the virus and carrier(s), other conventional pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

In some embodiments, the oncolytic virus compositions are formulated to reduce aggregation of viral particles in the composition, particularly where high virus concentrations are present (*e.g.,* about 10¹³ genome copies "GC"/ml or more). Methods for reducing aggregation of viral particles are well known in the art and include, for example, addition of surfactants, pH adjustment, and salt concentration adjustment.

Formulation of pharmaceutically acceptable excipients and carrier solutions is well-known to those of skill in the art, as is the development of suitable dosing and treatment regimens for using the particular compositions described herein in a variety of treatment regimens.

Typically, these formulations may contain at least about 0.1% of the oncolytic virus or more, although the percentage of the oncolytic virus may, of course, be varied and may conveniently be between about 1 about 80% (*e.g.,* between about 2-70%) or more of the weight or volume of the total formulation. Naturally, the amount of oncolytic virus in each therapeutically useful composition may be prepared is such a way that a suitable dosage will be obtained in any given unit dose of the oncolytic virus. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

The pharmaceutical forms suitable for injectable use include sterile aqueous solutions or dispersions and sterile powders for the extemporaneous preparation of sterile injectable solutions or dispersions. Dispersions may also be prepared in glycerol, liquid polyethylene glycols, and mixtures thereof and in oils. Under ordinary conditions of storage and use, these preparations contain a preservative to prevent the growth of microorganisms. The carrier can be a solvent or dispersion medium containing, for example, water, ethanol, polyol (*e.g.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

For administration of an injectable aqueous solution, for example, the solution may be suitably buffered, if necessary, and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art. For example, one dosage may be dissolved in 1 ml of isotonic NaCl solution and either added to 1000 ml of hypodermoclysis fluid or injected at the proposed site of infusion. Some variation in dosage will necessarily occur depending on the condition of the recipient.

Sterile injectable solutions are prepared by incorporating the active virions in the required amount in the appropriate solvent with various of the other ingredients enumerated herein, as required, followed by filtered sterilization. The viral compositions disclosed herein may also be formulated in a neutral or salt form. Pharmaceutically acceptable salts include the acid addition salts (formed with the free amino groups of the protein), which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms, such as injectable solutions, drug-release capsules, and the like.

Delivery vehicles such as liposomes, nanocapsules, microparticles, microspheres, lipid particles, vesicles, and the like, may be used for the introduction of the compositions of the present invention into target cells.

### TREATMENT METHODS

Treatment methods comprise administering to a subject having cancer a therapeutically effective amount of the oncolytic virus described in the present application. The administering may include a single administration, or alternatively comprises a series of administrations. For example, the oncolytic virus described herein may be administered at least once daily, once weekly, or once monthly over a period of two or more months. As another example, the oncolytic virus is administered once every 2, 3, or 4 weeks for 4 cycles. The length of the treatment period depends on a variety of factors, such as the severity of the disease, the age of the patient, the concentration, the activity of the oncolytic virus described herein, and/or a combination thereof. It will also be appreciated that the effective dosage used for the treatment or prophylaxis may increase or decrease over the course of a particular treatment or prophylaxis regimen. Changes in dosage may result and become apparent by standard diagnostic assays known in the art. In some instances, chronic administration may be required. In addition, the treatment regimen may change when another cancer therapy is administered.

The dosage administered will vary depending on the use and known factors such as the pharmacodynamic characteristics of the particular substance, and its mode and route of administration, age, health, and weight of the individual recipient, nature and extent of symptoms, kind of concurrent treatment, frequency of treatment, and the effect desired. Dosage regimen may be adjusted to provide the optimum therapeutic response.

A "therapeutically effective amount" will fall in a relatively broad range that can be determined through experimentation and/or clinical trials. For example, a therapeutically effective dose can be on the order of from about 10⁶ to about 10¹⁵ of the virus particles, *e.g.,* from about 10⁸ to 10¹² viral genomes (vg), from about 10⁶ vg to about 10¹⁵ vg of the oncolytic virus, *e.g.,* from about 10⁸ vg to 10¹² vg. Other effective dosages can be readily established by one of ordinary skill in the art through routine trials establishing dose-response curves.

In some embodiments, more than one administration (*e.g.,* two, three, four or more administrations) may be employed to achieve the desired level of gene expression. In some cases, the more than one administration is administered at various intervals, *e.g.,* daily, weekly, twice monthly, monthly, every 3 months, every 6 months, yearly, etc. In some cases, multiple administrations are administered over a period of time from 1 month to 2 months, from 2 months to 4 months, from 4 months to 8 months, from 8 months to 12 months, from 1 year to 2 years, from 2 years to 5 years, or more than 5 years.

In certain aspects, the oncolytic virus provided herein may be administered sequentially or contemporaneously with at least one other anti-cancer therapy. The anti-cancer therapy may be an immunotherapy, a chemotherapy, a surgical treatment, a radiation therapy, a hormone therapy, an anti-cancer small molecule, a growth factor inhibitor therapy, or a cytokine therapy.

The type of cancer that can be treated in a subject by administering the oncolytic virus of the present disclosure may be a primary cancer or a metastasized tumor. In certain embodiment, the cancer is breast cancer (*e.g.,* triple negative breast cancer), skin cancer (*e.g.,* melanoma), head or neck cancer, astrocytoma, hepatic cancer, lymphoma (*e.g.,* lymphocytic lymphoma, primary central nervous system lymphoma, or pediatric lymphoma), colon cancer, gastric cancer, lung cancer, non-small cell lung cancer, ovarian cancer, liver cancer, bronchial cancer, nasopharyngeal cancer, laryngeal cancer, pancreatic cancer, bladder cancer, cervical cancer, bone cancer, non-small cell cancer, bone cancer, blood cancer, uterine cancer, rectal cancer, cancer near the anus, fallopian tube cancer, endometrial cancer, vaginal cancer, vulvar cancer, Hodgkin's disease, esophageal cancer, small intestine cancer, endocrine adenocarcinoma, Thyroid cancer, parathyroid cancer, adrenal cancer, soft tissue sarcoma, urethral cancer, penile cancer, prostate cancer, chronic or acute leukemia, renal or ureteral cancer, renal cell carcinoma, renal pelvic carcinoma, salivary gland cancer, sarcoma cancer, pseudomyxoma, hepatoblastoma, testicular cancer, glioblastoma, cleft lip cancer, ovarian germ cell tumor, basal cell carcinoma, multiple myeloma, gallbladder cancer, choroidal melanoma, barter bulge cancer, peritoneal cancer, tongue cancer, small cell carcinoma, neuroblastoma, duodenal cancer, ureter cancer, meningioma, renal pelvis cancer, vulvar cancer, thymic cancer, central nervous system (CNS) tumor, spinal cord tumor, brain stem glioma, and pituitary adenoma. The cancer may be a recurrent cancer.

### Recombinant Oncolytic Virus Administration Methods

The oncolytic virus provided herein may be delivered to a subject according to any appropriate methods known in the art. The virus, may be suspended in a physiologically compatible carrier or a pharmaceutically acceptable excipient or diluent (*i.e.,* in a composition), may be administered to a subject, such as, for example, a human, mouse, rat, cat, dog, sheep, rabbit, horse, cow, goat, pig, guinea pig, hamster, chicken, turkey, or a non-human primate (*e.g.,* Macaque).

The virus is administered in sufficient amounts to transfect the target cells, *e.g.,* solid tumor cells, and to provide sufficient levels of gene transfer and expression without undue off-target effects. Conventional and pharmaceutically acceptable routes of administration include, but are not limited to, intravenous, direct delivery (*e.g.,* by injection) into a tumor, direct delivery to the selected organ (*e.g.,* intraportal or intrahepatic delivery to the liver, delivery to brain via a cannula), oral, inhalation (including intranasal and intratracheal delivery), intraocular, intramuscular, subcutaneous, intradermal, intrathecal, and other parenteral routes of administration. In certain aspects the administration may be into the brain, *e.g.,* intraparenchymal, intracerebroventricular, intra cisterna magna or subpial. Routes of administration may be combined, if desired.

Moreover, in certain instances, it may be desirable to deliver the virions to the CNS of a subject. By "CNS" is meant all cells and tissue of the brain and spinal cord of a vertebrate. Thus, the term includes, but is not limited to, neuronal cells, glial cells, astrocytes, cerebrospinal fluid (CSF), interstitial spaces, bone, cartilage and the like. Recombinant oncolytic virus may be delivered directly to the CNS or brain by injection into, *e.g.,* the ventricular region, as well as to the striatum (*e.g.,* the caudate nucleus or putamen of the striatum), spinal cord and neuromuscular junction, or cerebellar lobule, with a needle, catheter or related device, using neurosurgical techniques known in the art, such as by stereotactic injection.

In a further aspect, the present invention provides a virion of the present invention or a pharmaceutical composition of the present invention for use as a medicament.

In a further aspect, the present invention provides an oncolytic virus of the present invention or a pharmaceutical composition of the present invention for use in a method of treating cancer in a subject in need thereof.

The dose of an oncolytic virus required to achieve a particular "therapeutic effect," *e.g.,* the units of dose in genome copies/per kilogram of body weight (GC/kg), will vary based on several factors including, but not limited to: the route of administration, the level of gene expression required to achieve a therapeutic effect, the type of cancer being treated, and the like.

An effective amount of an oncolytic virus is an amount sufficient to infect a desired number of cancer cells. The effective amount will depend primarily on factors such as the species, age, weight, health of the subject, and the size/location of the tumor, and may thus vary among animal and tissue. For example, an effective amount of an oncolytic virus is generally in the range of from about 1 ml to about 100 ml of solution containing from about 10⁹ to 10¹⁶ genome copies. In some cases, a dosage between about 10¹¹ to 10¹² genome copies is appropriate. In certain embodiments, 10¹² viral genome copies is effective to target brain, liver, and pancreatic tissues. In certain embodiments, the dosage of the oncolytic virus is 10¹⁰, 10¹¹, 10¹², 10¹³, or 10¹⁴ genome copies per kg. In certain embodiments, the dosage of the oncolytic virus is 10¹⁰, 10¹¹, 10¹², 10¹¹, 10¹⁴, or 10¹⁵ genome copies per subject.

In certain aspects, a therapeutically effective dose of an oncolytic virus of the present disclosure provides a level and/or activity of GM-CSF in the subject that is at least 10% higher than the normal GM-CSF level and/or activity, respectively (*e.g.,* at least 20%, at least 30%, at least 40%, at least 45%, at least 50%, or higher than the normal GM-CSF level). The GM-CSF level and/or activity may be determined using a blood sample or part thereof, *e.g.,* serum or plasma from a subject who has been administered the virus. In certain aspects, a therapeutically effective dose of an oncolytic virus of the present disclosure provides a reduction in TGF-B activity that is at least 10% less *(e.g.,* at least 20%, at least 30%, at least 40%, at least 45%, at least 50%, or even lower than the normal TGF-B activity). The TGF-B activity may be determined using a blood sample or part thereof, *e.g.,* serum or plasma from a subject who has been administered the virus.

### Combination Therapy

In certain aspects, the treatment of a subject having cancer with an oncolytic virus comprising a nucleic acid encoding a sTGF-BRII and a GM-CSF as disclosed herein may or may not involve another anti-cancer therapy, such as, an immunotherapy, a chemotherapy, a surgical treatment, a radiation therapy, a hormone therapy, an anti-cancer small molecule, a growth factor inhibitor therapy, CAR-T therapy, or a cytokine therapy.

In some instances, a subject may be treated systemically, including with the subject oncolytic virus, with or without one or more additional reagents. By "systemic treatment", as used herein, is meant a treatment that is not directed solely to target a specific tumor (such as, *e.g.,* a primary tumor or a defined secondary tumor) or a specific cancer containing tissue (such as, *e.g.,* the liver in the case of liver cancer, the blood in the case of a blood cancer). Systemic treatments will generally be directed to the subject's body as a whole and may include, but are not limited to, *e.g.,* systemic radiation therapy, systemic chemotherapy, systemic immunotherapy, combinations thereof and the like.

In some instances, a subject may be treated locally, including with the subject oncolytic virus, with or without one or more additional reagents. By "local treatment", as used herein, is meant a treatment that is specifically directed to the location of a tumor (such as, *e.g.,* a primary tumor or a defined secondary tumor) or specifically directed to a cancer containing tissue (such as, *e.g.,* the liver in the case of liver cancer, the blood in the case of a blood cancer). In some instances, local treatment may also be administered in such a way as to affect the environment surrounding a tumor, such as tissue surrounding the tumor, such as tissue immediately adjacent to the tumor. Local treatment will generally not affect or not be targeted to tissues distant from the site of cancer including the site of a tumor, such as a primary tumor. Useful local treatments that may be administered in addition to, or in combination with, a subject oncolytic virus, *e.g.,* include but are not limited to surgery, local radiation therapy, local cryotherapy, local laser therapy, local topical therapy, combinations thereof, and the like.

Radiation therapy includes, but is not limited to, x-rays or gamma rays that are delivered from either an externally applied source such as a beam, or by implantation of small radioactive sources.

Suitable antibodies for use in cancer treatment include, but are not limited to, naked antibodies, *e.g.,* trastuzumab (Herceptin) , bevacizumab (Avastin^{™}), cetuximab (Erbitux^{™}), panitumumab (Vectibix^{™}), Ipilimumab (Yervoy^{™}), rituximab (Rituxan), alemtuzumab (Lemtrada^{™}), Ofatumumab (Arzerra^{™}), Oregovomab (OvaRex^{™}), Lambrolizumab (MK-3475), pertuzumab (Perjeta^{™}), ranibizumab (Lucentis^{™}), etc., and conjugated antibodies, *e.g.,* gemtuzumab ozogamicin (Mylortarg^{™}), Brentuximab vedotin (Adcetris^{™}), 90Y-labelled ibritumomab tiuxetan (Zevalin^{™}), 131I-labelled tositumoma (Bexxar^{™}), etc.

Suitable antibodies for use in cancer treatment also include, but are not limited to, antibodies raised against tumor-associated antigens. Such antigens include, but are not limited to, CD20, CD30, CD33, CD52, EpCAM, CEA, gpA33, Mucins, TAG-72, CAIX, PSMA, Folate-binding protein, Gangliosides (*e.g.,* GD2, GD3, GM2), Ley, VEGF, VEGFR, Integrin alpha-V-beta-3, Integrin alpha-5-beta-1, EGFR, ERBB2, ERBB3, MET, IGF1R, EPHA3, TRAILR1, TRAILR2, RANKL, FAP, Tenascin, Programmed Death-Ligand 1 (PD-L1), androgen receptor (AR), Bruton's Tyrosine Kinase (BTK), BCR-Abl, c-kit, PIK3CA, EML4-ALK, KRAS, ALK, ROS1, AKT1, BRAF, MEKJ, MEK2, NRAS, RAC1, ESR1, etc.

Biological response modifiers suitable for use in connection with the methods of the present disclosure include, but are not limited to, (1) inhibitors of tyrosine kinase (RTK) activity; (2) inhibitors of serine/threonine kinase activity; (3) tumor-associated antigen antagonists, such as antibodies that bind specifically to a tumor antigen; ( 4) apoptosis receptor agonists; (5) interleukin-2; (6) interferon-α.; (7) interferon-γ; (8) inhibitors of angiogenesis; and (9) antagonists of tumor necrosis factor.

Chemotherapeutic agents or antineoplastic agents are non-peptidic (*i.e.*, non-proteinaceous) compounds that reduce proliferation of cancer cells and/or kill cancer cells, and encompass cytotoxic agents and cytostatic agents. Non-limiting examples of chemotherapeutic agents include alkylating agents (*e.g.,* nitrosoureas), antimetabolites (*e.g.,* methotrexate), antitumor antibiotics (*e.g.,* anthracyclins), plant alkaloids (*e.g.,* vinca alkaloids, taxanes), toposiomerase inhibitors, and steroid hormones.

Agents that act to reduce cellular proliferation are known in the art and widely used. Such agents include alkylating agents, such as nitrogen mustards, nitrosoureas, ethylenimine derivatives, alkyl sulfonates, and triazenes, including, but not limited to, mechlorethamine, cyclophosphamide (Cytoxan^{™}), melphalan (L-sarcolysin), carmustine (BCNU), lomustine (CCNU), semustine (methyl-CCNU), streptozocin, chlorozotocin, uracil mustard, chlormethine, ifosfamide, chlorambucil, pipobroman, triethylenemelamine, triethylenethiophosphoramine, busulfan, dacarbazine, and temozolomide.

Antimetabolite agents include folic acid analogs, pyrimidine analogs, purine analogs, and adenosine deaminase inhibitors, including, but not limited to, cytarabine (CYTOSAR-U), cytosine arabinoside, fluorouracil (5-FU), floxuridine (FudR), 6-thioguanine, 6-mercaptopurine (6-MP), pentostatin, 5-fluorouracil (5-FU), methotrexate, 10-propargyl-5,8-dideazafolate (PDDF, CB3717), 5,8-dideazatetrahydrofolic acid (DDATHF), leucovorin, fludarabine phosphate, pentostatine, and gemcitabine.

Hormone modulators and steroids (including synthetic analogs) that are suitable for use include, but are not limited to, adrenocorticosteroids, *e.g.,* prednisone, dexamethasone; estrogens and pregestins, *e.g.,* hydroxyprogesterone caproate, medroxyprogesterone acetate, megestrol acetate, estradiol, clomiphene, tamoxifen, and adrenocortical suppressants, *e.g.,* aminoglutethimide; 17α-ethinylestradiol; diethylstilbestrol, testosterone, fluoxymesterone, dromostanolone propionate, testolactone, methylprednisolone, methyl-testosterone, prednisolone, triamcinolone, chlorotrianisene, hydroxyprogesterone, aminoglutethimide, estramustine, medroxyprogesterone acetate, leuprolide, Flutamide (Drogenil), Toremifene (Fareston), and Zoladex. Estrogens stimulate proliferation and differentiation, therefore compounds that bind to the estrogen receptor are used to block this activity. Corticosteroids may inhibit T cell proliferation.

Immune checkpoint inhibitor suitable for use include a PD-1 (programmed cell death-1) antagonist, a PD-L1 (programmed cell death-ligand 1) antagonist, a PD-L2 (programmed cell death-ligand 2) antagonist, a CD27 (cluster of differentiation 27) antagonist, CD28 (cluster of differentiation 28) antagonist, CD70 (cluster of differentiation 70) antagonist, CD80 (cluster of differentiation 80, also known as B7-1) antagonist, CD86 (cluster of differentiation 86, also known as B7-2) antagonist, CD137 (cluster of differentiation 137) antagonist, CD276 (cluster of differentiation 276) antagonist, KIRs (killer-cell immunoglobulin-like receptors) antagonist, LAG3 (lymphocyte-activation gene 3) antagonist, TNFRSF4 (tumor necrosis factor receptor superfamily, member 4, also known as CD 134) antagonist, GITR (glucocorticoid-induced TNFR-related protein) antagonist, GITRL (glucocorticoid-induced TNFR-related protein ligand) antagonist, 4-1BBL (4-1BB ligand) antagonist, CTLA-4 ( cytolytic T lymphocyte associated antign-4) antagonist, A2AR (Adenosine A2A receptor) antagonist, VTCN1 (V-set domain-containing T-cell activation inhibitor 1) antagonist, BTLA (B- and T-lymphocyte attenuator) antagonist, IDO (Indoleamine 2,3-dioxygenase) antagonist, TIM-3 (T-cell Immunoglobulin domain and Mucin domain 3) antagonist, VISTA (V-domain Ig suppressor of T cell activation) antagonist, KLRA (killer cell lectin- like receptor subfamily A) antagonists, and the like.

The immune checkpoint inhibitor may be an antibody. Examples of antibodies to checkpoint inhibitor molecules include Nivolumab (BMS-936558, MDX- 1 106, ONO-4538), a fully human Immunoglobulin G4 (lgG4) monoclonal PD-1 antibody; Lambrolizumab (MK-3475), a humanized monoclonal IgG4 PD-1 antibody, BMS-936559, a fully human IgG4 PD-L1 antibody, LAG-3 antagonistic antibodies such as relatlimab (BMS-986016; Bristol-Myers Squibb), IMP701 (Immutep), TSR-033 (anti-LAG-3 mAb; TESARO, Inc.).

In certain aspects, a subject suitable for treatment using a method of the present disclosure include an individual having a cancer; an individual diagnosed as having a cancer; an individual being treated for a cancer with chemotherapy, radiation therapy, antibody therapy, surgery, etc.; an individual who has been treated for a cancer (*e.g.,* with one or more of chemotherapy, radiation therapy, antibody therapy, surgery), and who has failed to respond to the treatment; an individual who has been treated for a cancer (*e.g.,* with one or more of chemotherapy, radiation therapy, antibody therapy, surgery), and who initially responded to the treatment but who subsequently relapsed, *i.e.,* the cancer recurred.

The methods of the present disclosure may be employed to target and treat a variety of cancers, including, *e.g.,* primary cancer, secondary cancers, re-growing cancers, recurrent cancers, refractory cancers and the like. For example, in some instances, the methods of the present disclosure may be employed as an initial treatment of a primary cancer identified in a subject. In some instances, the methods of the present disclosure may be employed as a non-primary *(e.g.,* secondary or later) treatment, *e.g.,* in a subject with a cancer that is refractory to a prior treatment, in a subject with a cancer that is re-growing following a prior treatment, in a subject with a mixed response to a prior treatment (*e.g.,* a positive response to at least one tumor in the subject and a negative or neutral response to at least a second tumor in the subject), and the like.

The methods of treating described herein may, in some instances, be performed in a subject that has previously undergone one or more conventional treatments. For example, the methods described herein may, in some instances, be performed following a conventional cancer therapy including, but not limited to, *e.g.,* conventional chemotherapy, conventional radiation therapy, conventional immunotherapy, or surgery. In some instances, the methods described herein may be used when a subject has not responded to, or is refractory to, a conventional therapy. In some instances, the methods described herein may be used when a subject has responded to a conventional therapy.

Two, three, or more of the treatments described herein may be combined in any order for treating cancer in a subject, which treatments may be administered simultaneously or contemporaneously. Such treatments include a dual-therapy and a triple-therapy.

### KITS

Aspects of the present disclosure also include kits. The kits may include, *e.g.,* any combination of the oncolytic virus, reagents, compositions, formulations, cells, nucleic acids, viral vectors, or the like, described herein. A subject kit can include one or more of: an oncolytic virus, an oncolytic viral vector, a cell line for producing the oncolytic virus. Kits may be configured for various purposes, including, *e.g.,* treatment kits *(e.g.,* where a kit may include a dose of an oncolytic virus), kits for the oncolytic virus, kits comprising antibodies for detecting expression of GM-CSF and/or sTGF-BRII, and the like.

Optional components of the kit will vary and may include, *e.g.,* a buffer; or a bacterial growth inhibitor. Where a subject kit comprises a subject nucleic acid, the nucleic acid may also have restriction sites, multiple cloning sites, primer sites, etc. and the kit may include restriction enzymes, primers, etc.

The various components of the kit may be present in separate containers or certain compatible components may be pre-combined into a single container, as desired.

In addition to the above-mentioned components, a subject kit can include instructions for using the components of the kit to practice a subject method. The instructions for practicing a subject method are generally recorded on a suitable recording medium. For example, the instructions may be printed on a substrate, such as paper or plastic, etc. As such, the instructions may be present in the kits as a package insert, in the labeling of the container of the kit or components thereof (*i.e.,* associated with the packaging or subpackaging) etc. In other embodiments, the instructions are present as an electronic storage data file present on a suitable computer-readable storage medium, *e.g.,* compact disc-read only memory (CD-ROM), digital versatile disk (DVD), diskette, etc. In yet other embodiments, the actual instructions are not present in the kit, but means for obtaining the instructions from a remote source, *e.g.,* via the internet, are provided. An example of this embodiment is a kit that includes a web address where the instructions can be viewed and/or from which the instructions can be downloaded. As with the instructions, this means for obtaining the instructions is recorded on a suitable substrate.

### EXEMPLARY NON-LIMITING ASPECTS OF THE DISCLOSURE

Aspects, including embodiments, of the present subject matter described above may be beneficial alone or in combination with one or more other aspects or embodiments.

### EXAMPLES

As can be appreciated from the disclosure provided above, the present disclosure has a wide variety of applications. Accordingly, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Those of skill in the art will readily recognize a variety of noncritical parameters that could be changed or modified to yield essentially similar results. Thus, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (*e.g.,* amounts, dimensions) but some experimental errors and deviations should be accounted for.

### EXAMPLE 1: RAD.ST.GM INHIBITS 4T1 TUMOR GROWTH IN IMMUNE COMPETENT BALB/C MICE

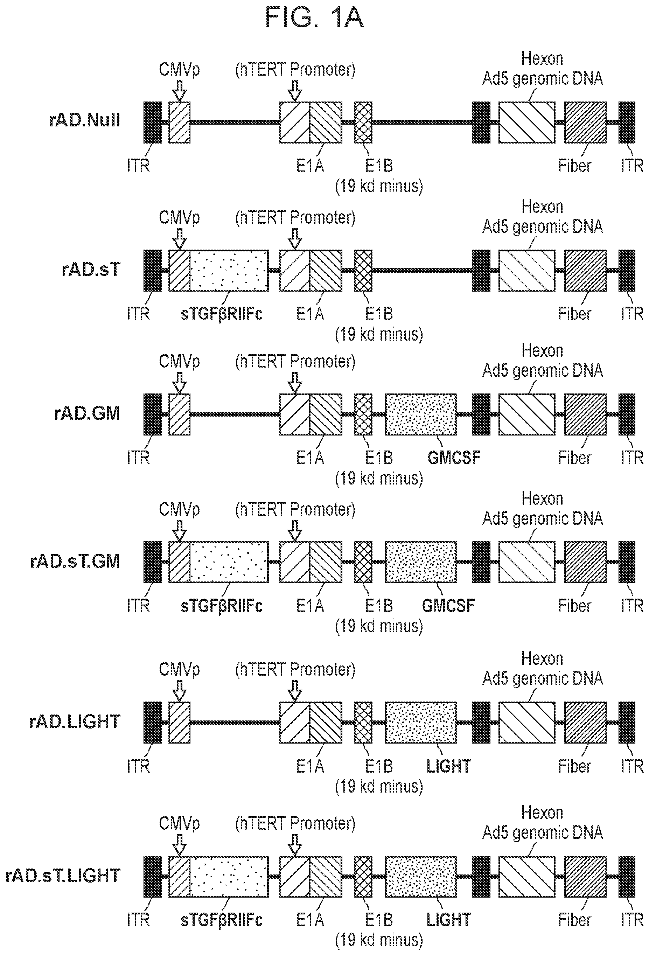

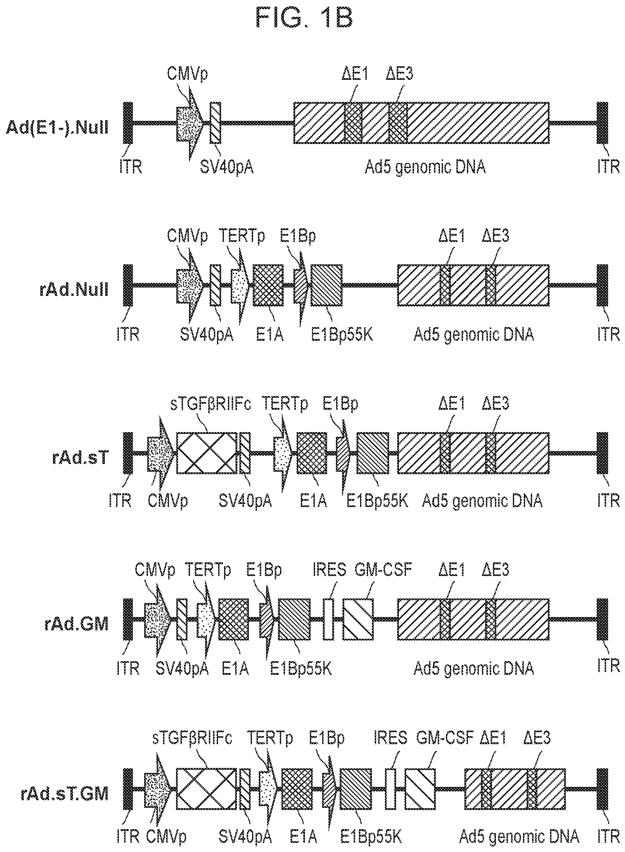

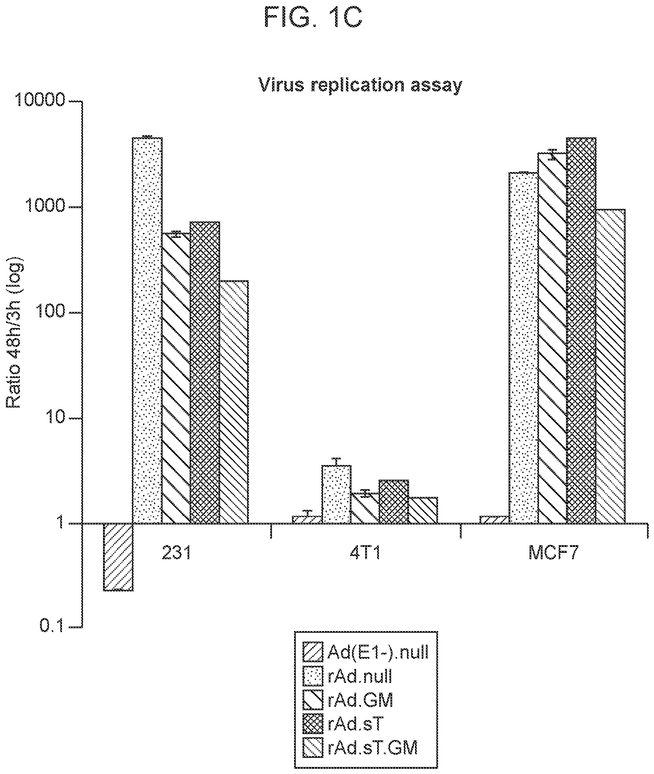

**Methods.** To establish a mouse mammary tumor syngeneic mouse model, 2 × 10⁶ 4T1 cells per mouse were injected subcutaneously (day 0) into the dorsal right flank of female BALB/c mice (4-6 weeks old). Mice were monitored every day. On day 6, tumor dimensions were measured (in mm) using a caliper. Then, tumor-bearing mice were divided into seven groups without statistical differences between each group. The adenoviral vectors diagrammed in Fig. 1 were tested. rAD.sT.GM vector expressed both soluble TGFbRIIFc and GM-CSF proteins, with CMV promoter (CMVp) controlling TGFbRIIFc expression while expression of GM-CSF is under the control of adenoviral E1B promoter (E1Bp). rAd.GM vector only expresses GM-CSF, under the control of adenoviral E1B promoter. rAd.sT vector only expresses soluble TGFbRIIFc, under the control of CMV promoter. rAd.null vector served as control. In each of the vectors, the expression of the E1A gene is under the control of the human TERT promoter to provide for selective replication of the virus in cancer cells. Adenovirus were produced that included rAD.sT.GM vector, rAd. GM vector, rAd.sT vector, or rAd.null vector. The adenoviruses were injected directly into the tumors (5x10¹⁰ viral particles/per injection, each injection 100µl volume). Two injections were administered on each of days 7 and 9. Tumor volumes were measured over time and calculated by the following formula: tumor volume = (width² × length)/2.

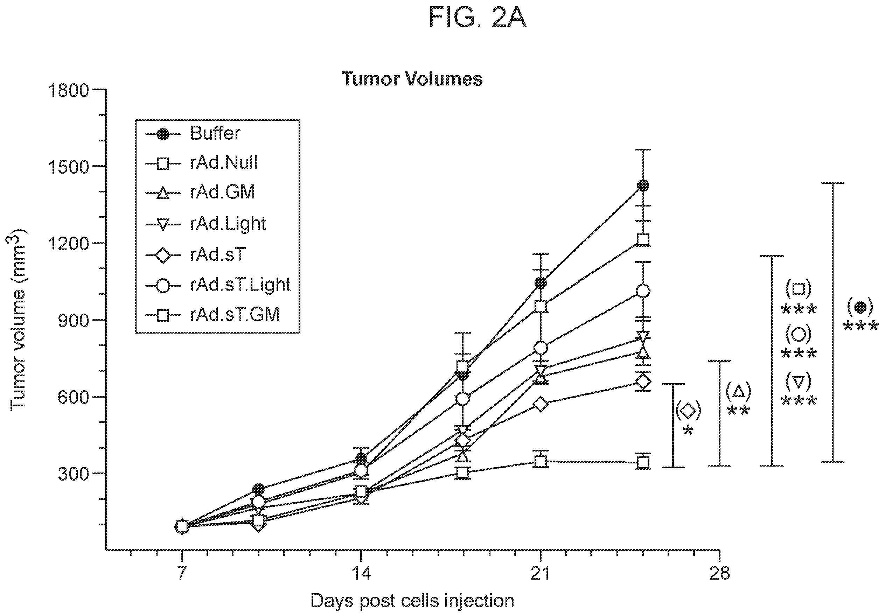

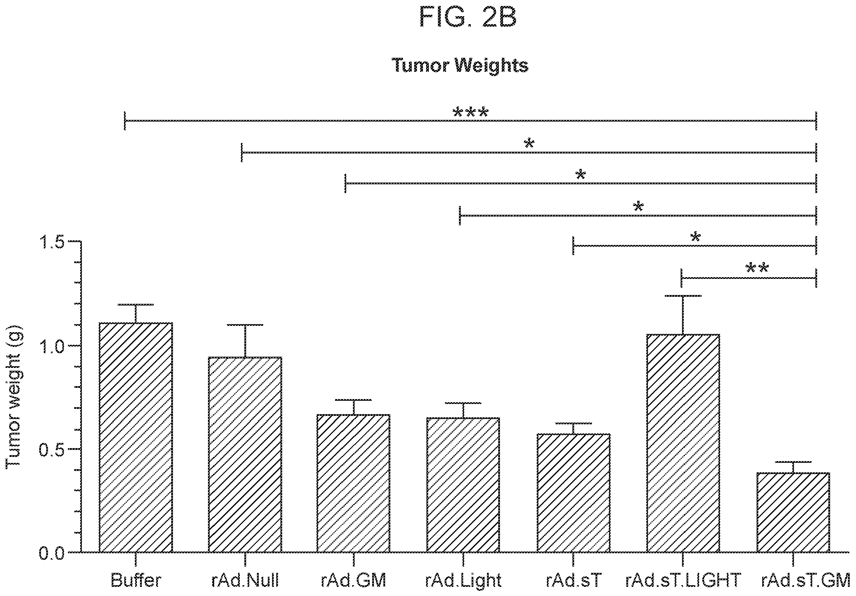

The tumor volumes were monitored on various days shown in Fig. 2. Animals were euthanized on day 25, tumors were removed, and tumor weights were measured.

**Results.** The results indicated that while the tumors treated with buffer grew over time, the intratumoral injection of all the adenoviruses inhibited tumor growth. Among the adenoviruses tested, rAd.sT.GM produced the strongest inhibition of the tumor growth. (Fig. 2A). Importantly, rAd.sT.GM produced inhibition of tumor growth which was statistically superior to all other viruses tested (Fig. 2A). Most notable is the superior response of rAd.sT.GM compared to both the rAd.GM (p<0.01) and rAd.sT (p<0.05), indicating a synergism between TGFbRIIFc and GM-CSF. * represents P < 0.05, ** represents P < 0.01, and *** represents P < 0.001.

On day 25, mice were euthanized, and tumors were removed and weighed. Again, among all the treatment groups, rAd.sT.GM was most effective in reducing the tumor weights (Fig. 2B). * represents P < 0.05, ** represents P < 0.01, and *** represents P < 0.001.

These results show that the direct injection of rAd.sT.GM virus into mammary tumors produces strong inhibition of tumor growth.

rAd.LIGHT adenovirus results in expression of LIGHT (also known as tumor-necrosis factor (TNF) superfamily member 14 (TNFSF14)). rAd.LIGHT adenovirus inhibits tumor growth via activation of anti-tumor immune responses (Dai et al., Cancer Gene Therapy 27, 923-933 (2020)). rAd.LIGHT reduced 4T1 mouse mammary tumor growth and tumor weight. However, the adenovirus expressing both LIGHT and sTGFbRIIFc, *i.e.,* rAd.sT.LIGHT, was less effective in inhibiting tumor growth and volume compared to rAd. LIGHT and rAd.sT alone. This result shows the unpredictability of effect of combining two molecules, LIGHT and sTGFbRIIFc, that are each individually effective in treating cancer. This unpredictability makes the discovery of the combined synergistic effect of sTGFbRIIFc with GM-CSF for treating cancers even more important, since it provides for a significant improvement in treatment of cancer.

**Cell lines and adenoviruses.** The mouse mammary tumor cell line, 4T1, was purchased from ATCC. 4T1 cells were cultured in Eagle's Minimal Essential Medium (EMEM) plus 10% fetal calf serum (FCS). Human embryonic kidney cells, HEK293 (ATCC) were cultured in Dulbecco's Minimal Essential Medium (DMEM) supplement with 10% FCS. All adenoviruses were grown in HEK293 cells and purified by double cesium chloride gradients.

**Animal studies.** All procedures for animal experiments were approved by the Institutional Animal Care and Use Committee (IACUC) at NorthShore University Health System.

**Statistical analysis.** Data are presented as mean±s.e.m. All statistical analyses were performed using GraphPad Prism software version 5 (GraphPad software, San Diego, CA, USA). Longitudinal data were analyzed by using a two-way repeated measure ANOVA followed by Bonferroni *post hoc* tests for the tumor growth data obtained over-time. One-way ANOVA followed by Bonferroni *post hoc* tests were performed to analyze other data. Differences were considered significant at two-sided p<0.05.

rAd.sT.GM was constructed by using a modified system that have three gene expression units: CMVp-controlled sTGFβRIIFc, TERTp-controlled E1A and E1Bp-controlled GM-CSF that also harbors an IRES-linked E1B55K. Three control replicating adenoviruses: rAd.GM, rAd.sT and rAd.Null are devoid of either sTGFβRIIFc or GM-CSF, or both. Nonreplicating adenoviruse Ad(E1-).Null was constructed by the Ad-easy System.

### Vector Construction.

### Construction of Ad-GM-RE:

1. Enzyme digestion: pShuttle-cmv was digested by MfeI and dephosphorylated. Then, the larger fragment was recovered. TE-TP-GM (dIRES) was digested with MfeI, and then the larger fragment was also obtained.
2. The fragments from step 1 were ligated with T4 DNA Ligase, transformed into *E. coli* DH5α, and selected by Kanamycin.
3. Picked up more than 24 clones, and grown in LB overnight (37°C, 250rpm).
4. Plasmids were extracted using conventional techniques.
5. Identification by enzyme digestion. Using pshuttle-cmv-TGFβRIIFc as control. NotI (GC'GGCC_GC) [CciNI] cuts 2 times at positions 966, 1257 generating fragment sizes 291, 10693. Control, NotI (GC'GGCC_GC) [CciNI] cuts 1 time at position 966. One clone identified as containing correctly inserted GM-CSF, named "pShuttle-cmv-GM-RE (dIRES)".
6. Grew the correct clone from the second batch, and obtained more plasmids.
7. pShuttle-cmv-GM-RE (dIRES) was digested with PmeI and Dephosphorylated.
8. Linear plasmid was obtained by using ethanol precipitation.
9. 10µl linear plasmid was transformed into BJ5183-Adeasy competent cells by using Electroporation (2.5KV).
10. 12 clones were picked from the plates and grown overnight (37°C, 250rpm).
11. Extraction of the plasmids and identification by using PacI digestion. 7 clones were correctly inserted and were named "Ad -GM-RE (dIRES)".
12. Transformed the plasmids from #2 clone into *E. coli* DH5α and then extracted the plasmids for transfection.
13. Identification of the plasmids by using PacI, again.
14. 173µl plasmid Ad -GM-RE (dIRES) was cut by PacI (7µl), and dephosphorylated. Then, the PacI linear Ad -GM-RE (dIRES) was recovered by using ethanol precipitation. Total volume was 35µl.
15. Transfection:

Day0: HEK293 cells were seeded into 6-well plate at a density of 5x10⁶ cells/well. (one 10cm plate could be seeded into 15 wells).

Day1: Transfection- the PacI lined Ad -GM-RE (dIRES) was transfected into HEK293 cells according to the protocol. (Two wells, 5µl and 10µl respectively.) The culture medium was replaced by 5% FBS DMEM (2ml) 6 hours after transfection.

Day4: The culture medium was replaced by fresh 5% FBS DMEM (2ml).

Day7: 2ml fresh 5% FBS DMEM (2ml) were added to each well.

Day8-10: Plaques were observed.

Day10: 2ml fresh 5% FBS DMEM (2ml) were added to each well.

Day12-Day16: Adenovirus (rAd.GM) was collected and purified.

### Construction of Ad-TGFβRIIFc-RE:

1. Enzyme digestion:
   pShuttle-cmv-TGFβRIIFc-GM-RE (dIRES) was digested by SpeI and Dephosphorylated. Then, the larger fragment was recovered. TE-TP-E1A was digested with MfeI, and then the larger fragment was obtained.
2. The fragments from step1 were recombined in BJ5183 bacteria.
3. 12 clones were picked and grown in LB overnight (37°C, 250rpm).
4. Plasmids were extracted using conventional techniques.
5. Identification by enzyme digestion. pshuttle-cmv-TGFβRIIFc-GM-RE dIRES was used as a control. KpnI (G_GTAC'C) [neoschizomers Acc65I,Asp718I] cuts 2 times at positions 2203 and 4186, generating fragment sizes 1983 and 9810 bp. Control, KpnI (G_GTAC'C) [neoschizomers Acc65I,Asp718I] cuts 2 times, at positions 2203 and 4623, generating fragment sizes 2420 9810 bp. SalI (G'TCGA_C) cuts 3 times, at positions 2193, 2205, and 4129, generating fragment sizes of 12, 1924, and 9857 bp. Control, SalI (G'TCGA_C) cuts 3 times, at positions 2193, 2205, and 4566, generating fragment sizes of 12, 2361, and 9857 bp. All clones were correctly inserted and named "pshuttle-cmv-TGFbetaRIIFc-RE".
6. Grew the correct clone #11, and obtained more plasmids.
7. pShuttle-cmv-TGFbetaRIIFc-RE was digested with PmeI and dephosphorylated.
8. Linear plasmid was obtained by using ethanol precipitation.
9. 10µl linear plasmid was transformed into BJ5183-Adeasy competent cells by using Electroporation (2.5KV).
10. 12 clones were picked from the plates and grown overnight (37°C, 250rpm).
11. The plasmids were extracted and identified using PacI digestion. One clone was correct and was named "Ad-TGFbetaRIIFc-RE".
12. Transformed the plasmids from #6 clone into *E. coli* DH5α and then extracted the plasmids for transfection.
13. Plasmids were again identified using PacI, and also by PCR amplification.
14. 173µl plasmid Ad-TGFbetaRIIFc-RE was cut by PacI (7µl), and dephosphorylated. Then, the PacI linearized Ad-TGFbetaRIIFc-RE was recovered using ethanol precipitation. Total volume was 35µl.
15. Transfection:

Day0: HEK293 cells were seeded into 6-well plates at a density of 5x10° cells/well. (one 10cm plate could be seeded into 15 wells).

Day1: Transfection the PacI linearized Ad-TGFbetaRIIFc-RE into HEK293 cells according to the protocol. (Two wells, 5µl and 10µl respectively.) The culture medium was replaced by 5% FBS DMEM (2ml) 6 hours after transfection.

Day4: The culture medium was replaced by fresh 5% FBS DMEM (2ml).

Day7: 2ml fresh 5% FBS DMEM (2ml) were added to each well.

Day8-10: Plaques were observed.

Day10: 2ml fresh 5% FBS DMEM (2ml) were added to each well.

Day12-Day16: Adenovirus (rAd.sT) was collected and purified.

### Construction of Ad-Null-RE:

### Method1:

1. Enzyme digestion:
   pShuttle-cmv-TGFβRIIFc-RE was digested by XhoI, and the larger fragment was recovered.
2. The fragment was self-ligated by using T4DNA Ligase.

### Method2:

1. Enzyme digestion:
   pShuttle-cmv-GM-RE (dIRES) was digested by SpeI and dephosphorylated. Then, the fragment was recovered. TE-TP-E1A was digested by MfeI, and dephosphorylated. Then, the larger fragment was recovered.
2. The fragments from step1 were recombined in BJ5183 bacteria.
3. 12 clones were picked from each method, and grown in LB overnight (37°C, 250rpm).
4. Plasmids were extracted (#1-6 clones were chosen from a total of 12 clones).
5. Plasmids were identified by enzyme digestionusing pshuttle-cmv-E1Bp-Light-RE and pshuttle-cmv-TGFβRIIFc-RE as controls. XhoI cuts 1 time. One band for both methods, control (pshuttle-cmv-TGFβRIIFc-RE). XhoI cuts 2 times producing 2 bands; one band is 1267 bp. Control (pshuttle-cmv-E1Bp-Light-RE), XhoI cuts 1 time producing 1 band. KpnI (G_GTAC'C) [neoschizomers Acc65I,Asp718I] for recombination methods cuts 2 times, at positions 957 and 2940, generating fragment sizes of 1983 and 8564 bp. For XhoI digestion and self-ligation method, there is only one band. Control, pshuttle-cmv-TGFβRIIFc-RE, KpnI (G_GTAC'C) [neoschizomers Acc65I,Asp718I] cuts 2 times, at positions 2203 and 4186, generating fragment sizes of 1983 and 9810 bp. pshuttle-cmv-E1Bp-Light-RE, KpnI (G_GTAC'C) [neoschizomers Acc65I,Asp718I] cuts 3 times, at positions 957, 4367, and 4564, generating fragment sizes of 3410, 197, and 8564 bp. Correct cloanes were named "pshuttle-cmv-Null-RE".
6. Grew the correct clone #6 and obtained more plasmid.
7. pShuttle-cmv-Null-RE was digested with PmeI and dephosphorylated.
8. Linear plasmid was obtained using ethanol precipitation.
9. 10µl linearized plasmid was transformed into BJ5183-Adeasy competent cells by using Electroporation (2.5KV).
10. 12 clones were picked from the plates and grown overnight (37°C, 250rpm).
11. Plasmids were extracted and identified using PacI digestion and PCR amplification. Correct clones were named "Ad-Null-RE".
12. Transformed the plasmids from #6 clone into *E. coli* DH5α and then plasmids were extracted for transfection.
13. Plasmids were identified using PacI, again.
14. 173µl plasmid Ad-Null-RE was cut by PacI (7µl), and dephosphorylated. Then, the PacI linearized Ad-Null-RE was recovered by using ethanol precipitation. Total volume was 35µl.
15. Transfection:

Day0: HEK293 cells were seeded into 6-well plates at a density of 5x10° cells/well.

(One 10cm plate could be seeded into 15 wells.)

Day1: ThePacI-linearized Ad-Null-RE was transfected into HEK293 cells according to the protocol. (Two wells, 5µl and 10µl respectively.) The culture medium was replaced by 5% FBS DMEM (2ml) 6 hours after transfection.

Day4: The culture medium was replaced by fresh 5% FBS DMEM (2ml).

Day7: 2ml fresh 5% FBS DMEM (2ml) was added to each well.

Day8-10: Plaques were observed.

Day10: Fresh 5% FBS DMEM (2ml) was added to each well.

Day12-Day16: Adenovirus (rAd.Null) was collected and purified.

### Construction of Ad-TGFβRIIFc-GM-RE

1. Enzyme digestion:
   pShuttle-cmv-TGFβRIIFc-RE was digested by SpeI and dephosphorylated. Then, the larger fragment was recovered. TE-PPT-GM was digested with MfeI and EcoRI to get the 4.5kb fragment.
2. The fragments from step1 were recombined in BJ5183 bacteria.
3. 12 clones were picked and grown in LB overnight (37°C, 250rpm).
4. Plasmid extraction.
5. Identification by enzyme digestion. pshuttle-cmv-TGFβRIIFc-RE was used as a control. BglII (A'GATC_T) cuts 3 times, at positions 947, 4782, and 6813, generating fragment sizes of 3835, 2031, and 7278 bp. Control, BglII (A'GATC_T) cuts 2 times, at positions 947 and 5462, generating fragment sizes of 4515 and 7278 bp. ScaI+SpeI double digests yielded fragments of 488 bp and 12656 bp. Control, ScaI+SpeI yielded a fragment of 11793 bp. Correct clones were named "pshuttle-cmv-TGFbetaRIIFc-RE".
6. Correct clone #1 was grown and more plasmid was obtained.
7. pShuttle-cmv-TGFbetaRIIFc-GM-RE was digested with PmeI and dephosphorylated.
8. Linearized plasmid was obtained by using ethanol precipitation.
9. 10µl linearized plasmid was transformed into BJ5183-Adeasy competent cells using Electroporation (2.5KV).
10. 12 clones were picked from the plates and grown overnight (37°C, 250rpm).
11. Plasmids were extracted and identified using PacI digestion. Correct clones were named "Ad-TGFbetaRIIFc-GM-RE".

### Construction of rAd.sT.GM

12. Plasmids from #12 Ad-TGFbetaRIIFc-GM-RE clone were amplified in E. *coli* DH5α and then the plasmids were extracted for transfection.
13. Plasmids were identified by using PacI again, and by PCR amplification.
14. 173µl plasmid Ad-TGFbetaRIIFc-GM-RE was cut by PacI (7µl), and dephosphorylated. Then, the PacI-linearized Ad-TGFbetaRIIFc-GM-RE was recovered by ethanol precipitation. Total volume was 35µl.
15. Transfection:

Day0: HEK293 cells were seeded into 6-well plate at a density of 5x10° cells/well.

(One 10cm plate could be seeded into 15 wells.)

Day1: PacI-linearized Ad-TGFbetaRIIFc-GM-RE was transfected into HEK293 cells according to the protocol. (Two wells, 5µl and 10µl respectively.) The culture medium was replaced by 5% FBS DMEM (2ml) 6 hours after transfection.

Day4: The culture medium was replaced by fresh 5% FBS DMEM (2ml).

Day7: Fresh 5% FBS DMEM (2ml) was added to each well.

Day8-10: The plaques were observed.

Day10: Fresh 5% FBS DMEM (2ml) was added to each well.

Day12-Day16: Adenovirus (rAd.sT.GM) was collected and purified.

### Sequences:

**Complete genome sequence of rAd.sT.GM:** 1 TCCCTTCCAG CTCTCTGCCC CTTTTGGATT GAAGCCAATA TGATAATGAG 
51 GGGGTGGAGT TTGTGACGTG GCGCGGGGCG TGGGAACGGG GCGGGTGACG
101 TAGTAGTGTG GCGGAAGTGT GATGTTGCAA GTGTGGCGGA ACACATGTAA
151 GCGACGGATG TGGCAAAAGT GACGTTTTTG GTGTGCGCCG GTGTACACAG
201 GAAGTGACAA TTTTCGCGCG GTTTTAGGCG GATGTTGTAG TAAATTTGGG
251 CGTAACCGAG TAAGATTTGG CCATTTTCGC GGGAAAACTG AATAAGAGGA
301 AGTGAAATCT GAATAATTTT GTGTTACTCA TAGCGCGTAA NNNNTAATAG
351 TAATCAATTA CGGGGTCATT AGTTCATAGC CCATATATGG AGTTCCGCGT
401 TACATAACTT ACGGTAAATG GCCCGCCTGG CTGACCGCCC AACGACCCCC
451 GCCCATTGAC GTCAATAATG ACGTATGTTC CCATAGTAAC GCCAATAGGG
501 ACTTTCCATT GACGTCAATG GGTGGAGTAT TTACGGTAAA CTGCCCACTT
551 GGCAGTACAT CAAGTGTATC ATATGCCAAG TACGCCCCCT ATTGACGTCA
601 ATGACGGTAA ATGGCCCGCC TGGCATTATG CCCAGTACAT GACCTTATGG
651 GACTTTCCTA CTTGGCAGTA CATCTACGTA TTAGTCATCG CTATTACCAT
701 GGTGATGCGG TTTTGGCAGT ACATCAATGG GCGTGGATAG CGGTTTGACT
751 CACGGGGATT TCCAAGTCTC CACCCCATTG ACGTCAATGG GAGTTTGTTT
801 TGGCACCAAA ATCAACGGGA CTTTCCAAAA TGTCGTAACA ACTCCGCCCC
851 ATTGACGCAA ATGGGCGGTA GGCGTGTACG GTGGGAGGTC TATATAAGCA
901 GAGCTGGTTT AGTGAACCGT CAGATCCGCT AGAGATCTCG AGCTCAAGCT
951 TACCTGCCAT GGGTCGGGGG CTGCTCAGGG GCCTGTGGCC GCTGCACATC
1001 GTCCTGTGGA CGCGTATCGC CAGCACGATC CCACCGCACG TTCAGAAGTC
1051 GGTTAATAAC GACATGATAG TCACTGACAA CAACGGTGCA GTCAAGTTTC
1101 CACAACTGTG TAAATTTTGT GATGTGAGAT TTTCCACCTG TGACAACCAG
1151 AAATCCTGCA TGAGCAACTG CAGCATCACC TCCATCTGTG AGAAGCCACA
1201 GGAAGTCTGT GTGGCTGTAT GGAGAAAGAA TGACGAGAAC ATAACACTAG
1251 AGACAGTTTG CCATGACCCC AAGCTCCCCT ACCATGACTT TATTCTGGAA
1301 GATGCTGCTT CTCCAAAGTG CATTATGAAG GAAAAAAAAA AGCCTGGTGA
1351 GACTTTCTTC ATGTGTTCCT GTAGCTCTGA TGAGTGCAAT GACAACATCA
1401 TCTTCTCAGA AGAATATAAC ACCAGCAATC CTGACATGGA TCCCAAATCT
1451 TGTGACAAAA CTCACACATG CCCACCGTGC CCAGCACCTG AACTCCTGGG
1501 GGGACCGTCA GTCTTCCTCT TCCCCCCAAA ACCCAAGGAC ACCCTCATGA
1551 TCTCCCGGAC CCCTGAGGTC ACATGCGTGG TGGTGGACGT GAGCCACGAA
1601 GACCCTGAGG TCAAGTTCAA CTGGTACGTG GACGGCGTGG AGGTGCATAA
1651 TGCCAAGACA AAGCCGCGGG AGGAGCAGTA CAACAGCACG TACCGTGTGG
1701 TCAGCGTCCT CACCGTCCTG CACCAGGACT GGCTGAATGG CAAGGAGTAC
1751 AAGTGCAAGG TATCCACCAA AGCCCTCCCA GCCCCCATCG AGAAAACCAT
1801 CTCCAAAGCC AAAGGGCAGC CCCGAGAACC ACAGGTGTAC ACCCTGCCCC
1851 CATCCCGGGA TGAGCTGACC AAGAACCAGG TCAGCCTGAC CTGCCTAGTC
1901 AAAGGCTTCT ATCCCAGCGA CATCGCCGTG GAGTGGGAGA GCAATGGGCA
 
1951 GCCGGAGAAC AACTACAAGG CCACGCCTCC CGTGCTGGAC TCCGACGGCT
2001 CCTTCTTCCT CTACAGCAAG CTCACCGTGG ACAAGAGCAG GTGGCAGCAG
2051 GGGAACGTCT TCTCATGCTC CGTGATGCAT GAGGCTCTGC ACAACCACTA
2101 CACGCAGAAG AGCCTCTCCC TGTCTCCGGG TAAATAAAGG CGCGCCAGTA
2151 TTGGATACTA GAGTCGAGCA TGCATCTAGT CGACGGTACC GTCGACGCGG
2201 CCGCTCGAGC CTAAGCTTCT AGATAAGATA TCCGATCCAC CGGATCTAGA
2251 TAACTGATCA TAATCAGCCA TACCACATTT GTAGAGGTTT TACTTGCTTT
2301 AAAAAACCTC CCACACCTCC CCCTGAACCT GAAACATAAA ATGAATGCAA
2351 TTGTTGTTGT TAACTTGTTT ATTGCAGCTT ATAATGGTTA CAAATAAAGC
2401 AATAGCATCA CAAATTTCAC AAATAAAGCA TTTTTTTCAC TGCATTCTAG
2451 TTGTGGTTTG TCCAAACTCA TCAATGTATC TTAACGCGCG GCCGCTGGCC
2501 CCTCCCTCGG GTTACCCCAC AGCTTAGGCC GATTCGACCT CTCTCCGCTG
2551 GGGCCCTCGC TGGCGTCCCT GCACCCTGGG AGCGCGAGCG GCGCGCGGGC
2601 GGGGAAGCGC GGCCCAGACC CCCGGGTCCG CCCGGAGCAG CTGCGCTGTC
2651 GGGGCCAGGC CGGGCTCCCA GTGGATTCGC GGGCACAGAC GCCCAGGACC
2701 GCGCTTCCCA CGTGGCGGAG GGACTGGGGA CCCGGGCACC CGTCCTGCCC
2751 CTTCACCTTC CAGCTCCGCC TCCTCCGCGC GGACCCCGCC CCGTCCCGAC
2801 CCCTCCCGGG TCCCCGGCCC AGCCCCCTCC GGGCCCTCCC AGCCCCTCCC
2851 CTTCCTTTCC GCGGCCCCGC CCTCTCCTCG CGGCGCGAGT TTCAGGCAGC
2901 GCTGCGTCCT GCTGCGCACG TGGGAAGCCC TGGCCCCGGC CACCCCCGCG
2951 GAATTCATGA GACATATTAT CTGCCACGGA GGTGTTATTA CCGAAGAAAT
3001 GGCCGCCAGT CTTTTGGACC AGCTGATCGA AGAGGTACTG GCTGATAATC
3051 TTCCACCTCC TAGCCATTTT GAACCACCTA CCCTTCACGA ACTGTATGAT
3101 TTAGACGTGA CGGCCCCCGA AGATCCCAAC GAGGAGGCGG TTTCGCAGAT
3151 TTTTCCCGAC TCTGTAATGT TGGCGGTGCA GGAAGGGATT GACTTACTCA
3201 CTTTTCCGCC GGCGCCCGGT TCTCCGGAGC CGCCTCACCT TTCCCGGCAG
3251 CCCGAGCAGC CGGAGCAGAG AGCCTTGGGT CCGGTTTCTA TGCCAAACCT
3301 TGTACCGGAG GTGATCGATC TTACCTGCCA CGAGGCTGGC TTTCCACCCA
3351 GTGACGACGA GGATGAAGAG GGTGAGGAGT TTGTGTTAGA TTATGTGGAG
3401 CACCCCGGGC ACGGTTGCAG GTCTTGTCAT TATCACCGGA GGAATACGGG
3451 GGACCCAGAT ATTATGTGTT CGCTTTGCTA TATGAGGACC TGTGGCATGT
3501 TTGTCTACAG TAAGTGAAAA TTATGGGCAG TGGGTGATAG AGTGGTGGGT
3551 TTGGTGTGGT AATTTTTTTT TTAATTTTTA CAGTTTTGTG GTTTAAAGAA
3601 TTTTGTATTG TGATTTTTTT AAAAGGTCCT GTGTCTGAAC CTGAGCCTGA
3651 GCCCGAGCCA GAACCGGAGC CTGCAAGACC TACCCGCCGT CCTAAAATGG
3701 CGCCTGCTAT CCTGAGACGC CCGACATCAC CTGTGTCTAG AGAATGCAAT
3751 AGTAGTACGG ATAGCTGTGA CTCCGGTCCT TCTAACACAC CTCCTGAGAT
3801 ACACCCGGTG GTCCCGCTGT GCCCCATTAA ACCAGTTGCC GTGAGAGTTG
 
3851 GTGGGCGTCG CCAGGCTGTG GAATGTATCG AGGACTTGCT TAACGAGCCT
3901 GGGCAACCTT TGGACTTGAG CTGTAAACGC CCCAGGCCAT AAGGTGTAAA
3951 CCTGTGATTG CGTGTGTGGT TAACGCCTTT GTTTGCTGAA TGAGTTGATG
4001 TAAGTTTAAT AAAGGGTGAG ATAATGTTTA ACTTGCATGG CGTGTTAAAT
4051 GGGGCGGGGC TTAAAGGGTA TATAATGCGC CGTGGGCTAA TCTTGGTTAC
4101 ATCTGACCTC ACTAGTATGT GGCTGCAGAG CCTGCTGCTC TTGGGCACTG
4151 TGGCCTGCAG CATCTCTGCA CCCGCCCGCT CGCCCAGCCC CAGCACGCAG
4201 CCCTGGGAGC ATGTGAATGC CATCCAGGAG GCCCGGCGTC TCCTGAACCT
4251 GAGTAGAGAC ACTGCTGCTG AGATGAATGA AACAGTAGAA GTCATCTCAG
4301 AAATGTTTGA CCTCCAGGAG CCGACCTGCC TACAGACCCG CCTGGAGCTG
4351 TACAAGCAGG GCCTGCGGGG CAGCCTCACC AAGCTCAAGG GCCCCTTGAC
4401 CATGATGGCC AGCCACTACA AGCAGCACTG CCCTCCAACC CCGGAAACTT
4451 CCTGTGCAAC CCAGATTATC ACCTTTGAAA GTTTCAAAGA GAACCTGAAG
4501 GACTTTCTGC TTGTCATCCC CTTTGACTGC TGGGAGCCAG TCCAGGAGTG
4551 AGTCGACGAA TTAATTCGCT GTCTGCGAGG GCCAGCTGTT GGGGTGAGTA
4601 CTCCCTCTCA AAAGCGGGCA TGACTTCTGC GCTAAGATTG TCAGTTTCCA
4651 AAAACGAGGA GGATTTGATA TTCACCTGGC CCGCGGTGAT GCCTTTGAGG
4701 GTGGCCGCGT CCATCTGGTC AGAAAAGACA ATCTTTTTGT TGTCAAGCTT
4751 GAGGTGTGGC AGGCTTGAGA TCTGGCCATA CACTTGAGTG ACAATGACAT
4801 CCACTTTGCC TTTCTCTCCA CAGGTGTCCA CTCCCAGGTC CAACTGCAGG
4851 TCGAGCATGC ATCTAGGGCG GCCAATTCCG CCCCTCTCCC TCCCCCCCCC
4901 CTAACGTTAC TGGCCGAAGC CGCTTGGAAT AAGGCCGGTG TGCGTTTGTC
4951 TATATGTGAT TTTCCACCAT ATTGCCGTCT TTTGGCAATG TGAGGGCCCG
5001 GAAACCTGGC CCTGTCTTCT TGACGAGCAT TCCTAGGGGT CTTTCCCCTC
5051 TCGCCAAAGG AATGCAAGGT CTGTTGAATG TCGTGAAGGA AGCAGTTCCT
5101 CTGGAAGCTT CTTGAAGACA AACAACGTCT GTAGCGACCC TTTGCAGGCA
5151 GCGGAACCCC CCACCTGGCG ACAGGTGCCT CTGCGGCCAA AAGCCACGTG
5201 TATAAGATAC ACCTGCAAAG GCGGCACAAC CCCAGTGCCA CGTTGTGAGT
5251 TGGATAGTTG TGGAAAGAGT CAAATGGCTC TCCTCAAGCG TATTCAACAA
5301 GGGGCTGAAG GATGCCCAGA AGGTACCCCA TTGTATGGGA TCTGATCTGG
5351 GGCCTCGGTG CACATGCTTT ACATGTGTTT AGTCGAGGTT AAAAAAACGT
5401 CTAGGCCCCC CGAACCACGG GGACGTGGTT TTCCTTTGAA AAACACGATG
5451 ATAAGCTTGC CACAACTCGA CGAGTTTTAT AAAGGATAAA TGGAGCGAAG
5501 AAACCCATCT GAGCGGGGGG TACCTGCTGG ATTTTCTGGC CATGCATCTG
5551 TGGAGAGCGG TTGTGAGACA CAAGAATCGC CTGCTACTGT TGTCTTCCGT
5601 CCGCCCGGCG ATAATACCGA CGGAGGAGCA GCAGCAGCAG CAGGAGGAAG
5651 CCAGGCGGCG GCGGCAGGAG CAGAGCCCAT GGAACCCGAG AGCCGGCCTG
5701 GACCCTCGGG AATGAATGTT GTACAGGTGG CTGAACTGTA TCCAGAACTG
 
5751 AGACGCATTT TGACAATTAC AGAGGATGGG CAGGGGCTAA AGGGGGTAAA
5801 GAGGGAGCGG GGGGCTTGTG AGGCTACAGA GGAGGCTAGG AATCTAGCTT
5851 TTAGCTTAAT GACCAGACAC CGTCCTGAGT GTATTACTTT TCAACAGATC
5901 AAGGATAATT GCGCTAATGA GCTTGATCTG CTGGCGCAGA AGTATTCCAT
5951 AGAGCAGCTG ACCACTTACT GGCTGCAGCC AGGGGATGAT TTTGAGGAGG
6001 CTATTAGGGT ATATGCAAAG GTGGCACTTA GGCCAGATTG CAAGTACAAG
6051 ATCAGCAAAC TTGTAAATAT CAGGAATTGT TGCTACATTT CTGGGAACGG
6101 GGCCGAGGTG GAGATAGATA CGGAGGATAG GGTGGCCTTT AGATGTAGCA
6151 TGATAAATAT GTGGCCGGGG GTGCTTGGCA TGGACGGGGT GGTTATTATG
6201 AATGTAAGGT TTACTGGCCC CAATTTTAGC GGTACGGTTT TCCTGGCCAA
6251 TACCAACCTT ATCCTACACG GTGTAAGCTT CTATGGGTTT AACAATACCT
6301 GTGTGGAAGC CTGGACCGAT GTAAGGGTTC GGGGCTGTGC CTTTTACTGC
6351 TGCTGGAAGG GGGTGGTGTG TCGCCCCAAA AGCAGGGCTT CAATTAAGAA
6401 ATGCCTCTTT GAAAGGTGTA CCTTGGGTAT CCTGTCTGAG GGTAACTCCA
6451 GGGTGCGCCA CAATGTGGCC TCCGACTGTG GTTGCTTCAT GCTAGTGAAA
6501 AGCGTGGCTG TGATTAAGCA TAACATGGTA TGTGGCAACT GCGAGGACAG
6551 GGCCTCTCAG ATGCTGACCT GCTCGGACGG CAACTGTCAC CTGCTGAAGA
6601 CCATTCACGT AGCCAGCCAC TCTCGCAAGG CCTGGCCAGT GTTTGAGCAT
6651 AACATACTGA CCCGCTGTTC CTTGCATTTG GGTAACAGGA GGGGGGTGTT
6701 CCTACCTTAC CAATGCAATT TGAGTCACAC TAAGATATTG CTTGAGCCCG
6751 AGAGCATGTC CAAGGTGAAC CTGAACGGGG TGTTTGACAT GACCATGAAG
6801 ATCTGGAAGG TGCTGAGGTA CGATGAGACC CGCACCAGGT GCAGACCCTG
6851 CGAGTGTGGC GGTAAACATA TTAGGAACCA GCCTGTGATG CTGGATGTGA
6901 CCGAGGAGCT GAGGCCCGAT CACTTGGTGC TGGCCTGCAC CCGCGCTGAG
6951 TTTGGCTCTA GCGATGAAGA TACAGATTGA GGTACTGAAA TGTGTGGGCG
7001 TGGCTTAAGG GTGGGAAAGA ATATATAAGG TGGGGGTCTT ATGTAGTTTT
7051 GTATCTGTTT TGCAGCAGCC GCCGCCGCCA TGAGCACCAA CTCGTTTGAT
7101 GGAAGCATTG TGAGCTCATA TTTGACAACG CGCATGCCCC CATGGGCCGG
7151 GGTGCGTCAG AATGTGATGG GCTCCAGCAT TGATGGTCGC CCCGTCCTGC
7201 CCGCAAACTC TACTACCTTG ACCTACGAGA CCGTGTCTGG AACGCCGTTG
7251 GAGACTGCAG CCTCCGCCGC CGCTTCAGCC GCTGCAGCCA CCGCCCGCGG
7301 GATTGTGACT GACTTTGCTT TCCTGAGCCC GCTTGCAAGC AGTGCAGCTT
7351 CCCGTTCATC CGCCCGCGAT GACAAGTTGA CGGCTCTTTT GGCACAATTG
7401 GATTCTTTGA CCCGGGAACT TAATGTCGTT TCTCAGCAGC TGTTGGATCT
7451 GCGCCAGCAG GTTTCTGCCC TGAAGGCTTC CTCCCCTCCC AATGCGGTTT
7501 AAAACATAAA TAAAAAACCA GACTCTGTTT GGATTTGGAT CAAGCAAGTG
7551 TCTTGCTGTC TTTATTTAGG GGTTTTGCGC GCGCGGTAGG CCCGGGACCA
7601 GCGGTCTCGG TCGTTGAGGG TCCTGTGTAT TTTTTCCAGG ACGTGGTAAA
 
7651 GGTGACTCTG GATGTTCAGA TACATGGGCA TAAGCCCGTC TCTGGGGTGG
7701 AGGTAGCACC ACTGCAGAGC TTCATGCTGC GGGGTGGTGT TGTAGATGAT
7751 CCAGTCGTAG CAGGAGCGCT GGGCGTGGTG CCTAAAAATG TCTTTCAGTA
7801 GCAAGCTGAT TGCCAGGGGC AGGCCCTTGG TGTAAGTGTT TACAAAGCGG
7851 TTAAGCTGGG ATGGGTGCAT ACGTGGGGAT ATGAGATGCA TCTTGGACTG
7901 TATTTTTAGG TTGGCTATGT TCCCAGCCAT ATCCCTCCGG GGATTCATGT
7951 TGTGCAGAAC CACCAGCACA GTGTATCCGG TGCACTTGGG AAATTTGTCA
8001 TGTAGCTTAG AAGGAAATGC GTGGAAGAAC TTGGAGACGC CCTTGTGACC
8051 TCCAAGATTT TCCATGCATT CGTCCATAAT GATGGCAATG GGCCCACGGG
8101 CGGCGGCCTG GGCGAAGATA TTTCTGGGAT CACTAACGTC ATAGTTGTGT
8151 TCCAGGATGA GATCGTCATA GGCCATTTTT ACAAAGCGCG GGCGGAGGGT
8201 GCCAGACTGC GGTATAATGG TTCCATCCGG CCCAGGGGCG TAGTTACCCT
8251 CACAGATTTG CATTTCCCAC GCTTTGAGTT CAGATGGGGG GATCATGTCT
8301 ACCTGCGGGG CGATGAAGAA AACGGTTTCC GGGGTAGGGG AGATCAGCTG
8351 GGAAGAAAGC AGGTTCCTGA GCAGCTGCGA CTTACCGCAG CCGGTGGGCC
8401 CGTAAATCAC ACCTATTACC GGGTGCAACT GGTAGTTAAG AGAGCTGCAG
8451 CTGCCGTCAT CCCTGAGCAG GGGGGCCACT TCGTTAAGCA TGTCCCTGAC
8501 TCGCATGTTT TCCCTGACCA AATCCGCCAG AAGGCGCTCG CCGCCCAGCG
8551 ATAGCAGTTC TTGCAAGGAA GCAAAGTTTT TCAACGGTTT GAGACCGTCC
8601 GCCGTAGGCA TGCTTTTGAG CGTTTGACCA AGCAGTTCCA GGCGGTCCCA
8651 CAGCTCGGTC ACCTGCTCTA CGGCATCTCG ATCCAGCATA TCTCCTCGTT
8701 TCGCGGGTTG GGGCGGCTTT CGCTGTACGG CAGTAGTCGG TGCTCGTCCA
8751 GACGGGCCAG GGTCATGTCT TTCCACGGGC GCAGGGTCCT CGTCAGCGTA
8801 GTCTGGGTCA CGGTGAAGGG GTGCGCTCCG GGCTGCGCGC TGGCCAGGGT
8851 GCGCTTGAGG CTGGTCCTGC TGGTGCTGAA GCGCTGCCGG TCTTCGCCCT
8901 GCGCGTCGGC CAGGTAGCAT TTGACCATGG TGTCATAGTC CAGCCCCTCC
8951 GCGGCGTGGC CCTTGGCGCG CAGCTTGCCC TTGGAGGAGG CGCCGCACGA
9001 GGGGCAGTGC AGACTTTTGA GGGCGTAGAG CTTGGGCGCG AGAAATACCG
9051 ATTCCGGGGA GTAGGCATCC GCGCCGCAGG CCCCGCAGAC GGTCTCGCAT
9101 TCCACGAGCC AGGTGAGCTC TGGCCGTTCG GGGTCAAAAA CCAGGTTTCC
9151 CCCATGCTTT TTGATGCGTT TCTTACCTCT GGTTTCCATG AGCCGGTGTC
9201 CACGCTCGGT GACGAAAAGG CTGTCCGTGT CCCCGTATAC AGACTTGAGA
9251 GGCCTGTCCT CGAGCGGTGT TCCGCGGTCC TCCTCGTATA GAAACTCGGA
9301 CCACTCTGAG ACAAAGGCTC GCGTCCAGGC CAGCACGAAG GAGGCTAAGT
9351 GGGAGGGGTA GCGGTCGTTG TCCACTAGGG GGTCCACTCG CTCCAGGGTG
9401 TGAAGACACA TGTCGCCCTC TTCGGCATCA AGGAAGGTGA TTGGTTTGTA
9451 GGTGTAGGCC ACGTGACCGG GTGTTCCTGA AGGGGGGCTA TAAAAGGGGG
9501 TGGGGGCGCG TTCGTCCTCA CTCTCTTCCG CATCGCTGTC TGCGAGGGCC
 
9551 AGCTGTTGGG GTGAGTACTC CCTCTGAAAA GCGGGCATGA CTTCTGCGCT
9601 AAGATTGTCA GTTTCCAAAA ACGAGGAGGA TTTGATATTC ACCTGGCCCG
9651 CGGTGATGCC TTTGAGGGTG GCCGCATCCA TCTGGTCAGA AAAGACAATC
9701 TTTTTGTTGT CAAGCTTGGT GGCAAACGAC CCGTAGAGGG CGTTGGACAG
9751 CAACTTGGCG ATGGAGCGCA GGGTTTGGTT TTTGTCGCGA TCGGCGCGCT
9801 CCTTGGCCGC GATGTTTAGC TGCACGTATT CGCGCGCAAC GCACCGCCAT
9851 TCGGGAAAGA CGGTGGTGCG CTCGTCGGGC ACCAGGTGCA CGCGCCAACC
9901 GCGGTTGTGC AGGGTGACAA GGTCAACGCT GGTGGCTACC TCTCCGCGTA
9951 GGCGCTCGTT GGTCCAGCAG AGGCGGCCGC CCTTGCGCGA GCAGAATGGC
10001 GGTAGGGGGT CTAGCTGCGT CTCGTCCGGG GGGTCTGCGT CCACGGTAAA
10051 GACCCCGGGC AGCAGGCGCG CGTCGAAGTA GTCTATCTTG CATCCTTGCA
10101 AGTCTAGCGC CTGCTGCCAT GCGCGGGCGG CAAGCGCGCG CTCGTATGGG
10151 TTGAGTGGGG GACCCCATGG CATGGGGTGG GTGAGCGCGG AGGCGTACAT
10201 GCCGCAAATG TCGTAAACGT AGAGGGGCTC TCTGAGTATT CCAAGATATG
10251 TAGGGTAGCA TCTTCCACCG CGGATGCTGG CGCGCACGTA ATCGTATAGT
10301 TCGTGCGAGG GAGCGAGGAG GTCGGGACCG AGGTTGCTAC GGGCGGGCTG
10351 CTCTGCTCGG AAGACTATCT GCCTGAAGAT GGCATGTGAG TTGGATGATA
10401 TGGTTGGACG CTGGAAGACG TTGAAGCTGG CGTCTGTGAG ACCTACCGCG
10451 TCACGCACGA AGGAGGCGTA GGAGTCGCGC AGCTTGTTGA CCAGCTCGGC
10501 GGTGACCTGC ACGTCTAGGG CGCAGTAGTC CAGGGTTTCC TTGATGATGT
10551 CATACTTATC CTGTCCCTTT TTTTTCCACA GCTCGCGGTT GAGGACAAAC
10601 TCTTCGCGGT CTTTCCAGTA CTCTTGGATC GGAAACCCGT CGGCCTCCGA
10651 ACGGTAAGAG CCTAGCATGT AGAACTGGTT GACGGCCTGG TAGGCGCAGC
10701 ATCCCTTTTC TACGGGTAGC GCGTATGCCT GCGCGGCCTT CCGGAGCGAG
10751 GTGTGGGTGA GCGCAAAGGT GTCCCTGACC ATGACTTTGA GGTACTGGTA
10801 TTTGAAGTCA GTGTCGTCGC ATCCGCCCTG CTCCCAGAGC AAAAAGTCCG
10851 TGCGCTTTTT GGAACGCGGA TTTGGCAGGG CGAAGGTGAC ATCGTTGAAG
10901 AGTATCTTTC CCGCGCGAGG CATAAAGTTG CGTGTGATGC GGAAGGGTCC
10951 CGGCACCTCG GAACGGTTGT TAATTACCTG GGCGGCGAGC ACGATCTCGT
11001 CAAAGCCGTT GATGTTGTGG CCCACAATGT AAAGTTCCAA GAAGCGCGGG
11051 ATGCCCTTGA TGGAAGGCAA TTTTTTAAGT TCCTCGTAGG TGAGCTCTTC
11101 AGGGGAGCTG AGCCCGTGCT CTGAAAGGGC CCAGTCTGCA AGATGAGGGT
11151 TGGAAGCGAC GAATGAGCTC CACAGGTCAC GGGCCATTAG CATTTGCAGG
11201 TGGTCGCGAA AGGTCCTAAA CTGGCGACCT ATGGCCATTT TTTCTGGGGT
11251 GATGCAGTAG AAGGTAAGCG GGTCTTGTTC CCAGCGGTCC CATCCAAGGT
11301 TCGCGGCTAG GTCTCGCGCG GCAGTCACTA GAGGCTCATC TCCGCCGAAC
11351 TTCATGACCA GCATGAAGGG CACGAGCTGC TTCCCAAAGG CCCCCATCCA
11401 AGTATAGGTC TCTACATCGT AGGTGACAAA GAGACGCTCG GTGCGAGGAT
 
11451 GCGAGCCGAT CGGGAAGAAC TGGATCTCCC GCCACCAATT GGAGGAGTGG
11501 CTATTGATGT GGTGAAAGTA GAAGTCCCTG CGACGGGCCG AACACTCGTG
11551 CTGGCTTTTG TAAAAACGTG CGCAGTACTG GCAGCGGTGC ACGGGCTGTA
11601 CATCCTGCAC GAGGTTGACC TGACGACCGC GCACAAGGAA GCAGAGTGGG
11651 AATTTGAGCC CCTCGCCTGG CGGGTTTGGC TGGTGGTCTT CTACTTCGGC
11701 TGCTTGTCCT TGACCGTCTG GCTGCTCGAG GGGAGTTACG GTGGATCGGA
11751 CCACCACGCC GCGCGAGCCC AAAGTCCAGA TGTCCGCGCG CGGCGGTCGG
11801 AGCTTGATGA CAACATCGCG CAGATGGGAG CTGTCCATGG TCTGGAGCTC
11851 CCGCGGCGTC AGGTCAGGCG GGAGCTCCTG CAGGTTTACC TCGCATAGAC
11901 GGGTCAGGGC GCGGGCTAGA TCCAGGTGAT ACCTAATTTC CAGGGGCTGG
11951 TTGGTGGCGG CGTCGATGGC TTGCAAGAGG CCGCATCCCC GCGGCGCGAC
12001 TACGGTACCG CGCGGCGGGC GGTGGGCCGC GGGGGTGTCC TTGGATGATG
12051 CATCTAAAAG CGGTGACGCG GGCGAGCCCC CGGAGGTAGG GGGGGCTCCG
12101 GACCCGCCGG GAGAGGGGGC AGGGGCACGT CGGCGCCGCG CGCGGGCAGG
12151 AGCTGGTGCT GCGCGCGTAG GTTGCTGGCG AACGCGACGA CGCGGCGGTT
12201 GATCTCCTGA ATCTGGCGCC TCTGCGTGAA GACGACGGGC CCGGTGAGCT
12251 TGAGCCTGAA AGAGAGTTCG ACAGAATCAA TTTCGGTGTC GTTGACGGCG
12301 GCCTGGCGCA AAATCTCCTG CACGTCTCCT GAGTTGTCTT GATAGGCGAT
12351 CTCGGCCATG AACTGCTCGA TCTCTTCCTC CTGGAGATCT CCGCGTCCGG
12401 CTCGCTCCAC GGTGGCGGCG AGGTCGTTGG AAATGCGGGC CATGAGCTGC
12451 GAGAAGGCGT TGAGGCCTCC CTCGTTCCAG ACGCGGCTGT AGACCACGCC
12501 CCCTTCGGCA TCGCGGGCGC GCATGACCAC CTGCGCGAGA TTGAGCTCCA
12551 CGTGCCGGGC GAAGACGGCG TAGTTTCGCA GGCGCTGAAA GAGGTAGTTG
12601 AGGGTGGTGG CGGTGTGTTC TGCCACGAAG AAGTACATAA CCCAGCGTCG
12651 CAACGTGGAT TCGTTGATAT CCCCCAAGGC CTCAAGGCGC TCCATGGCCT
12701 CGTAGAAGTC CACGGCGAAG TTGAAAAACT GGGAGTTGCG CGCCGACACG
12751 GTTAACTCCT CCTCCAGAAG ACGGATGAGC TCGGCGACAG TGTCGCGCAC
12801 CTCGCGCTCA AAGGCTACAG GGGCCTCTTC TTCTTCTTCA ATCTCCTCTT
12851 CCATAAGGGC CTCCCCTTCT TCTTCTTCTG GCGGCGGTGG GGGAGGGGGG
12901 ACACGGCGGC GACGACGGCG CACCGGGAGG CGGTCGACAA AGCGCTCGAT
12951 CATCTCCCCG CGGCGACGGC GCATGGTCTC GGTGACGGCG CGGCCGTTCT
13001 CGCGGGGGCG CAGTTGGAAG ACGCCGCCCG TCATGTCCCG GTTATGGGTT
13051 GGCGGGGGGC TGCCATGCGG CAGGGATACG GCGCTAACGA TGCATCTCAA
13101 CAATTGTTGT GTAGGTACTC CGCCGCCGAG GGACCTGAGC GAGTCCGCAT
13151 CGACCGGATC GGAAAACCTC TCGAGAAAGG CGTCTAACCA GTCACAGTCG
13201 CAAGGTAGGC TGAGCACCGT GGCGGGCGGC AGCGGGCGGC GGTCGGGGTT
13251 GTTTCTGGCG GAGGTGCTGC TGATGATGTA ATTAAAGTAG GCGGTCTTGA
13301 GACGGCGGAT GGTCGACAGA AGCACCATGT CCTTGGGTCC GGCCTGCTGA
 
13351 ATGCGCAGGC GGTCGGCCAT GCCCCAGGCT TCGTTTTGAC ATCGGCGCAG
13401 GTCTTTGTAG TAGTCTTGCA TGAGCCTTTC TACCGGCACT TCTTCTTCTC
13451 CTTCCTCTTG TCCTGCATCT CTTGCATCTA TCGCTGCGGC GGCGGCGGAG
13501 TTTGGCCGTA GGTGGCGCCC TCTTCCTCCC ATGCGTGTGA CCCCGAAGCC
13551 CCTCATCGGC TGAAGCAGGG CTAGGTCGGC GACAACGCGC TCGGCTAATA
13601 TGGCCTGCTG CACCTGCGTG AGGGTAGACT GGAAGTCATC CATGTCCACA
13651 AAGCGGTGGT ATGCGCCCGT GTTGATGGTG TAAGTGCAGT TGGCCATAAC
13701 GGACCAGTTA ACGGTCTGGT GACCCGGCTG CGAGAGCTCG GTGTACCTGA
13751 GACGCGAGTA AGCCCTCGAG TCAAATACGT AGTCGTTGCA AGTCCGCACC
13801 AGGTACTGGT ATCCCACCAA AAAGTGCGGC GGCGGCTGGC GGTAGAGGGG
13851 CCAGCGTAGG GTGGCCGGGG CTCCGGGGGC GAGATCTTCC AACATAAGGC
13901 GATGATATCC GTAGATGTAC CTGGACATCC AGGTGATGCC GGCGGCGGTG
13951 GTGGAGGCGC GCGGAAAGTC GCGGACGCGG TTCCAGATGT TGCGCAGCGG
14001 CAAAAAGTGC TCCATGGTCG GGACGCTCTG GCCGGTCAGG CGCGCGCAAT
14051 CGTTGACGCT CTAGACCGTG CAAAAGGAGA GCCTGTAAGC GGGCACTCTT
14101 CCGTGGTCTG GTGGATAAAT TCGCAAGGGT ATCATGGCGG ACGACCGGGG
14151 TTCGAGCCCC GTATCCGGCC GTCCGCCGTG ATCCATGCGG TTACCGCCCG
14201 CGTGTCGAAC CCAGGTGTGC GACGTCAGAC AACGGGGGAG TGCTCCTTTT
14251 GGCTTCCTTC CAGGCGCGGC GGCTGCTGCG CTAGCTTTTT TGGCCACTGG
14301 CCGCGCGCAG CGTAAGCGGT TAGGCTGGAA AGCGAAAGCA TTAAGTGGCT
14351 CGCTCCCTGT AGCCGGAGGG TTATTTTCCA AGGGTTGAGT CGCGGGACCC
14401 CCGGTTCGAG TCTCGGACCG GCCGGACTGC GGCGAACGGG GGTTTGCCTC
14451 CCCGTCATGC AAGACCCCGC TTGCAAATTC CTCCGGAAAC AGGGACGAGC
14501 CCCTTTTTTG CTTTTCCCAG ATGCATCCGG TGCTGCGGCA GATGCGCCCC
14551 CCTCCTCAGC AGCGGCAAGA GCAAGAGCAG CGGCAGACAT GCAGGGCACC
14601 CTCCCCTCCT CCTACCGCGT CAGGAGGGGC GACATCCGCG GTTGACGCGG
14651 CAGCAGATGG TGATTACGAA CCCCCGCGGC GCCGGGCCCG GCACTACCTG
14701 GACTTGGAGG AGGGCGAGGG CCTGGCGCGG CTAGGAGCGC CCTCTCCTGA
14751 GCGGTACCCA AGGGTGCAGC TGAAGCGTGA TACGCGTGAG GCGTACGTGC
14801 CGCGGCAGAA CCTGTTTCGC GACCGCGAGG GAGAGGAGCC CGAGGAGATG
14851 CGGGATCGAA AGTTCCACGC AGGGCGCGAG CTGCGGCATG GCCTGAATCG
14901 CGAGCGGTTG CTGCGCGAGG AGGACTTTGA GCCCGACGCG CGAACCGGGA
14951 TTAGTCCCGC GCGCGCACAC GTGGCGGCCG CCGACCTGGT AACCGCATAC
15001 GAGCAGACGG TGAACCAGGA GATTAACTTT CAAAAAAGCT TTAACAACCA
15051 CGTGCGTACG CTTGTGGCGC GCGAGGAGGT GGCTATAGGA CTGATGCATC
15101 TGTGGGACTT TGTAAGCGCG CTGGAGCAAA ACCCAAATAG CAAGCCGCTC
15151 ATGGCGCAGC TGTTCCTTAT AGTGCAGCAC AGCAGGGACA ACGAGGCATT
15201 CAGGGATGCG CTGCTAAACA TAGTAGAGCC CGAGGGCCGC TGGCTGCTCG
 
15251 ATTTGATAAA CATCCTGCAG AGCATAGTGG TGCAGGAGCG CAGCTTGAGC
15301 CTGGCTGACA AGGTGGCCGC CATCAACTAT TCCATGCTTA GCCTGGGCAA
15351 GTTTTACGCC CGCAAGATAT ACCATACCCC TTACGTTCCC ATAGACAAGG
15401 AGGTAAAGAT CGAGGGGTTC TACATGCGCA TGGCGCTGAA GGTGCTTACC
15451 TTGAGCGACG ACCTGGGCGT TTATCGCAAC GAGCGCATCC ACAAGGCCGT
15501 GAGCGTGAGC CGGCGGCGCG AGCTCAGCGA CCGCGAGCTG ATGCACAGCC
15551 TGCAAAGGGC CCTGGCTGGC ACGGGCAGCG GCGATAGAGA GGCCGAGTCC
15601 TACTTTGACG CGGGCGCTGA CCTGCGCTGG GCCCCAAGCC GACGCGCCCT
15651 GGAGGCAGCT GGGGCCGGAC CTGGGCTGGC GGTGGCACCC GCGCGCGCTG
15701 GCAACGTCGG CGGCGTGGAG GAATATGACG AGGACGATGA GTACGAGCCA
15751 GAGGACGGCG AGTACTAAGC GGTGATGTTT CTGATCAGAT GATGCAAGAC
15801 GCAACGGACC CGGCGGTGCG GGCGGCGCTG CAGAGCCAGC CGTCCGGCCT
15851 TAACTCCACG GACGACTGGC GCCAGGTCAT GGACCGCATC ATGTCGCTGA
15901 CTGCGCGCAA TCCTGACGCG TTCCGGCAGC AGCCGCAGGC CAACCGGCTC
15951 TCCGCAATTC TGGAAGCGGT GGTCCCGGCG CGCGCAAACC CCACGCACGA
16001 GAAGGTGCTG GCGATCGTAA ACGCGCTGGC CGAAAACAGG GCCATCCGGC
16051 CCGACGAGGC CGGCCTGGTC TACGACGCGC TGCTTCAGCG CGTGGCTCGT
16101 TACAACAGCG GCAACGTGCA GACCAACCTG GACCGGCTGG TGGGGGATGT
16151 GCGCGAGGCC GTGGCGCAGC GTGAGCGCGC GCAGCAGCAG GGCAACCTGG
16201 GCTCCATGGT TGCACTAAAC GCCTTCCTGA GTACACAGCC CGCCAACGTG
16251 CCGCGGGGAC AGGAGGACTA CACCAACTTT GTGAGCGCAC TGCGGCTAAT
16301 GGTGACTGAG ACACCGCAAA GTGAGGTGTA CCAGTCTGGG CCAGACTATT
16351 TTTTCCAGAC CAGTAGACAA GGCCTGCAGA CCGTAAACCT GAGCCAGGCT
16401 TTCAAAAACT TGCAGGGGCT GTGGGGGGTG CGGGCTCCCA CAGGCGACCG
16451 CGCGACCGTG TCTAGCTTGC TGACGCCCAA CTCGCGCCTG TTGCTGCTGC
16501 TAATAGCGCC CTTCACGGAC AGTGGCAGCG TGTCCCGGGA CACATACCTA
16551 GGTCACTTGC TGACACTGTA CCGCGAGGCC ATAGGTCAGG CGCATGTGGA
16601 CGAGCATACT TTCCAGGAGA TTACAAGTGT CAGCCGCGCG CTGGGGCAGG
16651 AGGACACGGG CAGCCTGGAG GCAACCCTAA ACTACCTGCT GACCAACCGG
16701 CGGCAGAAGA TCCCCTCGTT GCACAGTTTA AACAGCGAGG AGGAGCGCAT
16751 TTTGCGCTAC GTGCAGCAGA GCGTGAGCCT TAACCTGATG CGCGACGGGG
16801 TAACGCCCAG CGTGGCGCTG GACATGACCG CGCGCAACAT GGAACCGGGC
16851 ATGTATGCCT CAAACCGGCC GTTTATCAAC CGCCTAATGG ACTACTTGCA
16901 TCGCGCGGCC GCCGTGAACC CCGAGTATTT CACCAATGCC ATCTTGAACC
16951 CGCACTGGCT ACCGCCCCCT GGTTTCTACA CCGGGGGATT CGAGGTGCCC
17001 GAGGGTAACG ATGGATTCCT CTGGGACGAC ATAGACGACA GCGTGTTTTC
17051 CCCGCAACCG CAGACCCTGC TAGAGTTGCA ACAGCGCGAG CAGGCAGAGG
17101 CGGCGCTGCG AAAGGAAAGC TTCCGCAGGC CAAGCAGCTT GTCCGATCTA
 
17151 GGCGCTGCGG CCCCGCGGTC AGATGCTAGT AGCCCATTTC CAAGCTTGAT
17201 AGGGTCTCTT ACCAGCACTC GCACCACCCG CCCGCGCCTG CTGGGCGAGG
17251 AGGAGTACCT AAACAACTCG CTGCTGCAGC CGCAGCGCGA AAAAAACCTG
17301 CCTCCGGCAT TTCCCAACAA CGGGATAGAG AGCCTAGTGG ACAAGATGAG
17351 TAGATGGAAG ACGTACGCGC AGGAGCACAG GGACGTGCCA GGCCCGCGCC
17401 CGCCCACCCG TCGTCAAAGG CACGACCGTC AGCGGGGTCT GGTGTGGGAG
17451 GACGATGACT CGGCAGACGA CAGCAGCGTC CTGGATTTGG GAGGGAGTGG
17501 CAACCCGTTT GCGCACCTTC GCCCCAGGCT GGGGAGAATG TTTTAAAAAA
17551 AAAAAAGCAT GATGCAAAAT AAAAAACTCA CCAAGGCCAT GGCACCGAGC
17601 GTTGGTTTTC TTGTATTCCC CTTAGTATGC GGCGCGCGGC GATGTATGAG
17651 GAAGGTCCTC CTCCCTCCTA CGAGAGTGTG GTGAGCGCGG CGCCAGTGGC
17701 GGCGGCGCTG GGTTCTCCCT TCGATGCTCC CCTGGACCCG CCGTTTGTGC
17751 CTCCGCGGTA CCTGCGGCCT ACCGGGGGGA GAAACAGCAT CCGTTACTCT
17801 GAGTTGGCAC CCCTATTCGA CACCACCCGT GTGTACCTGG TGGACAACAA
17851 GTCAACGGAT GTGGCATCCC TGAACTACCA GAACGACCAC AGCAACTTTC
17901 TGACCACGGT CATTCAAAAC AATGACTACA GCCCGGGGGA GGCAAGCACA
17951 CAGACCATCA ATCTTGACGA CCGGTCGCAC TGGGGCGGCG ACCTGAAAAC
18001 CATCCTGCAT ACCAACATGC CAAATGTGAA CGAGTTCATG TTTACCAATA
18051 AGTTTAAGGC GCGGGTGATG GTGTCGCGCT TGCCTACTAA GGACAATCAG
18101 GTGGAGCTGA AATACGAGTG GGTGGAGTTC ACGCTGCCCG AGGGCAACTA
18151 CTCCGAGACC ATGACCATAG ACCTTATGAA CAACGCGATC GTGGAGCACT
18201 ACTTGAAAGT GGGCAGACAG AACGGGGTTC TGGAAAGCGA CATCGGGGTA
18251 AAGTTTGACA CCCGCAACTT CAGACTGGGG TTTGACCCCG TCACTGGTCT
18301 TGTCATGCCT GGGGTATATA CAAACGAAGC CTTCCATCCA GACATCATTT
18351 TGCTGCCAGG ATGCGGGGTG GACTTCACCC ACAGCCGCCT GAGCAACTTG
18401 TTGGGCATCC GCAAGCGGCA ACCCTTCCAG GAGGGCTTTA GGATCACCTA
18451 CGATGATCTG GAGGGTGGTA ACATTCCCGC ACTGTTGGAT GTGGACGCCT
18501 ACCAGGCGAG CTTGAAAGAT GACACCGAAC AGGGCGGGGG TGGCGCAGGC
18551 GGCAGCAACA GCAGTGGCAG CGGCGCGGAA GAGAACTCCA ACGCGGCAGC
18601 CGCGGCAATG CAGCCGGTGG AGGACATGAA CGATCATGCC ATTCGCGGCG
18651 ACACCTTTGC CACACGGGCT GAGGAGAAGC GCGCTGAGGC CGAAGCAGCG
18701 GCCGAAGCTG CCGCCCCCGC TGCGCAACCC GAGGTCGAGA AGCCTCAGAA
18751 GAAACCGGTG ATCAAACCCC TGACAGAGGA CAGCAAGAAA CGCAGTTACA
18801 ACCTAATAAG CAATGACAGC ACCTTCACCC AGTACCGCAG CTGGTACCTT
18851 GCATACAACT ACGGCGACCC TCAGACCGGA ATCCGCTCAT GGACCCTGCT
18901 TTGCACTCCT GACGTAACCT GCGGCTCGGA GCAGGTCTAC TGGTCGTTGC
18951 CAGACATGAT GCAAGACCCC GTGACCTTCC GCTCCACGCG CCAGATCAGC
19001 AACTTTCCGG TGGTGGGCGC CGAGCTGTTG CCCGTGCACT CCAAGAGCTT
 
19051 CTACAACGAC CAGGCCGTCT ACTCCCAACT CATCCGCCAG TTTACCTCTC
19101 TGACCCACGT GTTCAATCGC TTTCCCGAGA ACCAGATTTT GGCGCGCCCG
19151 CCAGCCCCCA CCATCACCAC CGTCAGTGAA AACGTTCCTG CTCTCACAGA
19201 TCACGGGACG CTACCGCTGC GCAACAGCAT CGGAGGAGTC CAGCGAGTGA
19251 CCATTACTGA CGCCAGACGC CGCACCTGCC CCTACGTTTA CAAGGCCCTG
19301 GGCATAGTCT CGCCGCGCGT CCTATCGAGC CGCACTTTTT GAGCAAGCAT
19351 GTCCATCCTT ATATCGCCCA GCAATAACAC AGGCTGGGGC CTGCGCTTCC
19401 CAAGCAAGAT GTTTGGCGGG GCCAAGAAGC GCTCCGACCA ACACCCAGTG
19451 CGCGTGCGCG GGCACTACCG CGCGCCCTGG GGCGCGCACA AACGCGGCCG
19501 CACTGGGCGC ACCACCGTCG ATGACGCCAT CGACGCGGTG GTGGAGGAGG
19551 CGCGCAACTA CACGCCCACG CCGCCACCAG TGTCCACAGT GGACGCGGCC
19601 ATTCAGACCG TGGTGCGCGG AGCCCGGCGC TATGCTAAAA TGAAGAGACG
19651 GCGGAGGCGC GTAGCACGTC GCCACCGCCG CCGACCCGGC ACTGCCGCCC
19701 AACGCGCGGC GGCGGCCCTG CTTAACCGCG CACGTCGCAC CGGCCGACGG
19751 GCGGCCATGC GGGCCGCTCG AAGGCTGGCC GCGGGTATTG TCACTGTGCC
19801 CCCCAGGTCC AGGCGACGAG CGGCCGCCGC AGCAGCCGCG GCCATTAGTG
19851 CTATGACTCA GGGTCGCAGG GGCAACGTGT ATTGGGTGCG CGACTCGGTT
19901 AGCGGCCTGC GCGTGCCCGT GCGCACCCGC CCCCCGCGCA ACTAGATTGC
19951 AAGAAAAAAC TACTTAGACT CGTACTGTTG TATGTATCCA GCGGCGGCGG
20001 CGCGCAACGA AGCTATGTCC AAGCGCAAAA TCAAAGAAGA GATGCTCCAG
20051 GTCATCGCGC CGGAGATCTA TGGCCCCCCG AAGAAGGAAG AGCAGGATTA
20101 CAAGCCCCGA AAGCTAAAGC GGGTCAAAAA GAAAAAGAAA GATGATGATG
20151 ATGAACTTGA CGACGAGGTG GAACTGCTGC ACGCTACCGC GCCCAGGCGA
20201 CGGGTACAGT GGAAAGGTCG ACGCGTAAAA CGTGTTTTGC GACCCGGCAC
20251 CACCGTAGTC TTTACGCCCG GTGAGCGCTC CACCCGCACC TACAAGCGCG
20301 TGTATGATGA GGTGTACGGC GACGAGGACC TGCTTGAGCA GGCCAACGAG
20351 CGCCTCGGGG AGTTTGCCTA CGGAAAGCGG CATAAGGACA TGCTGGCGTT
20401 GCCGCTGGAC GAGGGCAACC CAACACCTAG CCTAAAGCCC GTAACACTGC
20451 AGCAGGTGCT GCCCGCGCTT GCACCGTCCG AAGAAAAGCG CGGCCTAAAG
20501 CGCGAGTCTG GTGACTTGGC ACCCACCGTG CAGCTGATGG TACCCAAGCG
20551 CCAGCGACTG GAAGATGTCT TGGAAAAAAT GACCGTGGAA CCTGGGCTGG
20601 AGCCCGAGGT CCGCGTGCGG CCAATCAAGC AGGTGGCGCC GGGACTGGGC
20651 GTGCAGACCG TGGACGTTCA GATACCCACT ACCAGTAGCA CCAGTATTGC
20701 CACCGCCACA GAGGGCATGG AGACACAAAC GTCCCCGGTT GCCTCAGCGG
20751 TGGCGGATGC CGCGGTGCAG GCGGTCGCTG CGGCCGCGTC CAAGACCTCT
20801 ACGGAGGTGC AAACGGACCC GTGGATGTTT CGCGTTTCAG CCCCCCGGCG
20851 CCCGCGCGGT TCGAGGAAGT ACGGCGCCGC CAGCGCGCTA CTGCCCGAAT
20901 ATGCCCTACA TCCTTCCATT GCGCCTACCC CCGGCTATCG TGGCTACACC
 
20951 TACCGCCCCA GAAGACGAGC AACTACCCGA CGCCGAACCA CCACTGGAAC
21001 CCGCCGCCGC CGTCGCCGTC GCCAGCCCGT GCTGGCCCCG ATTTCCGTGC
21051 GCAGGGTGGC TCGCGAAGGA GGCAGGACCC TGGTGCTGCC AACAGCGCGC
21101 TACCACCCCA GCATCGTTTA AAAGCCGGTC TTTGTGGTTC TTGCAGATAT
21151 GGCCCTCACC TGCCGCCTCC GTTTCCCGGT GCCGGGATTC CGAGGAAGAA
21201 TGCACCGTAG GAGGGGCATG GCCGGCCACG GCCTGACGGG CGGCATGCGT
21251 CGTGCGCACC ACCGGCGGCG GCGCGCGTCG CACCGTCGCA TGCGCGGCGG
21301 TATCCTGCCC CTCCTTATTC CACTGATCGC CGCGGCGATT GGCGCCGTGC
21351 CCGGAATTGC ATCCGTGGCC TTGCAGGCGC AGAGACACTG ATTAAAAACA
21401 AGTTGCATGT GGAAAAATCA AAATAAAAAG TCTGGACTCT CACGCTCGCT
21451 TGGTCCTGTA ACTATTTTGT AGAATGGAAG ACATCAACTT TGCGTCTCTG
21501 GCCCCGCGAC ACGGCTCGCG CCCGTTCATG GGAAACTGGC AAGATATCGG
21551 CACCAGCAAT ATGAGCGGTG GCGCCTTCAG CTGGGGCTCG CTGTGGAGCG
21601 GCATTAAAAA TTTCGGTTCC ACCGTTAAGA ACTATGGCAG CAAGGCCTGG
21651 AACAGCAGCA CAGGCCAGAT GCTGAGGGAT AAGTTGAAAG AGCAAAATTT
21701 CCAACAAAAG GTGGTAGATG GCCTGGCCTC TGGCATTAGC GGGGTGGTGG
21751 ACCTGGCCAA CCAGGCAGTG CAAAATAAGA TTAACAGTAA GCTTGATCCC
21801 CGCCCTCCCG TAGAGGAGCC TCCACCGGCC GTGGAGACAG TGTCTCCAGA
21851 GGGGCGTGGC GAAAAGCGTC CGCGCCCCGA CAGGGAAGAA ACTCTGGTGA
21901 CGCAAATAGA CGAGCCTCCC TCGTACGAGG AGGCACTAAA GCAAGGCCTG
21951 CCCACCACCC GTCCCATCGC GCCCATGGCT ACCGGAGTGC TGGGCCAGCA
22001 CACACCCGTA ACGCTGGACC TGCCTCCCCC CGCCGACACC CAGCAGAAAC
22051 CTGTGCTGCC AGGCCCGACC GCCGTTGTTG TAACCCGTCC TAGCCGCGCG
22101 TCCCTGCGCC GCGCCGCCAG CGGTCCGCGA TCGTTGCGGC CCGTAGCCAG
22151 TGGCAACTGG CAAAGCACAC TGAACAGCAT CGTGGGTCTG GGGGTGCAAT
22201 CCCTGAAGCG CCGACGATGC TTCTGAATAG CTAACGTGTC GTATGTGTGT
22251 CATGTATGCG TCCATGTCGC CGCCAGAGGA GCTGCTGAGC CGCCGCGCGC
22301 CCGCTTTCCA AGATGGCTAC CCCTTCGATG ATGCCGCAGT GGTCTTACAT
22351 GCACATCTCG GGCCAGGACG CCTCGGAGTA CCTGAGCCCC GGGCTGGTGC
22401 AGTTTGCCCG CGCCACCGAG ACGTACTTCA GCCTGAATAA CAAGTTTAGA
22451 AACCCCACGG TGGCGCCTAC GCACGACGTG ACCACAGACC GGTCCCAGCG
22501 TTTGACGCTG CGGTTCATCC CTGTGGACCG TGAGGATACT GCGTACTCGT
22551 ACAAGGCGCG GTTCACCCTA GCTGTGGGTG ATAACCGTGT GCTGGACATG
22601 GCTTCCACGT ACTTTGACAT CCGCGGCGTG CTGGACAGGG GCCCTACTTT
22651 TAAGCCCTAC TCTGGCACTG CCTACAACGC CCTGGCTCCC AAGGGTGCCC
22701 CAAATCCTTG CGAATGGGAT GAAGCTGCTA CTGCTCTTGA AATAAACCTA
22751 GAAGAAGAGG ACGATGACAA CGAAGACGAA GTAGACGAGC AAGCTGAGCA
22801 GCAAAAAACT CACGTATTTG GGCAGGCGCC TTATTCTGGT ATAAATATTA
 
22851 CAAAGGAGGG TATTCAAATA GGTGTCGAAG GTCAAACACC TAAATATGCC
22901 GATAAAACAT TTCAACCTGA ACCTCAAATA GGAGAATCTC AGTGGTACGA
22951 AACTGAAATT AATCATGCAG CTGGGAGAGT CCTTAAAAAG ACTACCCCAA
23001 TGAAACCATG TTACGGTTCA TATGCAAAAC CCACAAATGA AAATGGAGGG
23051 CAAGGCATTC TTGTAAAGCA ACAAAATGGA AAGCTAGAAA GTCAAGTGGA
23101 AATGCAATTT TTCTCAACTA CTGAGGCGAC CGCAGGCAAT GGTGATAACT
23151 TGACTCCTAA AGTGGTATTG TACAGTGAAG ATGTAGATAT AGAAACCCCA
23201 GACACTCATA TTTCTTACAT GCCCACTATT AAGGAAGGTA ACTCACGAGA
23251 ACTAATGGGC CAACAATCTA TGCCCAACAG GCCTAATTAC ATTGCTTTTA
23301 GGGACAATTT TATTGGTCTA ATGTATTACA ACAGCACGGG TAATATGGGT
23351 GTTCTGGCGG GCCAAGCATC GCAGTTGAAT GCTGTTGTAG ATTTGCAAGA
23401 CAGAAACACA GAGCTTTCAT ACCAGCTTTT GCTTGATTCC ATTGGTGATA
23451 GAACCAGGTA CTTTTCTATG TGGAATCAGG CTGTTGACAG CTATGATCCA
23501 GATGTTAGAA TTATTGAAAA TCATGGAACT GAAGATGAAC TTCCAAATTA
23551 CTGCTTTCCA CTGGGAGGTG TGATTAATAC AGAGACTCTT ACCAAGGTAA
23601 AACCTAAAAC AGGTCAGGAA AATGGATGGG AAAAAGATGC TACAGAATTT
23651 TCAGATAAAA ATGAAATAAG AGTTGGAAAT AATTTTGCCA TGGAAATCAA
23701 TCTAAATGCC AACCTGTGGA GAAATTTCCT GTACTCCAAC ATAGCGCTGT
23751 ATTTGCCCGA CAAGCTAAAG TACAGTCCTT CCAACGTAAA AATTTCTGAT
23801 AACCCAAACA CCTACGACTA CATGAACAAG CGAGTGGTGG CTCCCGGGTT
23851 AGTGGACTGC TACATTAACC TTGGAGCACG CTGGTCCCTT GACTATATGG
23901 ACAACGTCAA CCCATTTAAC CACCACCGCA ATGCTGGCCT GCGCTACCGC
23951 TCAATGTTGC TGGGCAATGG TCGCTATGTG CCCTTCCACA TCCAGGTGCC
24001 TCAGAAGTTC TTTGCCATTA AAAACCTCCT TCTCCTGCCG GGCTCATACA
24051 CCTACGAGTG GAACTTCAGG AAGGATGTTA ACATGGTTCT GCAGAGCTCC
24101 CTAGGAAATG ACCTAAGGGT TGACGGAGCC AGCATTAAGT TTGATAGCAT
24151 TTGCCTTTAC GCCACCTTCT TCCCCATGGC CCACAACACC GCCTCCACGC
24201 TTGAGGCCAT GCTTAGAAAC GACACCAACG ACCAGTCCTT TAACGACTAT
24251 CTCTCCGCCG CCAACATGCT CTACCCTATA CCCGCCAACG CTACCAACGT
24301 GCCCATATCC ATCCCCTCCC GCAACTGGGC GGCTTTCCGC GGCTGGGCCT
24351 TCACGCGCCT TAAGACTAAG GAAACCCCAT CACTGGGCTC GGGCTACGAC
24401 CCTTATTACA CCTACTCTGG CTCTATACCC TACCTAGATG GAACCTTTTA
24451 CCTCAACCAC ACCTTTAAGA AGGTGGCCAT TACCTTTGAC TCTTCTGTCA
24501 GCTGGCCTGG CAATGACCGC CTGCTTACCC CCAACGAGTT TGAAATTAAG
24551 CGCTCAGTTG ACGGGGAGGG TTACAACGTT GCCCAGTGTA ACATGACCAA
24601 AGACTGGTTC CTGGTACAAA TGCTAGCTAA CTACAACATT GGCTACCAGG
24651 GCTTCTATAT CCCAGAGAGC TACAAGGACC GCATGTACTC CTTCTTTAGA
24701 AACTTCCAGC CCATGAGCCG TCAGGTGGTG GATGATACTA AATACAAGGA
 
24751 CTACCAACAG GTGGGCATCC TACACCAACA CAACAACTCT GGATTTGTTG
24801 GCTACCTTGC CCCCACCATG CGCGAAGGAC AGGCCTACCC TGCTAACTTC
24851 CCCTATCCGC TTATAGGCAA GACCGCAGTT GACAGCATTA CCCAGAAAAA
24901 GTTTCTTTGC GATCGCACCC TTTGGCGCAT CCCATTCTCC AGTAACTTTA
24951 TGTCCATGGG CGCACTCACA GACCTGGGCC AAAACCTTCT CTACGCCAAC
25001 TCCGCCCACG CGCTAGACAT GACTTTTGAG GTGGATCCCA TGGACGAGCC
25051 CACCCTTCTT TATGTTTTGT TTGAAGTCTT TGACGTGGTC CGTGTGCACC
25101 GGCCGCACCG CGGCGTCATC GAAACCGTGT ACCTGCGCAC GCCCTTCTCG
25151 GCCGGCAACG CCACAACATA AAGAAGCAAG CAACATCAAC AACAGCTGCC
25201 GCCATGGGCT CCAGTGAGCA GGAACTGAAA GCCATTGTCA AAGATCTTGG
25251 TTGTGGGCCA TATTTTTTGG GCACCTATGA CAAGCGCTTT CCAGGCTTTG
25301 TTTCTCCACA CAAGCTCGCC TGCGCCATAG TCAATACGGC CGGTCGCGAG
25351 ACTGGGGGCG TACACTGGAT GGCCTTTGCC TGGAACCCGC ACTCAAAAAC
25401 ATGCTACCTC TTTGAGCCCT TTGGCTTTTC TGACCAGCGA CTCAAGCAGG
25451 TTTACCAGTT TGAGTACGAG TCACTCCTGC GCCGTAGCGC CATTGCTTCT
25501 TCCCCCGACC GCTGTATAAC GCTGGAAAAG TCCACCCAAA GCGTACAGGG
25551 GCCCAACTCG GCCGCCTGTG GACTATTCTG CTGCATGTTT CTCCACGCCT
25601 TTGCCAACTG GCCCCAAACT CCCATGGATC ACAACCCCAC CATGAACCTT
25651 ATTACCGGGG TACCCAACTC CATGCTCAAC AGTCCCCAGG TACAGCCCAC
25701 CCTGCGTCGC AACCAGGAAC AGCTCTACAG CTTCCTGGAG CGCCACTCGC
25751 CCTACTTCCG CAGCCACAGT GCGCAGATTA GGAGCGCCAC TTCTTTTTGT
25801 CACTTGAAAA ACATGTAAAA ATAATGTACT AGAGACACTT TCAATAAAGG
25851 CAAATGCTTT TATTTGTACA CTCTCGGGTG ATTATTTACC CCCACCCTTG
25901 CCGTCTGCGC CGTTTAAAAA TCAAAGGGGT TCTGCCGCGC ATCGCTATGC
25951 GCCACTGGCA GGGACACGTT GCGATACTGG TGTTTAGTGC TCCACTTAAA
26001 CTCAGGCACA ACCATCCGCG GCAGCTCGGT GAAGTTTTCA CTCCACAGGC
26051 TGCGCACCAT CACCAACGCG TTTAGCAGGT CGGGCGCCGA TATCTTGAAG
26101 TCGCAGTTGG GGCCTCCGCC CTGCGCGCGC GAGTTGCGAT ACACAGGGTT
26151 GCAGCACTGG AACACTATCA GCGCCGGGTG GTGCACGCTG GCCAGCACGC
26201 TCTTGTCGGA GATCAGATCC GCGTCCAGGT CCTCCGCGTT GCTCAGGGCG
26251 AACGGAGTCA ACTTTGGTAG CTGCCTTCCC AAAAAGGGCG CGTGCCCAGG
26301 CTTTGAGTTG CACTCGCACC GTAGTGGCAT CAAAAGGTGA CCGTGCCCGG
26351 TCTGGGCGTT AGGATACAGC GCCTGCATAA AAGCCTTGAT CTGCTTAAAA
26401 GCCACCTGAG CCTTTGCGCC TTCAGAGAAG AACATGCCGC AAGACTTGCC
26451 GGAAAACTGA TTGGCCGGAC AGGCCGCGTC GTGCACGCAG CACCTTGCGT
26501 CGGTGTTGGA GATCTGCACC ACATTTCGGC CCCACCGGTT CTTCACGATC
26551 TTGGCCTTGC TAGACTGCTC CTTCAGCGCG CGCTGCCCGT TTTCGCTCGT
26601 CACATCCATT TCAATCACGT GCTCCTTATT TATCATAATG CTTCCGTGTA
 
26651 GACACTTAAG CTCGCCTTCG ATCTCAGCGC AGCGGTGCAG CCACAACGCG
26701 CAGCCCGTGG GCTCGTGATG CTTGTAGGTC ACCTCTGCAA ACGACTGCAG
26751 GTACGCCTGC AGGAATCGCC CCATCATCGT CACAAAGGTC TTGTTGCTGG
26801 TGAAGGTCAG CTGCAACCCG CGGTGCTCCT CGTTCAGCCA GGTCTTGCAT
26851 ACGGCCGCCA GAGCTTCCAC TTGGTCAGGC AGTAGTTTGA AGTTCGCCTT
26901 TAGATCGTTA TCCACGTGGT ACTTGTCCAT CAGCGCGCGC GCAGCCTCCA
26951 TGCCCTTCTC CCACGCAGAC ACGATCGGCA CACTCAGCGG GTTCATCACC
27001 GTAATTTCAC TTTCCGCTTC GCTGGGCTCT TCCTCTTCCT CTTGCGTCCG
27051 CATACCACGC GCCACTGGGT CGTCTTCATT CAGCCGCCGC ACTGTGCGCT
27101 TACCTCCTTT GCCATGCTTG ATTAGCACCG GTGGGTTGCT GAAACCCACC
27151 ATTTGTAGCG CCACATCTTC TCTTTCTTCC TCGCTGTCCA CGATTACCTC
27201 TGGTGATGGC GGGCGCTCGG GCTTGGGAGA AGGGCGCTTC TTTTTCTTCT
27251 TGGGCGCAAT GGCCAAATCC GCCGCCGAGG TCGATGGCCG CGGGCTGGGT
27301 GTGCGCGGCA CCAGCGCGTC TTGTGATGAG TCTTCCTCGT CCTCGGACTC
27351 GATACGCCGC CTCATCCGCT TTTTTGGGGG CGCCCGGGGA GGCGGCGGCG
27401 ACGGGGACGG GGACGACACG TCCTCCATGG TTGGGGGACG TCGCGCCGCA
27451 CCGCGTCCGC GCTCGGGGGT GGTTTCGCGC TGCTCCTCTT CCCGACTGGC
27501 CATTTCCTTC TCCTATAGGC AGAAAAAGAT CATGGAGTCA GTCGAGAAGA
27551 AGGACAGCCT AACCGCCCCC TCTGAGTTCG CCACCACCGC CTCCACCGAT
27601 GCCGCCAACG CGCCTACCAC CTTCCCCGTC GAGGCACCCC CGCTTGAGGA
27651 GGAGGAAGTG ATTATCGAGC AGGACCCAGG TTTTGTAAGC GAAGACGACG
27701 AGGACCGCTC AGTACCAACA GAGGATAAAA AGCAAGACCA GGACAACGCA
27751 GAGGCAAACG AGGAACAAGT CGGGCGGGGG GACGAAAGGC ATGGCGACTA
27801 CCTAGATGTG GGAGACGACG TGCTGTTGAA GCATCTGCAG CGCCAGTGCG
27851 CCATTATCTG CGACGCGTTG CAAGAGCGCA GCGATGTGCC CCTCGCCATA
27901 GCGGATGTCA GCCTTGCCTA CGAACGCCAC CTATTCTCAC CGCGCGTACC
27951 CCCCAAACGC CAAGAAAACG GCACATGCGA GCCCAACCCG CGCCTCAACT
28001 TCTACCCCGT ATTTGCCGTG CCAGAGGTGC TTGCCACCTA TCACATCTTT
28051 TTCCAAAACT GCAAGATACC CCTATCCTGC CGTGCCAACC GCAGCCGAGC
28101 GGACAAGCAG CTGGCCTTGC GGCAGGGCGC TGTCATACCT GATATCGCCT
28151 CGCTCAACGA AGTGCCAAAA ATCTTTGAGG GTCTTGGACG CGACGAGAAG
28201 CGCGCGGCAA ACGCTCTGCA ACAGGAAAAC AGCGAAAATG AAAGTCACTC
28251 TGGAGTGTTG GTGGAACTCG AGGGTGACAA CGCGCGCCTA GCCGTACTAA
28301 AACGCAGCAT CGAGGTCACC CACTTTGCCT ACCCGGCACT TAACCTACCC
28351 CCCAAGGTCA TGAGCACAGT CATGAGTGAG CTGATCGTGC GCCGTGCGCA
28401 GCCCCTGGAG AGGGATGCAA ATTTGCAAGA ACAAACAGAG GAGGGCCTAC
28451 CCGCAGTTGG CGACGAGCAG CTAGCGCGCT GGCTTCAAAC GCGCGAGCCT
28501 GCCGACTTGG AGGAGCGACG CAAACTAATG ATGGCCGCAG TGCTCGTTAC
 
28551 CGTGGAGCTT GAGTGCATGC AGCGGTTCTT TGCTGACCCG GAGATGCAGC
28601 GCAAGCTAGA GGAAACATTG CACTACACCT TTCGACAGGG CTACGTACGC
28651 CAGGCCTGCA AGATCTCCAA CGTGGAGCTC TGCAACCTGG TCTCCTACCT
28701 TGGAATTTTG CACGAAAACC GCCTTGGGCA AAACGTGCTT CATTCCACGC
28751 TCAAGGGCGA GGCGCGCCGC GACTACGTCC GCGACTGCGT TTACTTATTT
28801 CTATGCTACA CCTGGCAGAC GGCCATGGGC GTTTGGCAGC AGTGCTTGGA
28851 GGAGTGCAAC CTCAAGGAGC TGCAGAAACT GCTAAAGCAA AACTTGAAGG
28901 ACCTATGGAC GGCCTTCAAC GAGCGCTCCG TGGCCGCGCA CCTGGCGGAC
28951 ATCATTTTCC CCGAACGCCT GCTTAAAACC CTGCAACAGG GTCTGCCAGA
29001 CTTCACCAGT CAAAGCATGT TGCAGAACTT TAGGAACTTT ATCCTAGAGC
29051 GCTCAGGAAT CTTGCCCGCC ACCTGCTGTG CACTTCCTAG CGACTTTGTG
29101 CCCATTAAGT ACCGCGAATG CCCTCCGCCG CTTTGGGGCC ACTGCTACCT
29151 TCTGCAGCTA GCCAACTACC TTGCCTACCA CTCTGACATA ATGGAAGACG
29201 TGAGCGGTGA CGGTCTACTG GAGTGTCACT GTCGCTGCAA CCTATGCACC
29251 CCGCACCGCT CCCTGGTTTG CAATTCGCAG CTGCTTAACG AAAGTCAAAT
29301 TATCGGTACC TTTGAGCTGC AGGGTCCCTC GCCTGACGAA AAGTCCGCGG
29351 CTCCGGGGTT GAAACTCACT CCGGGGCTGT GGACGTCGGC TTACCTTCGC
29401 AAATTTGTAC CTGAGGACTA CCACGCCCAC GAGATTAGGT TCTACGAAGA
29451 CCAATCCCGC CCGCCAAATG CGGAGCTTAC CGCCTGCGTC ATTACCCAGG
29501 GCCACATTCT TGGCCAATTG CAAGCCATCA ACAAAGCCCG CCAAGAGTTT
29551 CTGCTACGAA AGGGACGGGG GGTTTACTTG GACCCCCAGT CCGGCGAGGA
29601 GCTCAACCCA ATCCCCCCGC CGCCGCAGCC CTATCAGCAG CAGCCGCGGG
29651 CCCTTGCTTC CCAGGATGGC ACCCAAAAAG AAGCTGCAGC TGCCGCCGCC
29701 ACCCACGGAC GAGGAGGAAT ACTGGGACAG TCAGGCAGAG GAGGTTTTGG
29751 ACGAGGAGGA GGAGGACATG ATGGAAGACT GGGAGAGCCT AGACGAGGAA
29801 GCTTCCGAGG TCGAAGAGGT GTCAGACGAA ACACCGTCAC CCTCGGTCGC
29851 ATTCCCCTCG CCGGCGCCCC AGAAATCGGC AACCGGTTCC AGCATGGCTA
29901 CAACCTCCGC TCCTCAGGCG CCGCCGGCAC TGCCCGTTCG CCGACCCAAC
29951 CGTAGATGGG ACACCACTGG AACCAGGGCC GGTAAGTCCA AGCAGCCGCC
30001 GCCGTTAGCC CAAGAGCAAC AACAGCGCCA AGGCTACCGC TCATGGCGCG
30051 GGCACAAGAA CGCCATAGTT GCTTGCTTGC AAGACTGTGG GGGCAACATC
30101 TCCTTCGCCC GCCGCTTTCT TCTCTACCAT CACGGCGTGG CCTTCCCCCG
30151 TAACATCCTG CATTACTACC GTCATCTCTA CAGCCCATAC TGCACCGGCG
30201 GCAGCGGCAG CGGCAGCAAC AGCAGCGGCC ACACAGAAGC AAAGGCGACC
30251 GGATAGCAAG ACTCTGACAA AGCCCAAGAA ATCCACAGCG GCGGCAGCAG
30301 CAGGAGGAGG AGCGCTGCGT CTGGCGCCCA ACGAACCCGT ATCGACCCGC
30351 GAGCTTAGAA ACAGGATTTT TCCCACTCTG TATGCTATAT TTCAACAGAG
30401 CAGGGGCCAA GAACAAGAGC TGAAAATAAA AAACAGGTCT CTGCGATCCC
 
30451 TCACCCGCAG CTGCCTGTAT CACAAAAGCG AAGATCAGCT TCGGCGCACG
30501 CTGGAAGACG CGGAGGCTCT CTTCAGTAAA TACTGCGCGC TGACTCTTAA
30551 GGACTAGTTT CGCGCCCTTT CTCAAATTTA AGCGCGAAAA CTACGTCATC
30601 TCCAGCGGCC ACACCCGGCG CCAGCACCTG TCGTCAGCGC CATTATGAGC
30651 AAGGAAATTC CCACGCCCTA CATGTGGAGT TACCAGCCAC AAATGGGACT
30701 TGCGGCTGGA GCTGCCCAAG ACTACTCAAC CCGAATAAAC TACATGAGCG
30751 CGGGACCCCA CATGATATCC CGGGTCAACG GAATCCGCGC CCACCGAAAC
30801 CGAATTCTCT TGGAACAGGC GGCTATTACC ACCACACCTC GTAATAACCT
30851 TAATCCCCGT AGTTGGCCCG CTGCCCTGGT GTACCAGGAA AGTCCCGCTC
30901 CCACCACTGT GGTACTTCCC AGAGACGCCC AGGCCGAAGT TCAGATGACT
30951 AACTCAGGGG CGCAGCTTGC GGGCGGCTTT CGTCACAGGG TGCGGTCGCC
31001 CGGGCAGGGT ATAACTCACC TGACAATCAG AGGGCGAGGT ATTCAGCTCA
31051 ACGACGAGTC GGTGAGCTCC TCGCTTGGTC TCCGTCCGGA CGGGACATTT
31101 CAGATCGGCG GCGCCGGCCG TCCTTCATTC ACGCCTCGTC AGGCAATCCT
31151 AACTCTGCAG ACCTCGTCCT CTGAGCCGCG CTCTGGAGGC ATTGGAACTC
31201 TGCAATTTAT TGAGGAGTTT GTGCCATCGG TCTACTTTAA CCCCTTCTCG
31251 GGACCTCCCG GCCACTATCC GGATCAATTT ATTCCTAACT TTGACGCGGT
31301 AAAGGACTCG GCGGACGGCT ACGACTGAAT GTTAAGTGGA GAGGCAGAGC
31351 AACTGCGCCT GAAACACCTG GTCCACTGTC GCCGCCACAA GTGCTTTGCC
31401 CGCGACTCCG GTGAGTTTTG CTACTTTGAA TTGCCCGAGG ATCATATCGA
31451 GGGCCCGGCG CACGGCGTCC GGCTTACCGC CCAGGGAGAG CTTGCCCGTA
31501 GCCTGATTCG GGAGTTTACC CAGCGCCCCC TGCTAGTTGA GCGGGACAGG
31551 GGACCCTGTG TTCTCACTGT GATTTGCAAC TGTCCTAACC TTGGATTACA
31601 TCAAGATCTT TGTTGCCATC TCTGTGCTGA GTATAATAAA TACAGAAATT
31651 AAAATATACT GGGGCTCCTA TCGCCATCCT GTAAACGCCA CCGTCTTCAC
31701 CCGCCCAAGC AAACCAAGGC GAACCTTACC TGGTACTTTT AACATCTCTC
31751 CCTCTGTGAT TTACAACAGT TTCAACCCAG ACGGAGTGAG TCTACGAGAG
31801 AACCTCTCCG AGCTCAGCTA CTCCATCAGA AAAAACACCA CCCTCCTTAC
31851 CTGCCGGGAA CGTACGAGTG CGTCACCGGC CGCTGCACCA CACCTACCGC
31901 CTGACCGTAA ACCAGACTTT TTCCGGACAG ACCTCAATAA CTCTGTTTAC
31951 CAGAACAGGA GGTGAGCTTA GAAAACCCTT AGGGTATTAG GCCAAAGGCG
32001 CAGCTACTGT GGGGTTTATG AACAATTCAA GCAACTCTAC GGGCTATTCT
32051 AATTCAGGTT TCTCTAGAAT CGGGGTTGGG GTTATTCTCT GTCTTGTGAT
32101 TCTCTTTATT CTTATACTAA CGCTTCTCTG CCTAAGGCTC GCCGCCTGCT
32151 GTGTGCACAT TTGCATTTAT TGTCAGCTTT TTAAACGCTG GGGTCGCCAC
32201 CCAAGATGAT TAGGTACATA ATCCTAGGTT TACTCACCCT TGCGTCAGCC
32251 CACGGTACCA CCCAAAAGGT GGATTTTAAG GAGCCAGCCT GTAATGTTAC
32301 ATTCGCAGCT GAAGCTAATG AGTGCACCAC TCTTATAAAA TGCACCACAG
 
32351 AACATGAAAA GCTGCTTATT CGCCACAAAA ACAAAATTGG CAAGTATGCT
32401 GTTTATGCTA TTTGGCAGCC AGGTGACACT ACAGAGTATA ATGTTACAGT
32451 TTTCCAGGGT AAAAGTCATA AAACTTTTAT GTATACTTTT CCATTTTATG
32501 AAATGTGCGA CATTACCATG TACATGAGCA AACAGTATAA GTTGTGGCCC
32551 CCACAAAATT GTGTGGAAAA CACTGGCACT TTCTGCTGCA CTGCTATGCT
32601 AATTACAGTG CTCGCTTTGG TCTGTACCCT ACTCTATATT AAATACAAAA
32651 GCAGACGCAG CTTTATTGAG GAAAAGAAAA TGCCTTAATT TACTAAGTTA
32701 CAAAGCTAAT GTCACCACTA ACTGCTTTAC TCGCTGCTTG CAAAACAAAT
32751 TCAAAAAGTT AGCATTATAA TTAGAATAGG ATTTAAACCC CCCGGTCATT
32801 TCCTGCTCAA TACCATTCCC CTGAACAATT GACTCTATGT GGGATATGCT
32851 CCAGCGCTAC AACCTTGAAG TCAGGCTTCC TGGATGTCAG CATCTGACTT
32901 TGGCCAGCAC CTGTCCCGCG GATTTGTTCC AGTCCAACTA CAGCGACCCA
32951 CCCTAACAGA GATGACCAAC ACAACCAACG CGGCCGCCGC TACCGGACTT
33001 ACATCTACCA CAAATACACC CCAAGTTTCT GCCTTTGTCA ATAACTGGGA
33051 TAACTTGGGC ATGTGGTGGT TCTCCATAGC GCTTATGTTT GTATGCCTTA
33101 TTATTATGTG GCTCATCTGC TGCCTAAAGC GCAAACGCGC CCGACCACCC
33151 ATCTATAGTC CCATCATTGT GCTACACCCA AACAATGATG GAATCCATAG
33201 ATTGGACGGA CTGAAACACA TGTTCTTTTC TCTTACAGTA TGATTAAATG
33251 AGACATGATT CCTCGAGTTT TTATATTACT GACCCTTGTT GCGCTTTTTT
33301 GTGCGTGCTC CACATTGGCT GCGGTTTCTC ACATCGAAGT AGACTGCATT
33351 CCAGCCTTCA CAGTCTATTT GCTTTACGGA TTTGTCACCC TCACGCTCAT
33401 CTGCAGCCTC ATCACTGTGG TCATCGCCTT TATCCAGTGC ATTGACTGGG
33451 TCTGTGTGCG CTTTGCATAT CTCAGACACC ATCCCCAGTA CAGGGACAGG
33501 ACTATAGCTG AGCTTCTTAG AATTCTTTAA TTATGAAATT TACTGTGACT
33551 TTTCTGCTGA TTATTTGCAC CCTATCTGCG TTTTGTTCCC CGACCTCCAA
33601 GCCTCAAAGA CATATATCAT GCAGATTCAC TCGTATATGG AATATTCCAA
33651 GTTGCTACAA TGAAAAAAGC GATCTTTCCG AAGCCTGGTT ATATGCAATC
33701 ATCTCTGTTA TGGTGTTCTG CAGTACCATC TTAGCCCTAG CTATATATCC
33751 CTACCTTGAC ATTGGCTGGA AACGAATAGA TGCCATGAAC CACCCAACTT
33801 TCCCCGCGCC CGCTATGCTT CCACTGCAAC AAGTTGTTGC CGGCGGCTTT
33851 GTCCCAGCCA ATCAGCCTCG CCCCACTTCT CCCACCCCCA CTGAAATCAG
33901 CTACTTTAAT CTAACAGGAG GAGATGACTG ACACCCTAGA TCTAGAAATG
33951 GACGGAATTA TTACAGAGCA GCGCCTGCTA GAAAGACGCA GGGCAGCGGC
34001 CGAGCAACAG CGCATGAATC AAGAGCTCCA AGACATGGTT AACTTGCACC
34051 AGTGCAAAAG GGGTATCTTT TGTCTGGTAA AGCAGGCCAA AGTCACCTAC
34101 GACAGTAATA CCACCGGACA CCGCCTTAGC TACAAGTTGC CAACCAAGCG
34151 TCAGAAATTG GTGGTCATGG TGGGAGAAAA GCCCATTACC ATAACTCAGC
34201 ACTCGGTAGA AACCGAAGGC TGCATTCACT CACCTTGTCA AGGACCTGAG
 
34251 GATCTCTGCA CCCTTATTAA GACCCTGTGC GGTCTCAAAG ATCTTATTCC
34301 CTTTAACTAA TAAAAAAAAA TAATAAAGCA TCACTTACTT AAAATCAGTT
34351 AGCAAATTTC TGTCCAGTTT ATTCAGCAGC ACCTCCTTGC CCTCCTCCCA
34401 GCTCTGGTAT TGCAGCTTCC TCCTGGCTGC AAACTTTCTC CACAATCTAA
34451 ATGGAATGTC AGTTTCCTCC TGTTCCTGTC CATCCGCACC CACTATCTTC
34501 ATGTTGTTGC AGATGAAGCG CGCAAGACCG TCTGAAGATA CCTTCAACCC
34551 CGTGTATCCA TATGACACGG AAACCGGTCC TCCAACTGTG CCTTTTCTTA
34601 CTCCTCCCTT TGTATCCCCC AATGGGTTTC AAGAGAGTCC CCCTGGGGTA
34651 CTCTCTTTGC GCCTATCCGA ACCTCTAGTT ACCTCCAATG GCATGCTTGC
34701 GCTCAAAATG GGCAACGGCC TCTCTCTGGA CGAGGCCGGC AACCTTACCT
34751 CCCAAAATGT AACCACTGTG AGCCCACCTC TCAAAAAAAC CAAGTCAAAC
34801 ATAAACCTGG AAATATCTGC ACCCCTCACA GTTACCTCAG AAGCCCTAAC
34851 TGTGGCTGCC GCCGCACCTC TAATGGTCGC GGGCAACACA CTCACCATGC
34901 AATCACAGGC CCCGCTAACC GTGCACGACT CCAAACTTAG CATTGCCACC
34951 CAAGGACCCC TCACAGTGTC AGAAGGAAAG CTAGCCCTGC AAACATCAGG
35001 CCCCCTCACC ACCACCGATA GCAGTACCCT TACTATCACT GCCTCACCCC
35051 CTCTAACTAC TGCCACTGGT AGCTTGGGCA TTGACTTGAA AGAGCCCATT
35101 TATACACAAA ATGGAAAACT AGGACTAAAG TACGGGGCTC CTTTGCATGT
35151 AACAGACGAC CTAAACACTT TGACCGTAGC AACTGGTCCA GGTGTGACTA
35201 TTAATAATAC TTCCTTGCAA ACTAAAGTTA CTGGAGCCTT GGGTTTTGAT
35251 TCACAAGGCA ATATGCAACT TAATGTAGCA GGAGGACTAA GGATTGATTC
35301 TCAAAACAGA CGCCTTATAC TTGATGTTAG TTATCCGTTT GATGCTCAAA
35351 ACCAACTAAA TCTAAGACTA GGACAGGGCC CTCTTTTTAT AAACTCAGCC
35401 CACAACTTGG ATATTAACTA CAACAAAGGC CTTTACTTGT TTACAGCTTC
35451 AAACAATTCC AAAAAGCTTG AGGTTAACCT AAGCACTGCC AAGGGGTTGA
35501 TGTTTGACGC TACAGCCATA GCCATTAATG CAGGAGATGG GCTTGAATTT
35551 GGTTCACCTA ATGCACCAAA CACAAATCCC CTCAAAACAA AAATTGGCCA
35601 TGGCCTAGAA TTTGATTCAA ACAAGGCTAT GGTTCCTAAA CTAGGAACTG
35651 GCCTTAGTTT TGACAGCACA GGTGCCATTA CAGTAGGAAA CAAAAATAAT
35701 GATAAGCTAA CTTTGTGGAC CACACCAGCT CCATCTCCTA ACTGTAGACT
35751 AAATGCAGAG AAAGATGCTA AACTCACTTT GGTCTTAACA AAATGTGGCA
35801 GTCAAATACT TGCTACAGTT TCAGTTTTGG CTGTTAAAGG CAGTTTGGCT
35851 CCAATATCTG GAACAGTTCA AAGTGCTCAT CTTATTATAA GATTTGACGA
35901 AAATGGAGTG CTACTAAACA ATTCCTTCCT GGACCCAGAA TATTGGAACT
35951 TTAGAAATGG AGATCTTACT GAAGGCACAG CCTATACAAA CGCTGTTGGA
36001 TTTATGCCTA ACCTATCAGC TTATCCAAAA TCTCACGGTA AAACTGCCAA
36051 AAGTAACATT GTCAGTCAAG TTTACTTAAA CGGAGACAAA ACTAAACCTG
36101 TAACACTAAC CATTACACTA AACGGTACAC AGGAAACAGG AGACACAACT
 
36151 CCAAGTGCAT ACTCTATGTC ATTTTCATGG GACTGGTCTG GCCACAACTA
36201 CATTAATGAA ATATTTGCCA CATCCTCTTA CACTTTTTCA TACATTGCCC
36251 AAGAATAAAG AATCGTTTGT GTTATGTTTC AACGTGTTTA TTTTTCAATT
36301 GCAGAAAATT TCAAGTCATT TTTCATTCAG TAGTATAGCC CCACCACCAC
36351 ATAGCTTATA CAGATCACCG TACCTTAATC AAACTCACAG AACCCTAGTA
36401 TTCAACCTGC CACCTCCCTC CCAACACACA GAGTACACAG TCCTTTCTCC
36451 CCGGCTGGCC TTAAAAAGCA TCATATCATG GGTAACAGAC ATATTCTTAG
36501 GTGTTATATT CCACACGGTT TCCTGTCGAG CCAAACGCTC ATCAGTGATA
36551 TTAATAAACT CCCCGGGCAG CTCACTTAAG TTCATGTCGC TGTCCAGCTG
36601 CTGAGCCACA GGCTGCTGTC CAACTTGCGG TTGCTTAACG GGCGGCGAAG
36651 GAGAAGTCCA CGCCTACATG GGGGTAGAGT CATAATCGTG CATCAGGATA
36701 GGGCGGTGGT GCTGCAGCAG CGCGCGAATA AACTGCTGCC GCCGCCGCTC
36751 CGTCCTGCAG GAATACAACA TGGCAGTGGT CTCCTCAGCG ATGATTCGCA
36801 CCGCCCGCAG CATAAGGCGC CTTGTCCTCC GGGCACAGCA GCGCACCCTG
36851 ATCTCACTTA AATCAGCACA GTAACTGCAG CACAGCACCA CAATATTGTT
36901 CAAAATCCCA CAGTGCAAGG CGCTGTATCC AAAGCTCATG GCGGGGACCA
36951 CAGAACCCAC GTGGCCATCA TACCACAAGC GCAGGTAGAT TAAGTGGCGA
37001 CCCCTCATAA ACACGCTGGA CATAAACATT ACCTCTTTTG GCATGTTGTA
37051 ATTCACCACC TCCCGGTACC ATATAAACCT CTGATTAAAC ATGGCGCCAT
37101 CCACCACCAT CCTAAACCAG CTGGCCAAAA CCTGCCCGCC GGCTATACAC
37151 TGCAGGGAAC CGGGACTGGA ACAATGACAG TGGAGAGCCC AGGACTCGTA
37201 ACCATGGATC ATCATGCTCG TCATGATATC AATGTTGGCA CAACACAGGC
37251 ACACGTGCAT ACACTTCCTC AGGATTACAA GCTCCTCCCG CGTTAGAACC
37301 ATATCCCAGG GAACAACCCA TTCCTGAATC AGCGTAAATC CCACACTGCA
37351 GGGAAGACCT CGCACGTAAC TCACGTTGTG CATTGTCAAA GTGTTACATT
37401 CGGGCAGCAG CGGATGATCC TCCAGTATGG TAGCGCGGGT TTCTGTCTCA
37451 AAAGGAGGTA GACGATCCCT ACTGTACGGA GTGCGCCGAG ACAACCGAGA
37501 TCGTGTTGGT CGTAGTGTCA TGCCAAATGG AACGCCGGAC GTAGTCATAT
37551 TTCCTGAAGC AAAACCAGGT GCGGGCGTGA CAAACAGATC TGCGTCTCCG
37601 GTCTCGCCGC TTAGATCGCT CTGTGTAGTA GTTGTAGTAT ATCCACTCTC
37651 TCAAAGCATC CAGGCGCCCC CTGGCTTCGG GTTCTATGTA AACTCCTTCA
37701 TGCGCCGCTG CCCTGATAAC ATCCACCACC GCAGAATAAG CCACACCCAG
37751 CCAACCTACA CATTCGTTCT GCGAGTCACA CACGGGAGGA GCGGGAAGAG
37801 CTGGAAGAAC CATGTTTTTT TTTTTATTCC AAAAGATTAT CCAAAACCTC
37851 AAAATGAAGA TCTATTAAGT GAACGCGCTC CCCTCCGGTG GCGTGGTCAA
37901 ACTCTACAGC CAAAGAACAG ATAATGGCAT TTGTAAGATG TTGCACAATG
37951 GCTTCCAAAA GGCAAACGGC CCTCACGTCC AAGTGGACGT AAAGGCTAAA
38001 CCCTTCAGGG TGAATCTCCT CTATAAACAT TCCAGCACCT TCAACCATGC
 
38051 CCAAATAATT CTCATCTCGC CACCTTCTCA ATATATCTCT AAGCAAATCC
38101 CGAATATTAA GTCCGGCCAT TGTAAAAATC TGCTCCAGAG CGCCCTCCAC
38151 CTTCAGCCTC AAGCAGCGAA TCATGATTGC AAAAATTCAG GTTCCTCACA
38201 GACCTGTATA AGATTCAAAA GCGGAACATT AACAAAAATA CCGCGATCCC
38251 GTAGGTCCCT TCGCAGGGCC AGCTGAACAT AATCGTGCAG GTCTGCACGG
38301 ACCAGCGCGG CCACTTCCCC GCCAGGAACC ATGACAAAAG AACCCACACT
38351 GATTATGACA CGCATACTCG GAGCTATGCT AACCAGCGTA GCCCCGATGT
38401 AAGCTTGTTG CATGGGCGGC GATATAAAAT GCAAGGTGCT GCTCAAAAAA
38451 TCAGGCAAAG CCTCGCGCAA AAAAGAAAGC ACATCGTAGT CATGCTCATG
38501 CAGATAAAGG CAGGTAAGCT CCGGAACCAC CACAGAAAAA GACACCATTT
38551 TTCTCTCAAA CATGTCTGCG GGTTTCTGCA TAAACACAAA ATAAAATAAC
38601 AAAAAAACAT TTAAACATTA GAAGCCTGTC TTACAACAGG AAAAACAACC
38651 CTTATAAGCA TAAGACGGAC TACGGCCATG CCGGCGTGAC CGTAAAAAAA
38701 CTGGTCACCG TGATTAAAAA GCACCACCGA CAGCTCCTCG GTCATGTCCG
38751 GAGTCATAAT GTAAGACTCG GTAAACACAT CAGGTTGATT CATCGGTCAG
38801 TGCTAAAAAG CGACCGAAAT AGCCCGGGGG AATACATACC CGCAGGCGTA
38851 GAGACAACAT TACAGCCCCC ATAGGAGGTA TAACAAAATT AATAGGAGAG
38901 AAAAACACAT AAACACCTGA AAAACCCTCC TGCCTAGGCA AAATAGCACC
38951 CTCCCGCTCC AGAACAACAT ACAGCGCTTC ACAGCGGCAG CCTAACAGTC
39001 AGCCTTACCA GTAAAAAAGA AAACCTATTA AAAAAACACC ACTCGACACG
39051 GCACCAGCTC AATCAGTCAC AGTGTAAAAA AGGGCCAAGT GCAGAGCGAG
39101 TATATATAGG ACTAAAAAAT GACGTAACGG TTAAAGTCCA CAAAAAACAC
39151 CCAGAAAACC GCACGCGAAC CTACGCCCAG AAACGAAAGC CAAAAAACCC
39201 ACAACTTCCT CAAATCGTCA CTTCCGTTTT CCCACGTTAC GTAACTTCCC
39251 ATTTTAAGAA AACTACAATT CCCAACACAT ACAAGTTACT CCGCCCTAAA
39301 ACCTACGTCA CCCGCCCCGT TCCCACGCCC CGCGCCACGT CACAAACTCC
39351 ACCCCCTCAT TATCATATTG GCTTCAATCC AAAATAAGGT ATATTATTGA
39401 TGAT (SEQ ID NO:4)
**humanTERT promoter sequence:** 
GGCCCCTCCC TCGGGTTACC CCACAGCTTA GGCCGATTCG
ACCTCTCTCC GCTGGGGCCC TCGCTGGCGT CCCTGCACCC TGGGAGCGCG
AGCGGCGCGC GGGCGGGGAA GCGCGGCCCA GACCCCCGGG TCCGCCCGGA
GCAGCTGCGC TGTCGGGGCC AGGCCGGGCT CCCAGTGGAT TCGCGGGCAC
AGACGCCCAG GACCGCGCTT CCCACGTGGC GGAGGGACTG GGGACCCGGG
CACCCGTCCT GCCCCTTCAC CTTCCAGCTC CGCCTCCTCC GCGCGGACCC
CGCCCCGTCC CGACCCCTCC CGGGTCCCCG GCCCAGCCCC CTCCGGGCCC
TCCCAGCCCC TCCCCTTCCT TTCCGCGGCC CCGCCCTCTC CTCGCGGCGC
 
GAGTTTCAGG CAGCGCTGCG TCCTGCTGCG CACGTGGGAA GCCCTGGCCC
CGGCCACCCC CGCG (SEQ ID NO:5)
**Human sTGFβRIIFc sequence:** 
MGRGLLRGLWPLHIVLWTRIASTIPPHVQKSVNNDMIVTDNNGAVKFPQ
L CKFCDVRFSTCDNQKSCMSNCSITSICEKPQEVCVAVWRKNDENITLETV
CHDPKLPYHDFILEDAASPKCIMKEKKKPGETFFMCSCSSDECNDNIIFS
EEYNTSNPDMDPKSCDKTHTCPPCPAPELLGGPSVFLFPPKPKDTLMISR
TPEVTCVVVDVSHEDPEVKFNWYVDGVEVHNAKTKPREEQYNSTYRVVSV
LTVLHQDWLNGKEYKCKVSTKALPAPIEKTISKAKGQPREPQVYTLPPSR
DELTKNQVSLTCLVKGFYPSDIAVEWESNGQPENNYKATPPVLDSDGSFF
LYSKLTVDKSRWQQGNVFSCSVMHEALHNHYTQKSLSLSPGK (SEQ ID NO:6)
**E1A sequence:** 
MRHIICHGGVITEEMAASLLDQLIEEVLADNLPPPSHFEPPTLHELYDLD
VTAPEDPNEEAVSQIFPDSVMLAVQEGIDLLTFPPAPGSPEPPHLSRQPE
QPEQRALGPVSMPNLVPEVIDLTCHEAGFPPSDDEDEEGEEFVLDYVEHP
GHGCRSCHYHRRNTGDPDIMCSLCYMRTCGMFVYSK (SEQ ID NO:7)
**GM-CSF sequence:** 
MWLQSLLLLGTVACSISAPARSPSPSTQPWEHVNAIQEARRLLNLSRDTA
AEMNETVEVISEMFDLQEPTCLQTRLELYKQGLRGSLTKLKGPLTMMASH
YKQHCPPTPETSCATQIITFESFKENLKDFLLVIPFDCWEPVQE (SEQ ID NO:2)
**E1B55K sequence:**

MERRNPSERGVPAGFSGHASVESGCETQESPATVVFRPPGDNTDGGAAA
A AGGSQAAAAGAEPMEPESRPGPSGMNVVQVAELYPELRRILTITEDGQGL
KGVKRERGACEATEEARNLAFSLMTRHRPECITFQQIKDNCANELDLLAQ
KYSIEQLTTYWLQPGDDFEEAIRVYAKVALRPDCKYKISKLVNIRNCCYI
SGNGAEVEIDTEDRVAFRCSMINMWPGVLGMDGVVIMNVRFTGPNFSGTV
FLANTNLILHGVSFYGFNNTCVEAWTDVRVRGCAFYCCWKGVVCRPKSRA
SIKKCLFERCTLGILSEGNSRVRHNVASDCGCFMLVKSVAVIKHNMVCGN
CEDRASQMLTCSDGNCHLLKTIHVASHSRKAWPVFEHNILTRCSLHLGNR
RGVFLPYQCNLSHTKILLEPESMSKVNLNGVFDMTMKIWKVLRYDETRTR
CRPCECGGKHIRNQPVMLDVTEELRPDHLVLACTRAEFGSSDEDTD (SEQ ID NO:8)

### PROPHETIC EXAMPLES 2-6:

### EXAMPLE 2: CHARACTERIZATION OF EXPRESSION FROM RAD.ST.GM, RAD.ST, AND RAD.ST.GM VECTORS

**Cell lines and adenoviruses.** Human mammary tumor cell lines, MCF-7 (ATCC, Manassas, VA), MDA-MB-231 (ATCC, Manassas, VA), and the mouse mammary tumor cell line, 4T1 (ATCC, Manassas, VA) are maintained as described earlier (Katayose, D., et al., (1995) Clin Cancer Res 1, 889-897; Craig, C., et al. (1997) Oncogene 14, 2283-2289; Zhang, Z., et al. (2012) Cancer Gene Ther 19, 630-636). All media components are purchased from Thermo Fisher Scientific (Waltham, MA).

### sTGFβRIIFc and GM-CSF expression in breast cancer cells infected with rAd. sT.GM

Experiments are conducted to examine if the infection of breast cancer cells can produce high levels of sTGFβRIIFc. Breast tumor cells (0.2x10⁶ cells per well in 6-well plates) are plated in DMEM containing 10% FBS and incubated at 37°C overnight. The next morning, cells are infected with 100 plaque-forming units (pfu)/cell of rAd.sT.GM, rAd.sT, rAd.GM, or rAd.Null adenovirus for 24 hours. Cells are washed and incubated with DMEM without FBS for 24 hours. Media and cells are subjected to Western blot analysis as previously described (Katayose, D., et al., (*supra*); Craig, C., (*supra*))*.* Blots are probed with antibody reactive against TGFβRII (H-567; Santa Cruz Biotechnology, Santa Cruz, CA), or actin protein (I-19; Santa Cruz Biotechnology, Santa Cruz, CA) as control.

**Expected Results.** Infection of MDA-MB-231 and MCF-7 cells with rAd.sT.GM and rAd.sT show a 60-80 Kd protein band of sTGFβRIIFc. in the extracellular media. However, sTGFβRIIFc protein is not detectable in the media of the tumor cells infected with rAd.GM or rAd.Null.

### sTGFβRIIFc and GMCSF expression in human breast cancer cells infected with rAd.sT.GM

**Methods.** Supernatants obtained from the rAd.sT.GM, rAd.sT, rAd.GM, or rAd.Null infected cells are also analyzed for sTGFβRIIFc and GM-CSF expression by ELISA, using a published method (Yang, Y. A., et al., (2002) J Clin Invest 109, 1607-1615). In brief, 96-well plates (Nunc, USA) are coated with the anti-human IgG-Fc specific capture antibody (Santa Cruz Biotechnology, Inc., Santa Cruz, CA), incubated with various dilutions of the samples, followed by detection with biotinylated anti-human TGFβRII antibody (R&D systems, Minneapolis, MN). The detection is carried out with streptavidin-conjugated peroxidase using the TMB/HRP substrate (BioFX Laboratories, Owing Mills, MD). After stopping the reaction with 1N HCl, the absorbance is measured at 450 nm using a SPECTRA max Plus ELISA plate reader (Molecular Devices, Sunnyvale, CA). Standard curves of sTGFβRIIFc are used to calculate the sTGFβRIIFc concentrations in the test samples. Human GM-CSF levels are determined using a commercially available ELISA kit from Invitrogen.

**Expected Results.** Based on the results reported in Example 1, incorporation of an additional GMCSF gene in rAd.sT.GM, does not seem to adversely affect the expression of sTGFβRIIFc. Thus, infection of breast tumor cells with rAd.sT.GM and rAd.sT is expected to produce sTGFβRIIFc that is subsequently released into the media and breast tumor cells infected with rAd.sT.GM or rAd.GM are expected to produce GMCSF.

### EXAMPLE 3: ADENOVIRUS CYTOTOXICITY AND REPLICATION

**Adenovirus cytotoxicity.** Tumor cells are seeded in 96-well plates at a density of 1×10³ cells/well, and the following day they are infected with various doses of oncolytic adenoviruses (rAd.sT.GM, rAd.sT, rAd.GM, or rAd.Null) and a non-replicating virus Ad(E1-).Null, ranging from 2 VPs/cell to 1.25 × 10⁵ VPs/cell. Incubations are continued for seven days. Cell survival is determined by the sulforhodamine B staining assay (Katayose, D., et al., (*supra*)).

**Expected Results.** All oncolytic viruses including rAd.sT.GM are expected to produce dose-dependent cytotoxicity in human breast tumor cells, as well as mouse mammary tumor cells. Based on the IC50 values (viral dose required to kill 50% of the cells), oncolytic viruses are usually about 100 times more cytotoxic than a non-replicating virus such as Ad(E1-).Null.

**Viral replication assay.** Cells are plated in 6-well plates at about 70% confluence and the viral titers are examined using a published method (Seth, P., et al. (2006) Hum Gene Ther 17, 1152-1160; Wang, Z. G., et al. (2006) Mol Cancer Ther 5, 367-373) with some modifications. Cells are infected with rAd.sT.GM, rAd.sT, rAd.GM, rAd.Null or Ad(E1⁻).Null for 3 hours at a multiplicity of infection (MOI) of 100. Cells are washed with DMEM and incubated in 1 ml DMEM for additional one hour at 37°C. At the end of the incubation, cells are washed and either collected in 0.5 ml growth media and frozen at -70°C or are maintained in 2 ml of growth media for an additional 48 hours. Media and cells are collected, and cells are subjected to three cycles of freezing and thawing to release the viruses. The viral titers in 3-hour or 48-hour crude viral lysates are determined using the Adeno-X Rapid titer kit (Clutch, Mountain view, CA). Viral burst size (an increase in hexon-expressing positive cells from 3 hours to 48 hours), is used as an indicator of viral replication, as described (Hu, Z., et al. (2012) Hum Gene Ther 23, 871-882).

**Expected Results.** rAd.sT.GM is expected to replicate in human breast cancer cells, MDA-MB-231 and MCF-7. At 3 hours, viral titers may be quite low, which should significantly increase at 48 hours (increase in viral titers from 3 hours to 48 hours is described as the "viral burst size"). On the other hand, infection of MDA-MB-231 or MCF-7 cells with Ad(E1⁻).Null is expected to result in less than a 2-fold increase in viral burst size, since Ad(E1⁻).Null is expected to be replication-deficient in the breast cancer cells.

### EXAMPLE 4: EXPRESSION OF STGFBRIIFC IN VIVO AND ORTHOTOPIC XENOGRAFT MODEL

**Methods.** 4T1luc2 mouse tumors are established in BALB/c mice. Various viruses (Ad.sT, rAd.sT.GM, rAd.GM and rAd.Null) and buffer are administered intratumorally into 4T1luc2 tumors (5.0 × 10¹⁰ VPs/mouse). After 48 hours, the sTGFβRIIFc levels in the blood are measured by ELISA, as described herein. To detect sTGFβRIIFc expression in the tumors, tumor sections are also subjected to immunohistochemical (IHC) staining for sTGFβRIIFc.

**Expected Results.** The intratumoral injection of rAd.sT.GM and rAd.sT are expected to result in secretion of sTGFβRIIFc and its detection in the blood. Intratumoral injection of rAd.sT.GM and rAd.GM is expected to result in secretion of GMCSF and its detection in the blood. sTGFβRIIFc expression is expected to be readily detected in the subcutaneous tumors of mice injected with rA.sT.GM and rAd.sT.

To establish an orthotopic xenograft model of breast cancer, 6.5×10⁵ 4T1-luc cells (100µl) are injected into number 3 and 4 mammary fat pads of BALB/c mice (4-6 weeks old). When tumors are visible (on day 7 after cell injection), tumor volumes are measured by caliper and calculated using the following formula: tumor volume = (Width²×Length)/2. The tumor burden is also analyzed by real-time bioluminescence imaging (BLI).

### EXAMPLE 5: SAFETY/TOXICITY STUDIES FOLLOWING INTRATUMORAL DELIVERY OF ONCOLYTIC VIRUSES IN BALB/c MICE BEARING 4T1LUc2 TUMORS

**Methods.** To establish subcutaneous tumors, 2 × 10⁶ 4T1luc2 cells per mouse (day 0) are injected into the dorsal right flank of female BALB/c mice (6-8 weeks old). Mice are monitored every day. When the solid subcutaneous tumors are established (day 12), rAd.sT.GM, rAd.sT, rAd.GM and the vehicle control (PBS buffer) are administered intratumorally (5.0 × 10¹⁰ VPs in 100 µl per mouse). Two days (48 hours) after the viral injection (on day 14), whole-mouse blood is withdrawn via cardiac puncture under anesthesia before animals are euthanized. Subcutaneous tumors are removed. Mouse serum is prepared from the blood and kept frozen. Tumor samples are fixed in 10% NBF solution for histology sample preparation and analyses are performed as described (Xu, W., et al. (2014) Mol Ther 22, 1504-1517).

Hepatic and systemic toxicity following the intratumoral administration of the test oncolytic viral vectors in BALB/c mice are evaluated. Serum samples obtained from the above experiment are analyzed for alanine aminotransferase (ALT), aspartate aminotransferase (AST), and lactate dehydrogenase (LDH) levels. ALT is generally considered a good marker for hepatic damage and LDH is a good indicator of systemic toxicity (Xu, W., et al. (*supra*)). Commercially available kits are used to detect enzyme levels.

**Expected Results.** Subclinical hepatic injury is possible, but the serum values of ALT, AST and LDH are expected to be within normal limits and not to exceed 2.5 times the upper limit of normal in most individual test subjects.

### EXAMPLE 6: COMBINATION THERAPY

The effectiveness of the combination of rAd.sT.GM with immune checkpoint inhibitors anti-PD-1 and anti-CTLA-4 in inhibiting tumor growth and metastases is tested using 4T1 tumors established in BALB/c mice.

**Methods.** As described above, 4T1 cells are injected (day 0) into the right flank of 4-6-week-old BALB/c mice (5x10⁶ cells/mouse). On day 7 following tumor cell injections, tumor volumes are calculated by the following formula: tumor volume = (Width²×Length)/2. Tumor bearing mice are divided into these groups: (i) rAd.sT.GM, (ii) anti-PD-1, (iii) anti-CTLA-4, (iv) anti-PD1and anti-CTLA-4, (v) rAd.sT.GM, anti-PD-1, (vi) rAd.sT.GM and anti-CTLA-4, (vii) rAd.sT.GM, anti-PD-1 and anti-CTLA-4, and (viii) buffer groups, without statistical differences among the groups. On day 7, rAd.sT,GM (2.5×10¹⁰ VPs in 100µl) or PBS is administrated directly into the tumors. A second injection (2.5×10¹⁰ VPs or PBS) is administered on day 10. On days 8, 10, 13, and 15, anti-PD-1 and anti-CTLA-4 antibodies are administered individually or together intraperitoneally (0.2 mg per mouse/each antibody) in the groups indicated as receiving the listed agent. The tumor volumes in the injected areas are monitored on day 10, 14, 17, 21, and 25. Mice are euthanized on day 25. Whole lungs are excised and photographed. Lung-surface tumor lesions are counted for each group. Parts of the lungs are processed for H&E staining according to a published protocol (Yang, Y., et al. (2019) Hum Gene Ther 30, 1117-1132). Pulmonary metastatic burdens are quantified by using Image J software (NIH, Bethesda, MD) for each lung section.

**Expected Results.** The triple treatment is expected to be more effective in reducing tumor volume and metastasis than dual treatments and single treatment.

Statistical analysis. All data are analyzed using GraphPad Prism software version 5 (GraphPad software, San Diego, CA). For all statistical analyses, data are plotted as the mean ± standard error of the mean (SEM). Longitudinal data are analyzed using a two-way repeated measure ANOVA followed by Bonferroni *post hoc* pair wise tests for the data obtained over the time course. For multiple group analyses, one-way ANOVA followed by Bonferroni *post hoc* tests adjusting for multiplicity are performed. Student's *t*-tests are performed to compare two sets of data. Differences are considered significant at two-sided *p* < 0.05.

### EXAMPLE 7: ADENOVIRAL-MEDIATED REPLICATION AND CYTOTOXICITY ASSAYS

All viral vectors were produced and purified to Good Laboratory Practice standards in Vector Development Lab/ Gene Vector Core of Baylor College of Medicine, Houston, TX. They also determined the concentration and titers of these viruses and performed sterility and endotoxin assays for the quality control and verification. All were maintained in 20mM Hepes, pH 7.8, 150mM NaCl + 10% glycerol buffer with the concentration of 5.0x1012 viral particles (VPs)/ml and stored in -80°C deep freezer with 500µl/aliquot. The total viral particles for each virus are ranging from 3.0-4.9x10¹³. Their titers are ranging from 1.6x10¹¹-1.0x10¹² pfu/ml. All viruses are tested negative for sterility. Their endotoxin levels are < 0.1 EU/ml except for rAd.Null (0.15 EU/ml). They all are below the endotoxin limits set by FDA for an investigational drug in the early clinical development.

For characterization, adenoviral-mediated replication and cytotoxicity assays were performed and adenoviral-mediated sTGFβRIIFc and GM-CSF expression was examined in both human and mouse breast cancer cell lines.

Briefly, for replication assay, human breast cancer cells (MDA-MB-231 and MCF-7) and mouse breast cancer cells (4T1-luc2) were plated into a 6-well plates (2x10⁵ cells/well). The next day, cells were infected with 2.5x10⁴ VPs/cell of adenoviruses. Three hours after infection, cells were washed with phosphate-buffered saline buffer (PBS), and crude viral lysates were either collected immediately (3 h samples) or 48 hours after infection with completed cell culture media. The viral titers in 3- or 48-h crude viral lysates were determined using the Adeno-X Rapid Titer Kit (Clutch, Mountain view, CA). Viral burst size (an increase in hexon-expressing positive cells from 3 to 48 h) was used as an indicator of viral replication.

For cytotoxicity assay, breast cancer cells were seeded into 96-well plates (1.0x10³ cells/well). The next day, cells were infected with various doses of adenoviruses (ranging from 80 to 1.25x10⁶ VPs/cell and continued to culture for 7 days. Uninfected cells were used as the control. Cell survival was determined by sulforhodamine B staining and the lab's standard protocol described in previous publications.

For Adenoviral-mediated sTGFβRIIFc and GM-CSF expression, breast cancer cells were plated into a 6-well plates (2x10⁵ cells/well). The next day, cells were infected with 2.5x10⁴ VPs/cell of adenoviruses. Twenty-four hours after infection, cells were washed with PBS, and the media were changed to serum-free media. Then, the incubations continued for another 24 hours and media were collected. To quantify sTGFβRIIFc protein in media, enzyme-linked immunosorbent assay (ELISA) was conducted using anti-human IgG, Fcγ Fragment Specific antibody and biotinylated anti-TGFβ RII (BAF241) antibody. To quantify GM-CSF protein in media, anti-human GM-CSF monoclonal antibody (Clone 6804, ELISA Capture) and biotinylated anti- human GM-CSF monoclonal antibody (Clone 3209, ELISA Detection) were used in ELISA.

### Results

All replicating adenoviruses: rAd.sT.GM, rAd.GM, rAd.sT and rAd.Null produced much higher levels of viral replication in human breast cancer cells (MDA-MB-231 and MCF-7) (with an average ratio of 1506 for 231 and 2699 for MCF-7) than those in mouse breast cancer cells (4T1-luc2) (Average is 2.45). It is consistent with the nature of human adenovirus and our previous studies (Zhang, et al, 2021, doi:10.1038/cgt.2012.41; Xu, et al, 2020, DOI: 10.1089/hum.2020.078). However, when comparing to the control replication-deficient Ad(E1-), all replicating viruses have significantly higher replication levels in all cells.

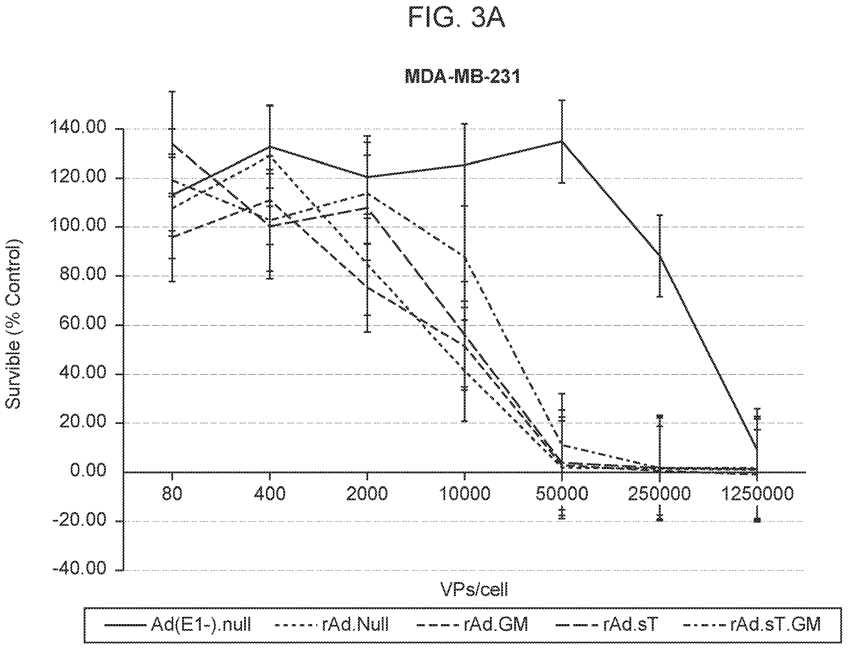

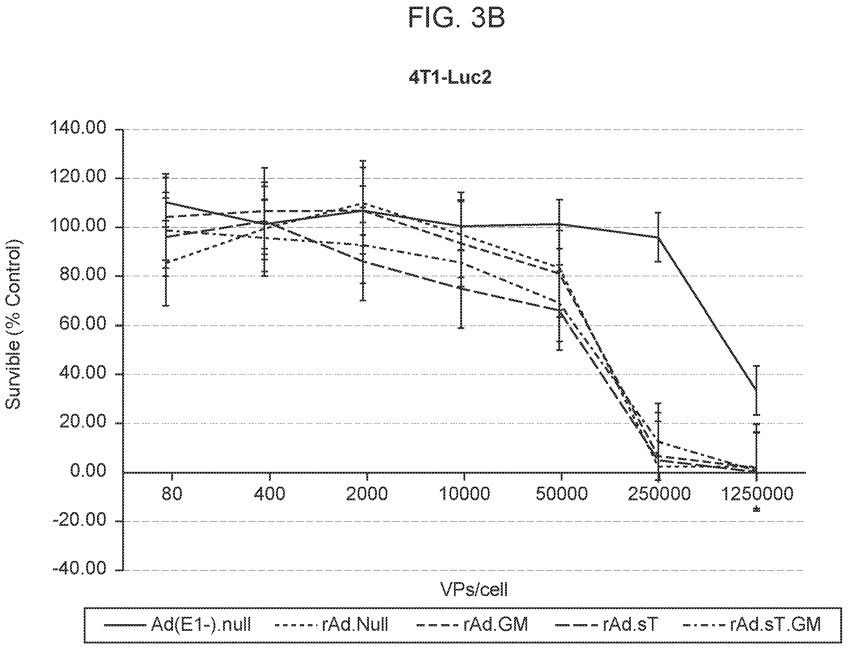

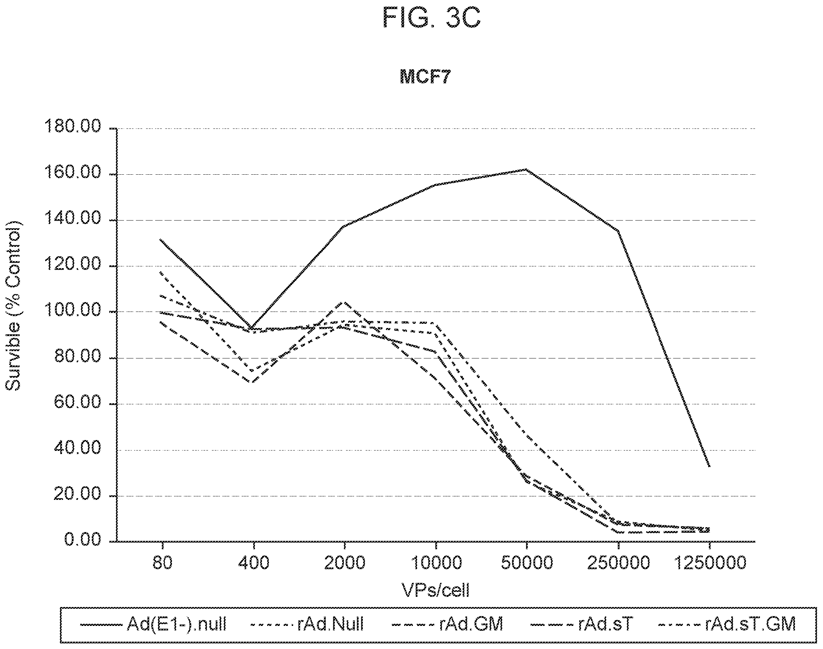

Cytotoxicity assay. All adenoviruses: rAd.sT.GM, rAd.GM, rAd.sT, rAd.Null and replication-deficient Ad(E1-). Null produced dose-dependent cytotoxicity in both human and mouse breast cancer cells. Cytotoxicity of replicating adenoviruses (50% survival rate at 1.0-5.0 x10⁴ VPs/cell in human cells and 1.0 x10⁵ VPs/cell in mouse cells) is much greater than replication-deficient Ad(E1-). Null (50% survival rate is at around 1.0x10⁶ VPs/cell in all cells). Mouse breast cancer cells 4T1, even with the much lower replication potentials for all replicating viruses, is susceptible to the killing ability of adenoviruses. See Figs. 3A-3C. This suggests, in addition to viral replication, there might be additional mechanisms and factors that contribute toward viral-induced cytotoxicity in the mouse cells. For example, in this experiment, the prolonged incubation with adenoviruses would increase viral-induced cytotoxicity in the mouse cells. Interestingly, for rAd.sT.GM, even has the lowest levels of replication when comparing to other replicating viruses, can kill 4T1 cells as effectively as the other replicating viruses (50% survival rates are around 1.0 x10⁵ VPs/cell for all replicating viruses).

### EXAMPLE 8: ADENOVIRAL-MEDIATED STGFBRIIFC AND GM-CSF EXPRESSION

Infection of the human and mouse breast cancer cells with rAd.sT.GM and rAd.sT produced high levels of sTGFβRIIFc protein, which was secreted into the cell culture media and detected by sTGFβRIIFc ELISA. sTGFβRIIFc protein concentration in those media are ranging from 3.9-5.3 µg/ml with an average amount of 4.5 µg/ml by our ELISA method. No sTGFβRIIFc expression was detected in cells infected with rAd.GM, rAd.Null and Ad(E1-). Null. There was no significant difference of sTGFβRIIFc expression levels among all cell types and between cell infected with rAd.sT.GM and those with rAd.sT.

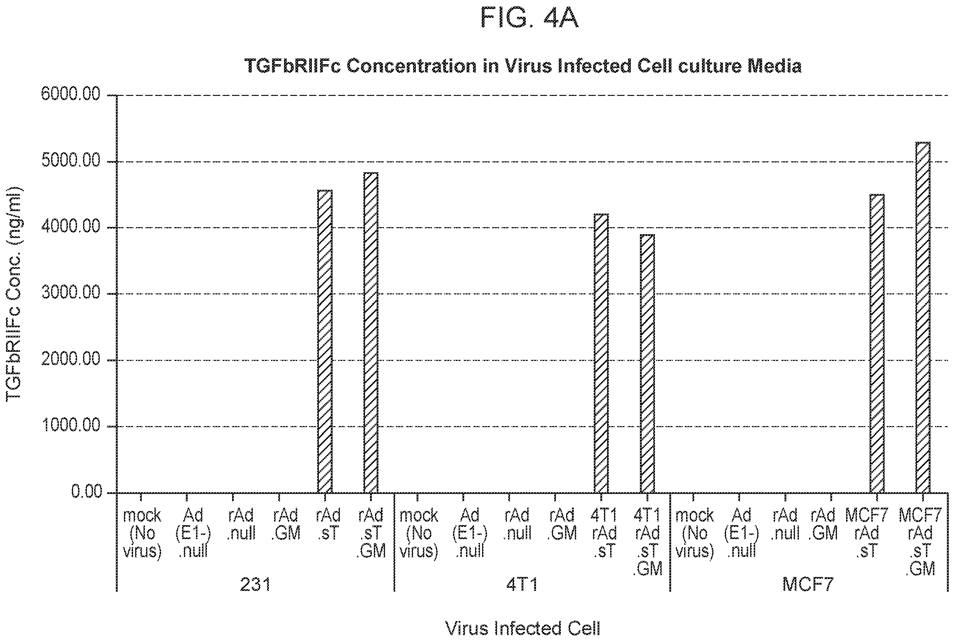

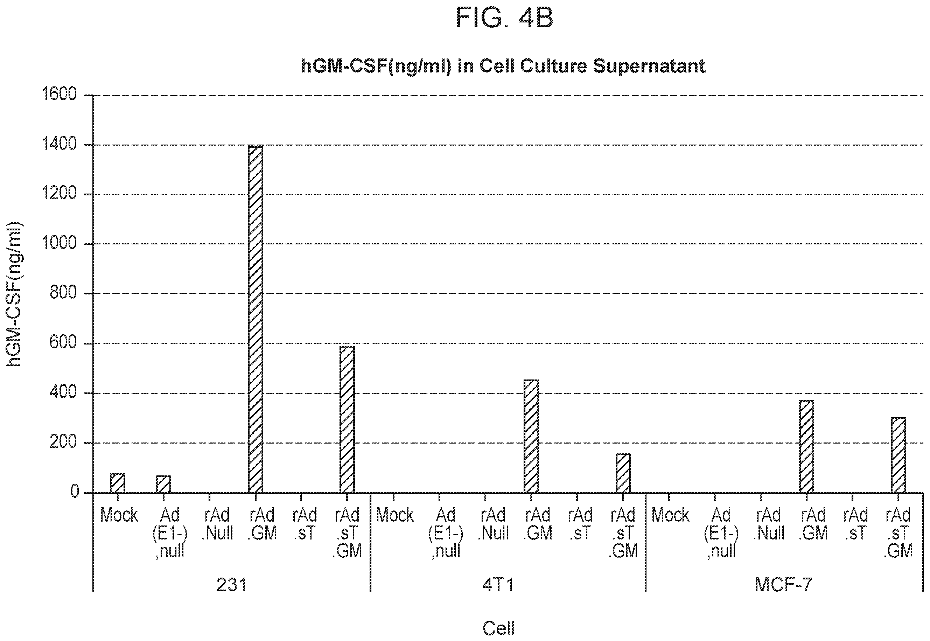

For GM-CSF expression, the high levels of GM-CSF were detected only in cells infected with adenoviruses with GM-CSF gene: rAd.sT.GM and rAd.GM. GM-CSF concentration are ranging from 159-1398 ng/ml with an average amount of 547.5 ng/ml by ELISA. Human breast cancer cells 231 have higher levels of GM-CSF expression (Avg: 995.5 ng/ml) when compared to another human cell MCF7 (Avg: 338.5 ng/ml) and mouse cell 4T1 (Avg: 308.5 ng/ml). It can be due to high replication levels of adenovirus and endogenous expression of human GM-CSF in 231 cells, since a low levels of GM-CSF expression has been detected from the mock and Ad(E1-). Null infection in 231 cells (mock: 78 ng/ml; Ad(E1-). Null: 71 ng/ml). Importantly, 4T1 cells can express high levels of sTGFβRIIFc and/or human GM-CSF when infected with corresponding viruses. Also, the new adenoviruses: rAd.sT.GM elicited high levels of sTGFβRIIFc protein and human GM-CSF in all cells types including mouse breast cancer cell line: 4T1. See FIGS. 4A-4B.

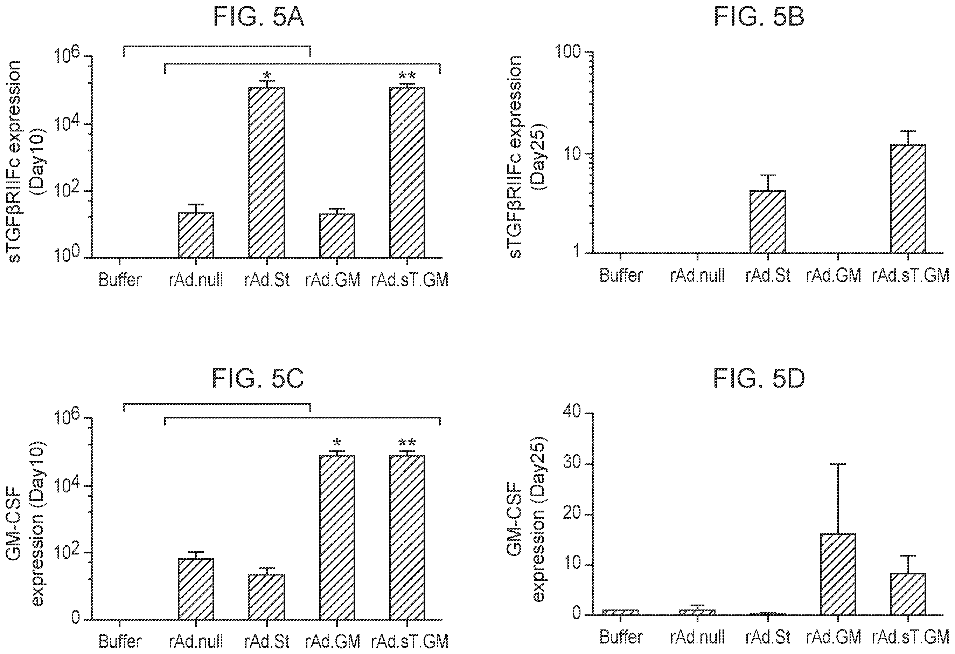

The expression of sTGFβRIIFc and human GM-CSF in 4T1 tumors from all groups was also quantified by qRT-PCR. sTGFβRIIFc expression in tumors (day 10) from rAd.sT or rAd.sT.GM treated mice is 115717.69 or 118065.27 times (average value of relative quantification (RQ)) higher than that of the buffer group. By using non-parametric Kruskal-Wallis one-way analysis of variance to compare all groups, rAd.sT vs the buffer group is P < 0.05 and rAd.sT.GM vs the buffer group is P < 0.01. This statistical difference is due to the rAd.sT.GM-treated group has less variation in sTGFβRIIFc expression which can be visually assessed by the sizes of error bars on the graph. We also analyzed sTGFβRIIFc expression in day 25 tumor samples. sTGFβRIIFc mRNA level is 4.338 times (average) higher than that of the buffer group in rAd.sT-treated samples and 11.940 times (average) higher in rAd.sT.GM-treated samples. No significant difference has been detected among all groups by using Kruskal-Wallis test. Next, viral-induced human GM-CSF expression in tumors was determined by qRT-PCR. On day 10 (viruses were injected intratumorally on day 7 and day 9), the average expression levels by RQ are 74526.09 times higher than that of the buffer group in rAd.GM treated tumors, and 79320.71 times higher in rAd.sT.GM treated tumors. By non-parametric Kruskal-Wallis one-way analysis, rAd.GM vs the buffer group is P < 0.05 and rAd.sT.GM vs the buffer group is P < 0.01. The variation of GM-CSF expression in rAd.sT.GM treated group is smaller than that in rAd.GM group, and as a result, it is more significant by Kruskal-Wallis test. For day 25 samples, the average expression levels of human GM-CSF are 4.34 times higher than that of the buffer group in rAd.GM treated tumors, and 11.94 times higher in rAd.sT.GM treated tumors, but no significant difference has been detected among all day 25 groups by using Kruskal-Wallis test. When taken together with relative quantification data of sTGFβRIIFc, not only expression of either sTGFβRIIFc or human GM-CSF elicited by rAd.sT.GM didn't interfere with each other, but also rAd.sT.GM seems to have a more stable expression profile and a more prolonged presence of both sTGFβRIIFc and human GM-CSF in tumors. See Figs. 5A-5D.

The scope of present invention is embodied by the appended claims.

## Claims

1. An oncolytic virus comprising a nucleic acid encoding a granulocyte-macrophage colony-stimulating factor (GM-CSF) and a soluble form of the TGF-B receptor-II (sTGF-BRII).

2. The oncolytic virus of claim 1, wherein the oncolytic virus is an adenovirus, herpes virus, lentivirus, vaccinia virus, Reo virus, maraba virus, Newcastle disease virus, sendai virus, pox virus, poliovirus, myxoma virus, or retrovirus.

3. The oncolytic virus of claim 1 or 2, wherein the oncolytic virus preferentially replicates in cancer cells.

4. The oncolytic virus of any one of claims 1-3, wherein the expression of genes required for replication of the virus is under the control of a promoter active in the cancer cells, optionally wherein the promoter active in cancer cells comprises a telomerase reverse transcriptase (TERT) promoter.

5. The oncolytic virus of any one of claims 1-4, wherein the expression of sTGF-BRII is under the control of a cytomegalovirus (CMV) promoter, and/or wherein the expression of GM-CSF is under the control of an adenoviral E1B promoter.

6. The oncolytic virus of any one of claims 1-5, wherein the virus comprises a capsid protein that specifically binds to a cancer cell that overexpresses an adenovirus receptor.

7. The oncolytic virus of claim 6, wherein the cancer cell is a breast cancer cell, skin cancer cell, lung cancer cell, renal cancer cell, ovarian cancer cell, prostate cancer cell, pancreatic cancer cell, brain cancer cell, musculoskeletal cancer, astrocytoma cancer cell, cervical cancer cell, testicular cancer cell, hepatic cancer cell, lymphoma cancer cell, colon cancer cell, or bladder cancer cell.

8. The oncolytic virus of claim 7, wherein the cancer cell is:
a) a breast cancer cell, optionally wherein the breast cancer cell is a triple negative breast cancer cell, or
b) a skin cancer cell, optionally wherein the skin cancer cell is a melanocyte.

9. The oncolytic virus of any one of claims 1-8, wherein the nucleic acid encoding the GM-CSF is codon-optimized to increase expression of the GM-CSF in a human subject, optionally wherein the GM-CSF is a human GM-CSF.

10. The oncolytic virus of any one of claims 1-9, wherein the sTGF-BRII is fused to a heterologous protein, optionally wherein the heterologous protein comprises a half-life-extending moiety, further optionally wherein the half-life-extending moiety comprises an immunoglobulin Fc region.

11. A pharmaceutical composition comprising: a therapeutically effective amount of the oncolytic virus of any one of claims 1-10 and a pharmaceutically acceptable excipient, diluent, or carrier.

12. The oncolytic virus of any one of claims 1-10 or the composition of claim 11 for use in a method of treating cancer in a subject, wherein the method comprises administering a therapeutically effective amount of the oncolytic virus of any one of claims 1-10 or the composition of claim 11 to the subject.

13. The oncolytic virus or composition for use of claim 12, wherein the cancer is breast cancer, skin cancer, lung cancer, bladder cancer, renal cancer, astrocytoma, hepatic cancer, lymphoma, colon cancer, or head/neck cancer, optionally wherein the cancer is a primary cancer or metastatic cancer.

14. The oncolytic virus or composition for use of claim 12 or 13, wherein the method further comprises administering to the subject one or more of an immunotherapy, a chemotherapy, a surgical treatment, a radiation therapy, a hormone therapy, an anti-cancer small molecule, a growth factor inhibitor therapy, CAR-T therapy, or a cytokine therapy.

15. The oncolytic virus or composition for use of claim 14, wherein:
a) an immunotherapy is administered to the subject;
b) a chemotherapy is administered to the subject; or
c) an immune checkpoint inhibitor is administered to the subject, optionally wherein the immune checkpoint inhibitor comprises an inhibitor of PD-1, PD-L1, CTLA-4, LAG-3, BCH-3, BCH-4, or TIM-3, further optionally wherein the inhibitor is an antibody.

## Patentansprüche

1. Onkolytisches Virus, umfassend eine Nukleinsäure, die einen Granulozyten-Makrophagenkoloniestimulierenden Faktor (GM-CSF) und eine lösliche Form des TGF-B-Rezeptors II (sTGF-BRII) kodiert.

2. Onkolytisches Virus nach Anspruch 1, wobei das onkolytische Virus ein Adenovirus, Herpesvirus, Lentivirus, Vacciniavirus, Reovirus, Marabavirus, Newcastle-Krankheit-Virus, Sendaivirus, Pockenvirus, Poliovirus, Myxomavirus oder Retrovirus ist.

3. Onkolytisches Virus nach Anspruch 1 oder 2, wobei sich das onkolytische Virus bevorzugt in Krebszellen vermehrt.

4. Onkolytisches Virus nach einem der Ansprüche 1-3, wobei die Expression von Genen, die zur Replikation des Virus erforderlich sind, unter der Kontrolle eines in den Krebszellen aktiven Promotors steht, wobei der in den Krebszellen aktive Promotor gegebenenfalls einen Telomerase-Reverse-Transkriptase-Promotor (TERT-Promotor) umfasst.

5. Onkolytisches Virus nach einem der Ansprüche 1-4, wobei die Expression von sTGF-BRII unter der Kontrolle eines Zytomegalievirus-Promotors (CMV-Promotors) steht und/oder wobei die Expression von GM-CSF unter der Kontrolle eines adenoviralen E1B-Promotors steht.

6. Onkolytisches Virus nach einem der Ansprüche 1-5, wobei das Virus ein Kapsidprotein umfasst, das spezifisch an eine Krebszelle bindet, die einen Adenovirusrezeptor überexprimiert.

7. Onkolytisches Virus nach Anspruch 6, wobei die Krebszelle eine Brustkrebszelle, Hautkrebszelle, Lungenkrebszelle, Nierenkrebszelle, Eierstockkrebszelle, Prostatakrebszelle, Bauchspeicheldrüsenkrebszelle, Hirnkrebszelle, Krebszelle des Bewegungsapparates, Astrozytomkrebszelle, Gebärmutterhalskrebszelle, Hodenkrebszelle, Leberkrebszelle, Lymphomkrebszelle, Darmkrebszelle oder Blasenkrebszelle ist.

8. Onkolytisches Virus nach Anspruch 7, wobei die Krebszelle Folgendes ist:
a) eine Brustkrebszelle, wobei die Brustkrebszelle gegebenenfalls eine dreifach negative Brustkrebszelle ist, oder
b) eine Hautkrebszelle, wobei die Hautkrebszelle gegebenenfalls ein Melanozyt ist.

9. Onkolytisches Virus nach einem der Ansprüche 1-8, wobei die Nukleinsäure, die den GM-CSF kodiert, kodonoptimiert ist, um die Expression des GM-CSF in einem menschlichen Subjekt zu steigern, wobei der GM-CSF gegebenenfalls ein menschlicher GM-CSF ist.

10. Onkolytisches Virus nach einem der Ansprüche 1-9, wobei der sTGF-BRII mit einem heterologen Protein fusioniert ist, wobei das heterologe Protein gegebenenfalls eine halbwertszeitverlängernde Einheit umfasst, wobei die halbwertszeitverlängernde Einheit weiter gegebenenfalls eine Immunglobulin-Fc-Region umfasst.

11. Pharmazeutische Zusammensetzung, umfassend: eine therapeutisch wirksame Menge des onkolytischen Virus nach einem der Ansprüche 1-10 und einen pharmazeutisch verträglichen Hilfsstoff, Verdünner oder Träger.

12. Onkolytisches Virus nach einem der Ansprüche 1-10 oder die Zusammensetzung nach Anspruch 11 zur Verwendung in einem Verfahren zum Behandeln von Krebs bei einem Subjekt, wobei das Verfahren das Verabreichen einer therapeutisch wirksamen Menge des onkolytischen Virus nach einem der Ansprüche 1-10 oder der Zusammensetzung nach Anspruch 11 an das Subjekt umfasst.

13. Onkolytisches Virus oder Zusammensetzung zur Verwendung nach Anspruch 12, wobei die Krebsart Brustkrebs, Hautkrebs, Lungenkrebs, Blasenkrebs, Nierenkrebs, Astrozytom, Leberkrebs, Lymphom, Darmkrebs oder Kopf-Hals-Krebs ist, wobei der Krebs gegebenenfalls einen primärer Krebs oder metastatischer Krebs ist.

14. Onkolytisches Virus oder Zusammensetzung zur Verwendung nach Anspruch 12 oder 13, wobei das Verfahren weiter das Verabreichen von einem oder mehreren von einer Immuntherapie, einer Chemotherapie, einer chirurgischen Behandlung, einer Strahlentherapie, einer Hormontherapie, eines kleinen Moleküls gegen Krebs, einer Wachstumsfaktorinhibitortherapie, CAR-T-Therapie oder einer Zytokintherapie an das Subjekt umfasst.

15. Onkolytisches Virus oder Zusammensetzung zur Verwendung nach Anspruch 14, wobei:
a) dem Subjekt eine Immuntherapie verabreicht wird;
b) dem Subjekt eine Chemotherapie verabreicht wird; oder
c) dem Subjekt ein Immun-Checkpoint-Inhibitor verabreicht wird, wobei der Immun-Checkpoint-Inhibitor gegebenenfalls einen Inhibitor von PD-1, PD-L1, CTLA-4, LAG-3, BCH-3, BCH-4 oder TIM-3 umfasst, wobei der Inhibitor weiter gegebenenfalls ein Antikörper ist.

## Revendications

1. Virus oncolytique comprenant un acide nucléique codant pour un facteur de stimulation des colonies de granulocytes-macrophages (GM-CSF) et une forme soluble du récepteur TGF-B-II (sTGF-BRII).

2. Virus oncolytique selon la revendication 1, dans lequel le virus oncolytique est un adénovirus, un virus herpès, un lentivirus, un virus de la vaccine, un virus de type Reo, un virus Maraba, un virus de la maladie de Newcastle, un virus Sendai, un virus de la variole, un poliovirus, un virus du myxome ou un rétrovirus.

3. Virus oncolytique selon la revendication 1 ou 2, dans lequel le virus oncolytique se réplique préférentiellement dans les cellules cancéreuses.

4. Virus oncolytique selon l'une quelconque des revendications 1-3, dans lequel l'expression des gènes nécessaires à la réplication du virus est régulée par un promoteur actif dans les cellules cancéreuses, éventuellement dans lequel le promoteur actif dans les cellules cancéreuses comprend un promoteur de transcriptase inverse de télomérase (TERT).

5. Virus oncolytique selon l'une quelconque des revendications 1-4, dans lequel l'expression de sTGF-BRII est régulée par un promoteur de cytomégalovirus (CMV), et/ou dans lequel l'expression du GM-CSF est régulée par un promoteur E1B adénoviral.

6. Virus oncolytique selon l'une quelconque des revendications 1-5, dans lequel le virus comprend une protéine capside qui se lie spécifiquement à une cellule cancéreuse qui surexprime un récepteur d'adénovirus.

7. Virus oncolytique selon la revendication 6, dans lequel la cellule cancéreuse est une cellule du cancer du sein, une cellule du cancer de la peau, une cellule du cancer du poumon, une cellule du cancer du rein, une cellule du cancer de l'ovaire, une cellule du cancer de la prostate, une cellule du cancer du pancréas, une cellule du cancer du cerveau, un cancer musculo-squelettique, une cellule du cancer du type astrocytome, une cellule du cancer du col de l'utérus, une cellule du cancer des testicules, une cellule du cancer hépatique, une cellule du cancer de type lymphome, une cellule du cancer du côlon ou une cellule du cancer de la vessie.

8. Virus oncolytique selon la revendication 7, dans lequel la cellule cancéreuse est :
a) une cellule du cancer du sein, éventuellement dans lequel la cellule du cancer du sein est une cellule du cancer du sein triple négatif, ou
b) une cellule du cancer de la peau, éventuellement dans lequel la cellule du cancer de la peau est un mélanocyte.

9. Virus oncolytique selon l'une quelconque des revendications 1-8, dans lequel l'acide nucléique codant pour le GM-CSF est à codons optimisés pour accroître l'expression du GM-CSF chez un sujet humain, éventuellement dans lequel le GM-CSF est un GM-CSF humain.

10. Virus oncolytique selon l'une quelconque des revendications 1-9, dans lequel le sTGF-BRII est fusionné à une protéine hétérologue, éventuellement dans lequel la protéine hétérologue comprend un fragment prolongeant la demi-vie, et éventuellement dans lequel le fragment prolongeant la demi-vie comprend une région Fc d'immunoglobuline.

11. Composition pharmaceutique comprenant : une quantité thérapeutiquement efficace du virus oncolytique de l'une quelconque des revendications 1-10 et un excipient, un diluant ou un véhicule pharmaceutiquement acceptable.

12. Virus oncolytique selon l'une quelconque des revendications 1-10 ou composition selon la revendication 11 pour utilisation dans un procédé de traitement du cancer chez un sujet, dans lequel ou laquelle le procédé comprend l'administration au sujet d'une quantité thérapeutiquement efficace du virus oncolytique de l'une quelconque des revendications 1-10 ou de la composition de la revendication 11.

13. Virus oncolytique ou composition pour utilisation selon la revendication 12, dans lequel ou laquelle le cancer est un cancer du sein, un cancer de la peau, un cancer du poumon, un cancer de la vessie, un cancer du rein, un astrocytome, un cancer hépatique, un lymphome, un cancer du côlon, ou un cancer de la tête et du cou, éventuellement le cancer est un cancer primitif ou cancer métastatique.

14. Virus oncolytique ou composition pour utilisation selon la revendication 12 ou 13, dans lequel ou laquelle le procédé comprend en outre l'administration au sujet d'un ou plusieurs parmi une immunothérapie, une chimiothérapie, un traitement chirurgical, une radiothérapie, une hormonothérapie, une petite molécule anticancéreuse, une thérapie par inhibiteur du facteur de croissance, une thérapie par CAR-T ou une thérapie par cytokines.

15. Virus oncolytique ou composition pour utilisation selon la revendication 14, dans lequel ou laquelle :
a) une immunothérapie est administrée au sujet ;
b) une chimiothérapie est administrée au sujet ; ou
c) un inhibiteur de point de contrôle immunitaire est administré au sujet, éventuellement dans lequel ou laquelle l'inhibiteur de point de contrôle immunitaire comprend un inhibiteur de PD-1, PD-L1, CTLA-4, LAG-3, BCH-3, BCH-4 ou TIM-3, en outre éventuellement dans lequel ou laquelle l'inhibiteur est un anticorps.
